# EUROPEAN PATENT APPLICATION

(11) **EP 3 470 428 A1**
(43) Date of publication of application: **17.04.2019**
(21) Application number: 17195780.6
(22) Date of filing: 10.10.2017
(51) Int. Cl.: C07K 16/28, A61K 39/00

(54) **ANTIBODIES TARGETING CD137 AND METHODS OF USE THEREOF**

(71) Applicant: Numab Innovation AG, 8820 Wädenswil (CH)
(72) Inventor: Gunde, Tea, 8005 Zurich (CH); Brock, Matthias, 8055 Zurich (CH); Hess, Christian, 8045 Zurich (CH); Simonin, Alexandre, 8804 Au ZH (CH)
(74) Representative: Virnekäs, Bernhard

(57) **Abstract**

The present invention relates generally to an isolated antibody or antigen-binding fragment thereof which specifically binds human CD137, a multispecific molecule comprising said isolated antibody of the invention or antigen-binding fragments thereof, and pharmaceutical compositions and methods of use thereof, a nucleic acid comprising a nucleotide sequence encoding said antibody, a vector comprising said nucleic acid, a host cell comprising said nucleic acid or said vector, and a method of producing said antibody.

## Description

### FIELD OF THE INVENTION

The present invention relates generally to an isolated antibody or antigen-binding fragment thereof which specifically binds human CD137, a multispecific molecule comprising said isolated antibody of the invention or antigen-binding fragments thereof, and pharmaceutical compositions and methods of use thereof, a nucleic acid comprising a nucleotide sequence encoding said antibody, a vector comprising said nucleic acid, a host cell comprising said nucleic acid or said vector, and a method of producing said antibody.

### BACKGROUND OF THE INVENTION

The tumor necrosis factor receptor superfamily (TNFRSF) is a protein superfamily of receptors characterized by their ability to bind tumor necrosis factors (TNFs) via cysteine-rich pseudorepeats in the extracellular domain (Locksley et al., 2001). At present, 27 TNF family members have been identified. TNFRSF members and their ligands are expressed mostly on immune cells, where they are playing a role of immunomodulators in T-cell-mediated immune responses. TNFRSF members play role in enhancement of dendric cell survival and priming capacity of T cells, optimal generation of effector T cells, optimal antibody responses, and amplification of inflammatory reactions.

CD137 (4-1BB, TNF-receptor superfamily 9, TNFRSF9) is a surface glycoprotein of the TNFR superfamily. It is an inducible costimulatory T cell receptor. CD137 expression is activation-dependent, and encompasses a broad subset of immune cells including activated NK and NKT cells, regulatory T cells, dendritic cells (DC) including follicular DC, stimulated mast cells, differentiating myeloid cells, monocytes, neutrophils, eosinophils (Wang et al, Immunol Rev. 229(1): 192-215 (2009)), and activated B cells (Zhang et al, J Immunol. 184(2):787-795 (2010)). In addition, CD137 expression has also been demonstrated on tumor vasculature (Broil K et al., Am J Clin Pathol. 115(4):543-549 (2001); Seaman et al, Cancer Cell 11(6):539-554 (2007)) and atherosclerotic endothelium (Olofsson et al, Circulation 117(10): 1292 1301 (2008)).

CD137-Ligand (CD137L, 4-1BBL or tnfsf9), a molecule of the TNF family, is an intercellular natural ligand known for CD137 (Alderson, M. R., et al., Eur. J. Immunol. 24:2219-2227 (1994); Pollok K., et al., Eur. J. Immunol. 24:367-374 (1994); Goodwin, R. G., et al., Eur. J. Immunol. 23: 2631-2641 (1993)). The ligand for CD137 forms a homotrimer, and the signaling via CD 137 proceeds from ligated molecules at the cell surface, which become cross-linked by trimerized ligand (Won, E. Y., et al., J. Biol. Chem. 285: 9202-9210 (2010)). The higher order clustering of CD 137 was suggested to be necessary for mediating the signaling. CD137 associates with the adaptors TRAF-2 and TRAF-1 in its cytoplasmic tail, resulting in coimmunoprecipitation, which is enhanced upon CD137 activation in T cells (Saoulli, K., et al., J. Exp. Med. 187: 1849-1862 (1998); Sabbagh, L., et al., J. Immunol. 180: 8093-8101 (2008)). Recruitment of TRAF-1 and TRAF-2 by CD137 results in downstream activation of NFKB and the Mitogen Activated Protein (MAP) Kinase cascade including ERK, JNK, and p38 MAP kinases. NFkB activation leads to upregulation of Bfl-1 and Bcl-XL, pro-survival members of the Bcl-2 family. The pro-apoptotic protein Bim is downregulated in a TRAF-1 and ERK dependent manner (Sabbagh et al., J Immunol. 180(12):8093-8101 (2008)). It has been suggested that the main action of CD137 is to place two or more TRAF-2 molecules in close molecular proximity to each other (Sanchez-Paulete, A. R., et al., Eur. J. Immunology 46(3): 513-522 (2016)). Based on this it was postulated that the major factor driving CD137 signaling is the relative density of TRAF-2-assembled CD137 moieties in micropatches of plasma membrane (Sanchez-Paulete, A. R., et al., Eur. J. Immunology 46(3): 513-522 (2016)). Overall, CD137 signaling is fostered by multimerization, and it was proposed that cross-linking CD137 molecules is the key factor in CD137 co-stimulatory activity.

CD137 co-stimulates T cells to carry out effector functions such as eradication of established tumors, broadening primary CD8⁺T cell responses, and enhancing the memory pool of antigen- specific CD8⁺ T cells, induction of interferon-gamma (IFN-γ) synthesis. The critical role of CD137 stimulation in CD8⁺ T-cell function and survival could be potentially utilized for the treatment of tumors through manipulation of the CD137/CD137L function. In fact, in vivo efficacy studies in mice have demonstrated that treatment with anti-CD 137 antibodies led to tumor regressions in multiple tumor models. For example, agonistic antimouse CD137 antibody were demonstrated to induce an immune response against P815 mastocytoma tumors, and low immunogenic tumor model Ag104 (I. Melero et al., Nat. Med., 3(6):682-5 (1997)). The efficacy of CD137 agonist mAbs in prophylactic and therapeutic settings for both monotherapy and combination therapy and anti-tumor protective T cell memory responses have been reported in several studies (Lynch et al., Immunol Rev. 222:277-286 (2008)). CD137 agonists also inhibit autoimmune reactions in a variety of autoimmunity models (Vinay et al, J Mol Med 84(9):726-736 (2006)).

Two anti-CD137 antibodies currently in the clinic are urelumab (Bristol-Myers Squibb), a fully humanized IgG4 mAb, and utomilumab (PF-05082566, Pfizer), a fully human IgG2 mAb (Chester C., et al., Cancer Immunol Immunother Oct;65(10):1243-8 (2016)). Although utilization of therapeutic antibodies agonizing CD137 is a very promising treatment strategy, it is coupled to such difficulties as low efficacy of anti-CD 137 agonist antibodies, high toxicities and adverse events. CD137 agonist antibodies were shown to lead to alterations in immune system and organ function increasing risks of toxicities. High doses of CD137 agonist antibodies in naïve and tumor-bearing mice have been reported to induce T-cell infiltration to the liver and elevations of aspartate aminotransferase and alanine aminotransferase consistent with liver inflammation (Niu L, et al. J Immunol 178(7):4194-4213 (2007); Dubrot J, et al., Int J Cancer 128(1):105-118 (2011)). Initial clinical studies into the human therapeutic use of CD137 agonist antibody have also demonstrated elevations of liver enzymes and increased incidence of hepatitis (Sznol M., et al., J Clin Oncol 26(115S):3007 (2008); Ascierto PA, et al., Semin Oncol 37(5):508-516 (2010); Chester C., et al., Cancer Immunol Immunother Oct;65(10):1243-8 (2016)). Potentially fatal hepatitis was observed in a Bristol-Myers Squibb (BMS) phase II anti-CD137 study for previously treated stage III/IV melanoma, National Clinical Trial (NCT) 00612664. This study and several others (NCT00803374, NCT00309023, NCT00461110, NCT00351325) were terminated due to adverse events (Chester C., et al., Cancer Immunol Immunother Oct;65(10):1243-8 (2016)). Such adverse events are most probably due to systemic overstimulation of T-cells.

Thus, there is a need in the field to generate improved therapeutic anti-human CD137 antibodies having high efficacy without the inherent side effects of general anti-proliferative drugs, in particular having lower toxicities comparable to the currently available CD137 antibodies.

### SUMMARY OF THE INVENTION

It is an object of the present invention to provide antibodies and antigen-binding fragments thereof that specifically bind to human CD137 protein, and which have beneficial properties.

In one aspect, the present invention relates to a novel CD 137 antibody antigen-binding fragment thereof.

In one aspect, the present invention relates to a multispecific molecule comprising the isolated antibody or antigen-binding fragments of the invention.

In one aspect, the present invention relates to a pharmaceutical composition comprising the isolated antibody or antigen-binding fragment thereof of the invention, or the multispecific molecule of the invention, and a pharmaceutically acceptable carrier.

In another aspect, the present invention relates to the antibody or antigen-binding fragment thereof of the invention, or the multispecific molecule of the invention, or the composition of the invention for use as a medicament

In one aspect, the present invention relates to the antibody or antigen-binding fragment thereof of the invention, or the multispecific molecule of the invention, or the composition of the invention for use in a manufacture of a medicament.

In one aspect, the present invention relates to the antibody or antigen-binding fragment thereof of the invention, or the multispecific molecule of the invention, or the composition of the invention for use in treating a cancer in a subject in need thereof.

In one aspect, the present invention relates to use of the antibody or antigen-binding fragment thereof of the invention, or the multispecific molecule of the invention, or the composition of the invention in the manufacture of a medicament for treatment of a cancer in a subject in need thereof.

In another aspect, the present invention relates to a method of treating a cancer in a subject in need thereof comprising administering to the subject a therapeutically effective amount of the antibody or antigen-binding fragment thereof of the invention, or the multispecific molecule of the invention, or the composition of the invention.

In yet another aspect, the present invention relates to a nucleic acid encoding the antibody or antigen-binding fragment thereof of the invention. In a further aspect, the present invention relates to a vector comprising said nucleic acid. In a further aspect, the present invention relates to a host cell comprising said nucleic acid or said vector.

In another aspect, the present invention relates to a method of producing the antibody or antigen-binding fragment thereof of the invention, the method comprising the step of culturing a host cell expressing a nucleic acid encoding the antibody or antigen-binding fragment thereof of the invention.

The aspects, advantageous features and preferred embodiments of the present invention summarized in the following items, respectively alone or in combination, further contribute to solving the object of the invention:
1. An isolated antibody or antigen-binding fragment thereof having a binding specificity for human CD137, which comprises: (a) a heavy chain variable region CDR1 comprising an amino acid sequence selected from any one of SEQ ID NOs: 1, 2, 5 and 8; (b) a heavy chain variable region CDR2 comprising an amino acid sequence selected from any of SEQ ID NOs: 3, 6 and 9; (c) a heavy chain variable region CDR3 comprising an amino acid sequence selected from any of SEQ ID NOs: 4, 7 and 10; (d) a light chain variable region CDR1 comprising an amino acid sequence selected from any of SEQ ID NOs: 14, 17 and 20; (e) a light chain variable region CDR2 comprising an amino acid sequence selected from any of SEQ ID NOs: 15, 18 and 21; and (f) a light chain variable region CDR3 comprising an amino acid sequence selected from any of SEQ ID NOs: 16, 19 and 22.
2. The antibody or antigen-binding fragment thereof of item 1, wherein the antibody comprises: (a) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively; (b) HCDR1, HCDR2, and HCDR3 sequences of SEQ ID NOs: 2, 3, and 4, respectively, and LCDR1, LCDR2, and LCDR3 sequences of SEQ ID NOs: 14, 15, and 16, respectively; (c) HCDR1, HCDR2, and HCDR3 sequences of SEQ ID NOs: 5, 6, and 7, respectively, and LCDR1, LCDR2, and LCDR3 sequences of SEQ ID NOs: 17, 18, and 19, respectively; (d) HCDR1, HCDR2, and HCDR3 sequences of SEQ ID NOs: 8, 9, and 10, respectively, and LCDR1, LCDR2, and LCDR3 sequences of SEQ ID NOs: 20, 21, and 22, respectively.
3. The isolated antibody or antigen-binding fragment thereof of item 1, comprising: (a) a HCDR1 comprising the amino acid sequence of SEQ ID NO: 1 or SEQ ID NO: 2; (b) a HCDR2 comprising the amino acid sequence of SEQ ID NO: 3; (c) a HCDR3 comprising the amino acid sequence of SEQ ID NO: 4; (d) a LCDR1 comprising the amino acid sequence of SEQ ID NOs: 14; (e) a LCDR2 comprising the amino acid sequence of SEQ ID NOs: 15; and (f) a LCDR3 comprising the amino acid sequence of SEQ ID NO: 16.
4. The antibody or antigen-binding fragment thereof of any one of the preceding items, wherein the antibody or antigen-binding fragment thereof comprises a heavy chain variable region comprising an amino acid sequence that is at least 90 percent identical to the amino acid sequence selected from the group consisting of SEQ ID NOs: 11, 12 and 13; and a light chain variable region comprising an amino acid sequence that is at least 90 percent identical to the amino acid sequence selected from the group consisting of SEQ ID NOs: 23, 24 and 25.
5. The antibody or antigen-binding fragment thereof of any one of the preceding items, wherein the antibody comprises: a heavy chain variable region comprising an amino acid sequence selected from any of SEQ ID NOs: 11, 12 and 13; and a light chain variable region comprising an amino acid sequence selected from any of SEQ ID NOs: 23, 24 and 25.
6. The isolated antibody or antigen-binding fragment thereof of any one of the preceding items, comprising: (a) a VH sequence of SEQ ID NO: 11 and a VL sequence of SEQ ID NO: 23; (b) a VH sequence of SEQ ID NO: 12 and a VL sequence of SEQ ID NO: 24; or (c) a VH sequence of SEQ ID NO: 13 and a VL sequence of SEQ ID NO: 25.
7. The isolated antibody or antigen-binding fragment thereof of item 3, comprising: (a) a HCDR1 comprising the amino acid sequence of SEQ ID NO: 1, a VH sequence of SEQ ID NO: 11 and a VL sequence of SEQ ID NO: 23; (b) a HCDR1 comprising the amino acid sequence of SEQ ID NO: 1, a VH sequence of SEQ ID NO: 12 and a VL sequence of SEQ ID NO: 24; or (c) a HCDR1 comprising the amino acid sequence of SEQ ID NO: 2, a VH sequence of SEQ ID NO: 13 and a VL sequence of SEQ ID NO: 25.
8. An isolated antibody or antigen-binding fragment thereof, wherein the antibody or antigen-binding fragment thereof comprises a heavy chain variable region comprising an amino acid sequence that is at least 90 percent identical to the amino acid sequence selected from the group consisting of SEQ ID NOs: 11, 12 and 13; and a light chain variable region comprising an amino acid sequence that is at least 90 percent identical to the amino acid sequence selected from the group consisting of SEQ ID NOs: 23, 24 and 25, wherein the antibody specifically binds to human CD137 protein.
9. The antibody or antigen-binding fragment thereof of any of the preceding items, wherein said antibody or said antigen-binding fragment
   (i) binds to human CD137 with a dissociation constant (KD) of less than 50nM, particularly less than 10 nM, particularly less than 5 nM, particularly less than 1nM, particularly less than 500 pM, more particularly less than 100 pM, more particularly less than 50 pM;
   (ii) binds to human CD137 with a K_{off} rate of 10⁻³ s⁻¹ or less, or 10⁻⁴ s⁻¹ or less, or 10⁻⁵ s⁻¹ or less as measured by SPR;
   (iii) binds to human CD137 with a Kₒₙ rate of at least 10⁴ M⁻¹s⁻¹ or greater, at least 10⁵ M⁻¹ s⁻¹ or greater, at least 10⁶ M⁻¹ s⁻¹ or greater, as measured by SPR;
   (iv) does not cross-compete with urelumab and utolimumab; and/or
   (v) is cross-reactive with Macaca fascicularis (Cynomolgus) CD137.
10. The antibody or antigen-binding fragment thereof of any one of the preceding items, which binds to human CD137 with a KD of less than 5nM, preferably less than 50pM.
11. The isolated antibody or antigen-binding fragment thereof of any of the previous items, wherein the isolated antibody or antigen-binding fragment is selected from the group consisting of: a monoclonal antibody, a chimeric antibody, a Fab, an Fv, a scFv, dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology), preferably Fv or scFv.
12. The antibody or functional fragment thereof of any one of the preceding items, which is a single-chain variable fragment (scFv).
13. The antibody or antigen-binding fragment thereof of item 12, wherein said scFv has the amino acid sequence selected from the group consisting of SEQ ID NO:27, SEQ ID NO: 28, and SEQ ID NO: 29.
14. The isolated antibody or antigen-binding fragment thereof of item 11, wherein the antibody or antigen-binding fragment thereof is an IgG selected from the group consisting of an IgG1, an IgG2, an IgG3 and an IgG4, preferably IgG4.
15. The isolated antibody or antigen-binding fragment thereof of any of the previous items, wherein the antibody or antigen-binding fragment thereof is chimeric, humanized or human.
16. An isolated antibody or antigen-binding fragment thereof binding to essentially the same epitope as the antibody or antigen-binding fragment of any one of items 1 to 15.
17. A multispecific molecule comprising any of the isolated antibody or antigen-binding fragments of any one of items 1 to 16.
18. The multispecific molecule of item 17, wherein said molecule is in a format selected from the group consisting of a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)2) or bibody (Fab-(scFv)1), Fab,, Fab-Fv2, Morrison (IgG CH₃-scFv fusion (Morrison L) or IgG CL-scFv fusion (Morrison H)), triabody (scDb-scFv), bispecific Fab2, di-miniantibody, tetrabody, scFv-Fc-scFv fusion, scFv-HSA-scFv fusion, di-diabody, DVD-Ig, COVD, IgG-scFab, scFab-dsscFv, Fv2-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs); an Fv, scFv, scDb, tandem-di-scFv, tandem tri-scFv, Fab-(scFv)2, Fab-(scFv)1, Fab, Fab-Fv2, COVD fused to the N- and/or the C-terminus of either chain of a heterodimeric Fc domain or any other heterodimerization domain, a MATCH and DuoBodies.
19. A pharmaceutical composition comprising the isolated antibody or antigen-binding fragment thereof of any one of items 1-16, or the multispecific molecule of items 17 to 18, and a pharmaceutically acceptable carrier.
20. The antibody or antigen-binding fragment thereof of any one of items 1-16, or the multispecific molecule of items 17 to 18, or the composition of item 19 for use as a medicament.
21. The antibody or antigen-binding fragment thereof of any one of items 1-16, or the multispecific molecule of items 17 to 18, or the composition of item 19 for use in a manufacture of a medicament.
22. The antibody or antigen-binding fragment thereof of any one of items 1-16, or the multispecific molecule of items 17 to 18, or the composition of item 19 for use in treating a cancer in a subject in need thereof.
23. Use of the antibody or antigen-binding fragment thereof of any one of items 1-16, or the multispecific molecule of items 17 to 18, or the composition of item 19 to treat a cancer in a subject in need thereof.
24. Use of the antibody or antigen-binding fragment thereof of any one of items 1-16, or the multispecific molecule of items 17 to 18, or the composition of item 19 in the manufacture of a medicament for treatment of a cancer, in a subject in need thereof.
25. A method of treating a cancer in a subject in need thereof comprising administering to the subject a therapeutically effective amount of the antibody or antigen-binding fragment thereof of any one of items 1-16, or the multispecific molecule of items 17 to 18, or the composition of item 19.
26. A nucleic acid encoding the antibody or antigen-binding fragment thereof of items 1-16.
27. A vector comprising the nucleic acid of item 26.
28. A host cell comprising the nucleic acid of item 26 or the vector of item 27.
29. A method of producing the antibody or antigen-binding fragment thereof of items 1-16, the method comprising the step of culturing a host cell expressing a nucleic acid encoding the antibody or antigen-binding fragment thereof of items 1-16.

### BRIEF DESCRIPTION OF THE DRAWINGS

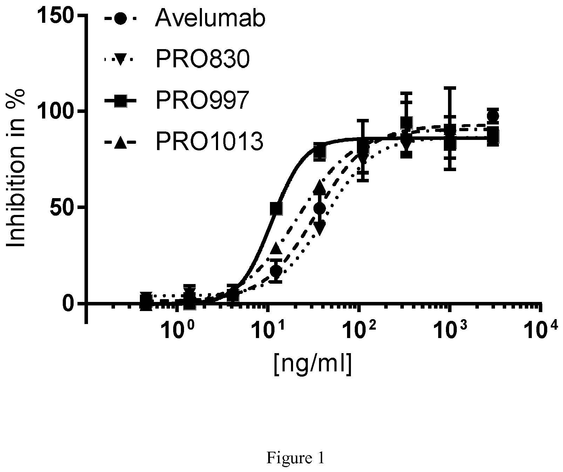

**FIG. 1** Effect of CDR set and framework selection on neutralization of the PDL1/PD-1 interaction in NFAT-Luciferase the reporter gene assay. % inhibition proportional to the luminescence signal obtained in the assay is represented in function of the molecules concentrations in ng/ml. Avelumab was used as reference.

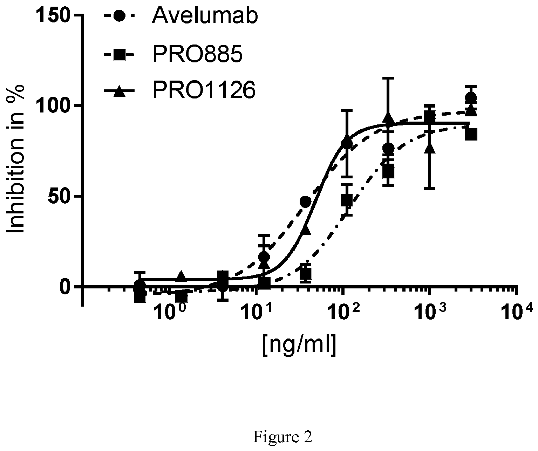

**FIG. 2** Effect of domain optimization on neutralization potency of the PDL1/PD-1 interaction in the NFAT-Luciferase reporter gene assay. % inhibition proportional to the luminescence signal obtained in the assay is represented in function of the scFv concentrations in ng/ml. Avelumab was used as reference.

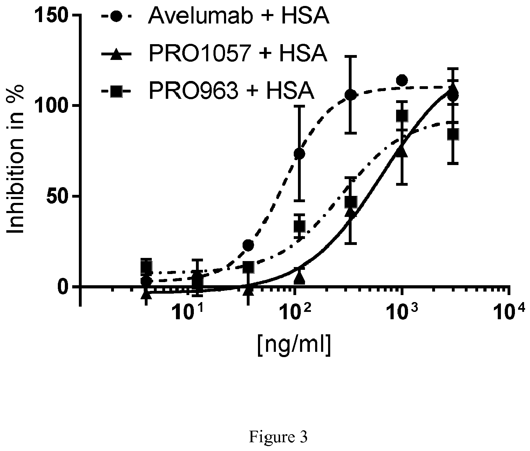

**FIG. 3** Neutralization potency of the PDL1/PD-1 interaction in the reporter gene assay by triabodies in presence of recombinant human serum albumin. % inhibition proportional to the luminescence signal obtained in the assay is represented in function of the scFv concentrations in ng/ml. Avelumab was used as reference.

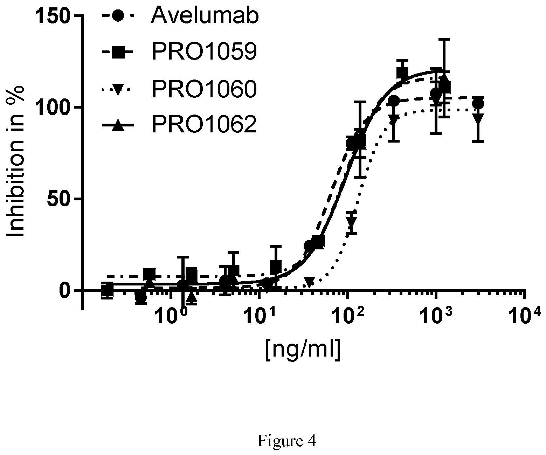

**FIG. 4** Potency of bivalent molecule and influence of LC or HC scFv fusion in Morrison formats on neutralization potency of the PDL1/PD-1 interaction in the NFAT-Luciferase reporter gene assay. % inhibition proportional to the luminescence signal obtained in the assay is represented in function of the molecules concentrations in ng/ml. Avelumab was used as reference.

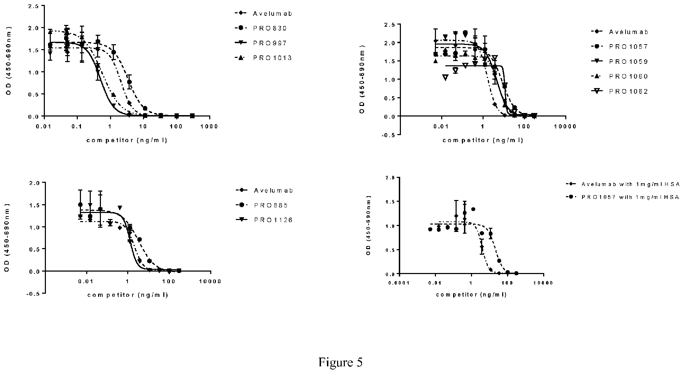

**FIG. 5** PD-1/PD-L1 competition ELISA. All molecules potently blocked the interaction between PD1 and PD-L1, with similar or smaller IC50 values than the reference IgG Avelumab.

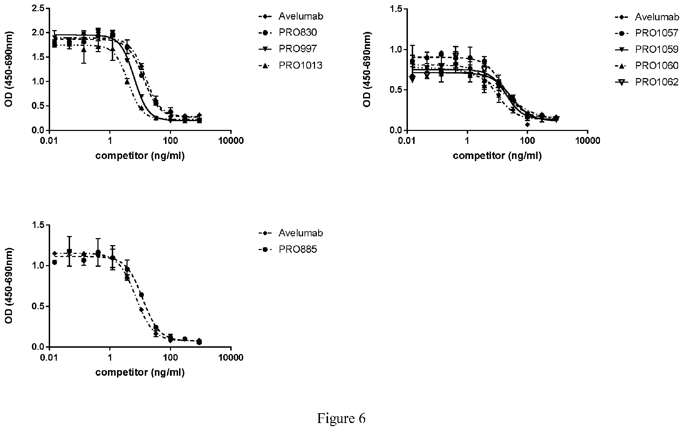

**FIG. 6** B7.1/PD-L1 competition ELISA. Similarly to Avelumab, all molecules potently blocked the interaction between B7.1 and PD-L1.

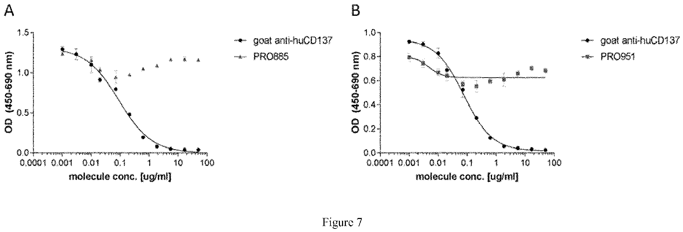

**FIG. 7** No inhibition of CD137 binding to CD137L in competition ELISA. The absorbance measured in the competitive ELISA assessing the binding of CD137L to CD137 are represented in function of increasing concentrations of PRO885 (A) or PRO951 (B) respectively. The inhibitory antibody goat anti-human CD137 served as a reference.

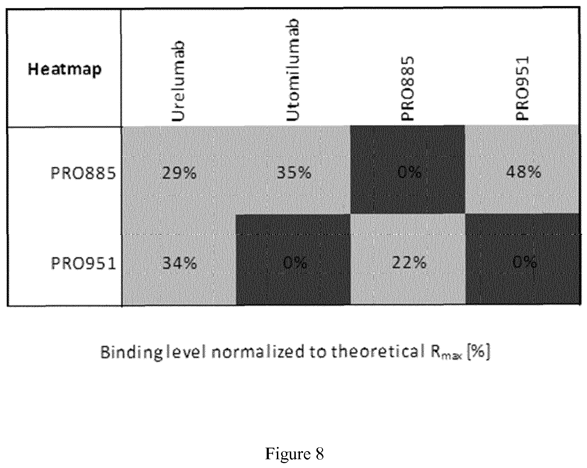

**FIG. 8** Heatmap of epitope binning results of PRO885 and PRO951 and Urelumab and Utomilumab. Binding level normalized to theoretical Rmax in percent (%) of analyte molecules (column) to immobilized molecules (row). No binding (dark grey) means same epitope, bright grey means the secondary molecule (analyte) can bind and has another epitope than the immobilized molecule.

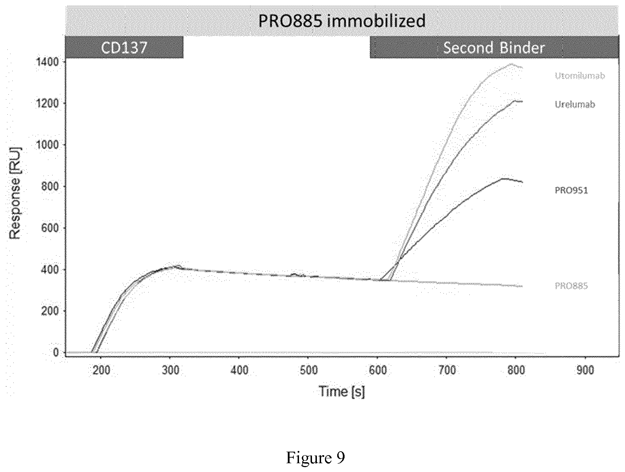

**FIG. 9** Epitope binning sensorgram of PRO885. PRO885 was immobilized on sensor chip and CD137 was captured by PRO885 in a first step (left hand side) followed by injections of the 4 different antibodies (right hand side). PRO951 as well as competitors were able to bind to captured CD137 whereas an injection of PRO885 did not show any binding.

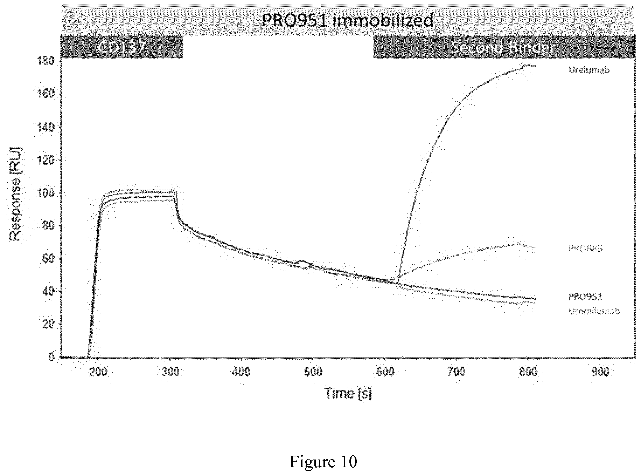

**FIG. 10** Epitope binning sensorgram of PRO951. PRO951 was immobilized on sensor chip and CD137 was captured by PRO951 in a first step (left hand side) followed by injections of the 4 different antibodies (right hand side). PRO885 as well as Urelumab was able to bind to captured CD137 whereas an injection of Utomilumab and PRO951 did not show further binding.

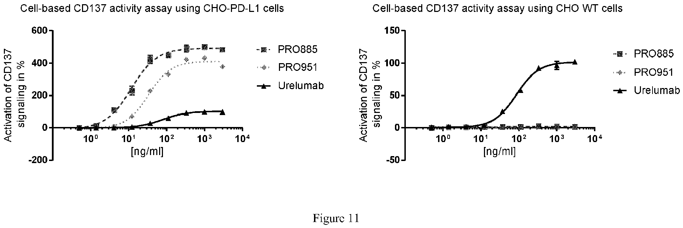

**FIG. 11** CD137 activation by PRO885 and PRO951 as assessed in the NFkB-Luciferase reporter gene assay. In the presence of PD-L1 expressing cells, PRO885 and PRO951 activated CD137 signaling in Jurkat cells whereas no activation was observed when CHO WT cells were tested. Urelumab activated CD137 signaling independently of PD-L1 expression. Luminescence was read 6h after addition of Jurkat reporter cells and data were fitted using sigmoidal 4PL fit (GraphPad Prism).

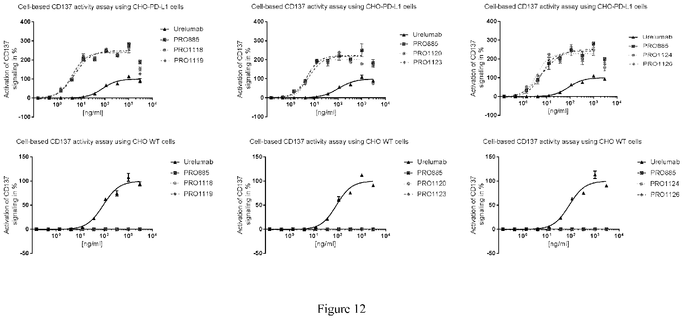

**FIG. 12** CD137 activation in the NFkB-Luciferase reporter gene assay by scDb with different affinities to PDL1 and CD137. In the presence of PD-L1 expressing CHO cells, all scDb activated CD137 signaling in Jurkat cells whereas no activation was observed when CHO WT cells were tested. Urelumab activated CD137 signaling independently of PD-L1 expression. Luminescence was read 6h after addition of Jurkat reporter cells and data were fitted using sigmoidal 4PL fit (GraphPad Prism).

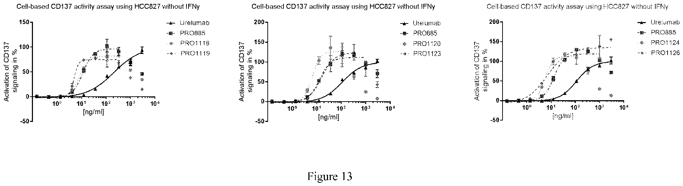

**FIG. 13** CD137 activation in the NFkB-Luciferase reporter gene assay by scDb with different affinities to PDL1 and CD137. In the presence of PD-L1 expressing HCC827 cells, all scDb activated CD137 signaling in Jurkat cells. Urelumab served as reference molecule to assess the relative activation of CD137 signaling. Potency increased slightly with increasing affinity to CD137 and PDL1. A signal decrease at high concentrations (bell-shaped curve) was more pronounced with increasing affinity to CD137, while increased affinity to PDL1 did not contribute to this effect. Luminescence was read 6h after addition of Jurkat reporter cells and data were fitted using sigmoidal 4PL fit (GraphPad Prism).

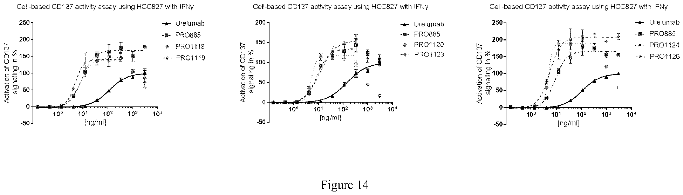

**FIG. 14** CD137 activation in the NFkB-Luciferase reporter gene assay by scDb with different affinities to PDL1 and CD137. In the presence of PD-L1 expressing HCC827 cells stimulated with IFNy for 24h at 10 ng/ml, STR grafted scDb activated CD137 signaling in Jurkat cells. Urelumab served as reference molecule to assess the relative activation of CD137 signaling. Potency increased slightly with increasing affinity to CD137 and PDL1. A signal decrease at high concentrations (bell-shaped curve) was more pronounced with increasing affinity to CD137, while increased affinity to PDL1 did not contribute to this effect. Luminescence was read 6h after addition of Jurkat reporter cells and data were fitted using sigmoidal 4PL fit (GraphPad Prism).

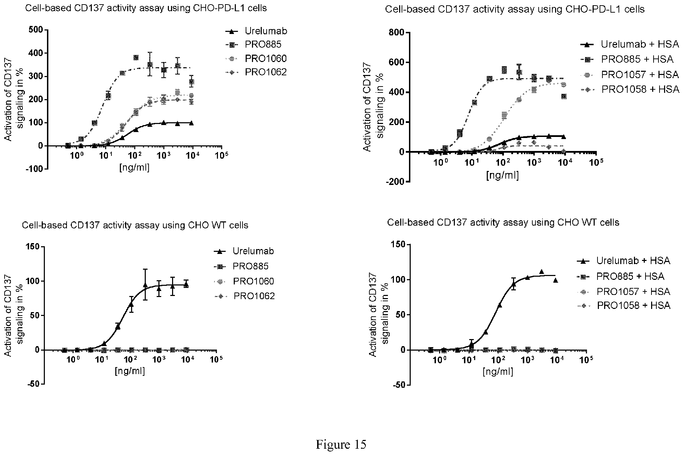

**FIG. 15** CD137 activation by molecules with prolonged serum half-life in the NFkB-Luciferase reporter gene assay after 6h. In the presence of PD-L1 expressing CHO cells, long half-life molecules activated CD137 signaling in Jurkat cells whereas no activation was observed when CHO WT cells were tested. Urelumab activated CD137 signaling independently of PD-L1 expression. Interestingly, despite similar affinities to both targets, PRO1057 showed a much higher maximal signal than PRO1058. And further, the monovalent scDb-scFv PRO1057 showed stronger activation than the respective bivalent Morrison format PRO1060. Luminescence was read 6h after addition of Jurkat reporter cells and data were fitted using sigmoidal 4PL fit (GraphPad Prism).

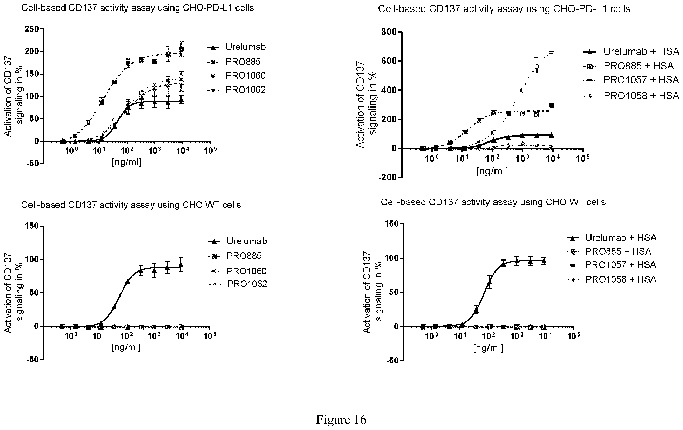

**FIG. 16** CD137 activation by molecules with prolonged serum half-life in the NFkB-Luciferase reporter gene assay after 24h. In the presence of PD-L1 expressing CHO cells, long half-life molecules activated CD137 signaling in Jurkat cells whereas no activation was observed when CHO WT cells were tested. Urelumab activated CD137 signaling independently of PD-L1 expression. Interestingly, despite similar affinities to both targets, PRO1057 showed a much higher maximal signal than PRO1058, which after 24 hours exceeded even the activity of the scDb Pro885. And further, the monovalent scDb-scFv PRO1057 showed much stronger activation than the respective bivalent Morrison format PRO1060. Luminscence was read 24h after addition of Jurkat reporter cells and data were fitted using sigmoidal 4PL fit (GraphPad Prism).

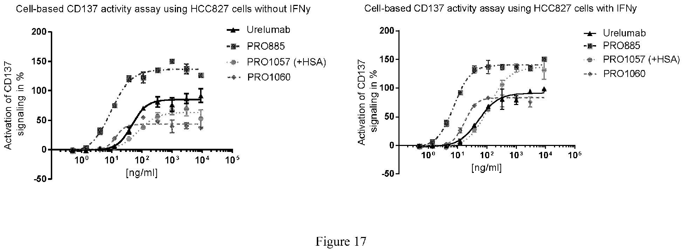

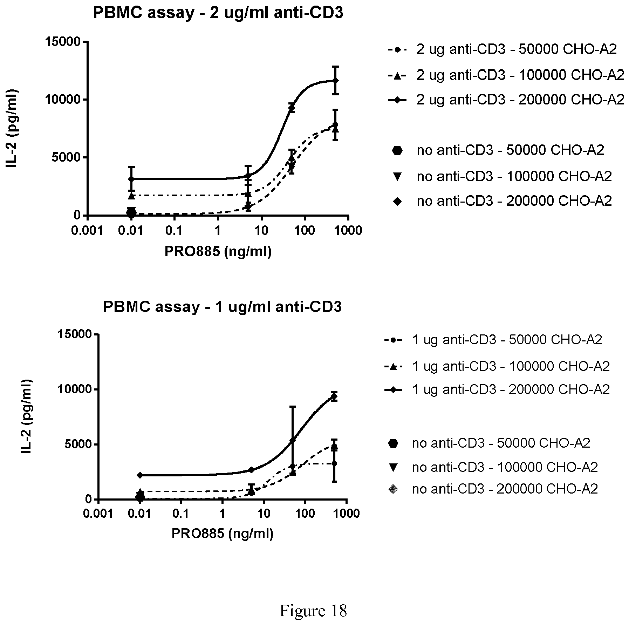

**FIG. 17** CD137 activation by molecules with prolonged half-life, after 24h. In the presence of HCC827 cells either unstimulated or stimulated with IFNy at 10 ng/ml for 24h, long half-life molecules activated CD137 signaling in Jurkat cells. Urelumab served as reference molecule to assess the relative activation of CD137 signaling. The monovalent scDb-scFv PRO1057 showed higher maximal activation than the respective bivalent Morrison format PRO1060. Luminescence was read 24h after addition of Jurkat reporter cells and data were fitted using sigmoidal 4PL fit (GraphPad Prism).**FIG. 18** Ex vivo T cell activation. The costimulatory engagement of PD-L1 and CD137 by PRO885 is shown, leading to IL-2 production clearly above background IL-2 levels. CHO-A2 cells are transgenic CHO cells expressing PD-L1.

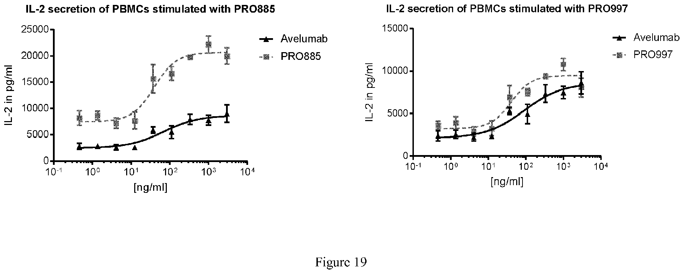

**FIG. 19** Ex vivo T cell activation assay. PBMC were stimulated with 10 ng/ml SEA and treated with serial dilutions of the scFv PRO997 or the scDb PRO885 for 96h. Activation of T-cells was assessed by quantification of IL-2 in harvested supernatants by ELISA. Treatment with PRO885 and PRO997 resulted in pronounced IL-2 secretion. PRO997 showed higher potency than Avelumab. PRO885 showed much increased effect size when compared to Avelumab. Data were fitted using sigmoidal 4PL fit (GraphPad Prism).

### DETAILED DESCRIPTION OF THE INVENTION

The present invention provides antibodies and antigen-binding fragments thereof that specifically bind to human CD137 protein, and pharmaceutical compositions, production methods, and methods of use of such antibodies and compositions.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

In a first aspect, the present invention relates to antibodies and antigen-binding fragments thereof that specifically bind to human CD 137.

The term "antibody" and the like, as used herein, include whole antibodies and any antigen-binding fragment (i.e., "antigen-binding portion") or single chains thereof. A naturally occurring "antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The terms "antigen-binding fragment", "antigen-binding fragment thereof", "antigen binding portion" of an antibody, and the like, as used herein, refer to one or more fragments of an intact antibody that retain the ability to specifically bind to a given antigen (e.g., CD 137). Antigen binding functions of an antibody can be performed by fragments of an intact antibody. Examples of binding fragments encompassed within the term "antigen binding portion" of an antibody include a Fab fragment, a monovalent fragment consisting of the VL, VH, CL and CHI domains; a F (ab)2 fragment, a bivalent fragment comprising two Fab fragments linked by a disulfide bridge at the hinge region; an Fd fragment consisting of the VH and CHI domains; an Fv fragment consisting of the VL and VH domains of a single arm of an antibody; a single domain antibody (dAb) fragment (Ward et al., 1989 Nature 341:544-546), which consists of a VH domain; an isolated complementarity determining region (CDR), dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology).

The term "Complementarity Determining Regions" ("CDRs") are amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273, 927-948 ("Chothia" numbering scheme), ImMunoGenTics (IMGT) numbering (Lefranc, M.-P., The Immunologist, 7, 132-136 (1999); Lefranc, M.-P. et al., Dev. Comp. Immunol., 27, 55-77 (2003) ("IMGT" numbering scheme) and numbering scheme described in Honegger & Plückthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 2624-32 34 (LCDR1), 50-52 56 (LCDR2), and 9189-96 97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align. In the context of the present invention, the numbering system suggested by Honegger & Plückthun ("AHo) is used (Honegger & Plückthun, J. Mol. Biol. 309 (2001) 657-670), unless specifically mentioned otherwise. Furthermore, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H26H27-H42; HCDR2 (also referred to as CDR-H2): H57-H76; HCDR3 (also referred to as CDR-H3): H108-H138.

Antigen binding portions can also be incorporated into single domain antibodies, maxibodies, minibodies, intrabodies, diabodies, triabodies, tetrabodies, v-NAR and bis-scFv (see, e.g., Hollinger and Hudson, 2005, Nature Biotechnology, 23, 9, 1 Hel 136). Antigen binding portions of antibodies can be grafted into scaffolds based on polypeptides such as Fibronectin type III (Fn3) (see U.S. Pat. No. 6,703,199, which describes fibronectin polypeptide monobodies). Antigen binding portions can be incorporated into single chain molecules comprising a pair of tandem Fv segments (VH-CH1-VH-CH1) which, together with complementary light chain polypeptides, form a pair of antigen binding regions (Zapata et al., 1995 Protein Eng. 8 (10): 1057-1062; and U.S. Pat. No. 5,641,870).

The term "binding specificity" as used herein refers to the ability of an individual antibody combining site to react with one antigenic determinant and not with a different antigenic determinant. As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. The antibody of the invention or antigen-binding fragment thereof has a binding specificity for human CD137.

Suitably, the antibody of the invention is an isolated antibody. The term "isolated antibody", as used herein, refers to an antibody that is substantially free of other antibodies having different antigenic specificities (e.g., an isolated antibody that specifically binds CD 137 is substantially free of antibodies that specifically bind antigens other than CD 137). An isolated antibody that specifically binds CD137 may, however, have cross-reactivity to other antigens, such CD137 molecules from other species. Moreover, an isolated antibody may be substantially free of other cellular material and/or chemicals.

Suitably, the antibody of the invention is a monoclonal antibody. The term "monoclonal antibody" or "monoclonal antibody composition" as used herein refers to antibodies that are substantially identical to amino acid sequence or are derived from the same genetic source. A monoclonal antibody composition displays a binding specificity and affinity for a particular epitope, or binding specificities and affinities for specific epitopes.

Antibodies of the invention include, but are not limited to, the chimeric, human and humanized.

The term "chimeric antibody" (or antigen-binding fragment thereof) is an antibody molecule (or antigen-binding fragment thereof) in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody" (or antigen-binding fragment thereof), as used herein, is intended to include antibodies (and antigen-binding fragments thereof) having variable regions in which both the framework and CDR regions are derived from sequences of human origin. Furthermore, if the antibody contains a constant region, the constant region also is derived from such human sequences, e.g., human germline sequences, or mutated versions of human germline sequences. The human antibodies and antigen-binding fragments thereof of the invention may include amino acid residues not encoded by human sequences (e.g., mutations introduced by random or site-specific mutagenesis in vitro or by somatic mutation in vivo). This definition of a human antibody specifically excludes a humanized antibody comprising non-human antigen-binding residues. Human antibodies can be produced using various techniques known in the art, including phage-display libraries (Hoogenboom and Winter, J. Mol. Biol, 227:381 (1991); Marks et al, J. Mol. Biol, 222:581 (1991)). Also available for the preparation of human monoclonal antibodies are methods described in Cole et al, Monoclonal Antibodies and Cancer Therapy, Alan R. Liss, p. 77 (1985); Boemer et al, J. Immunol, 147(1):86-95 (1991). See also van Dijk and van de Winkel, Curr. Opin. Pharmacol, 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE™ technology). See also, for example, Li et al, Proc. Natl. Acad. Sci. USA, 103:3557- 3562 (2006) regarding human antibodies generated via a human B-cell hybridoma technology.

A "humanized" antibody (or antigen-binding fragment thereof), as used herein, is an antibody (or antigen-binding fragment thereof) that retains the reactivity of a non-human antibody while being less immunogenic in humans. This can be achieved, for instance, by retaining the non-human CDR regions and replacing the remaining parts of the antibody with their human counterparts (i.e., the constant region as well as the framework portions of the variable region). Additional framework region modifications may be made within the human framework sequences as well as within the CDR sequences derived from the germline of another mammalian species. The humanized antibodies of the invention may include amino acid residues not encoded by human sequences (e.g., mutations introduced by random or site-specific mutagenesis in vitro or by somatic mutation in vivo, or a conservative substitution to promote stability or manufacturing). See, e.g., Morrison et al., Proc. Natl. Acad. Sci. USA, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include, but is not limited to Xoma technology disclosed in U.S. Pat. No. 5,766,886. The term "recombinant humanized antibody" as used herein, includes all human antibodies that are prepared, expressed, created or isolated by recombinant means, such as antibodies isolated from a host cell transformed to express the humanized antibody, e.g., from a transfectoma, and antibodies prepared, expressed, created or isolated by any other means that involve splicing of all or a portion of a human immunoglobulin gene, sequences to other DNA sequences.

The term "CD137" refers in particular to human CD137 with UniProt ID number Q07011 reproduced herein as SEQ ID NO: 52, or a variant thereof. Suitably, the antibodies of the invention or antigen-binding fragments thereof target CD137, in particular human CD137 as shown in UniProt ID number Q07011, or a variant thereof, reproduced herein as SEQ ID NO: 52. Suitably, the antibodies of the invention or antigen-binding fragments thereof target human and cynomoglous (Macaca fascicularis) CD137. The antibodies of the invention or antigen-binding fragments thereof specifically bind CD137. Preferably, the antibodies of the invention or antigen-binding fragments thereof do not block CD137/CD137L interaction.

Suitably, the antibody of the present invention or antigen-binding fragment thereof is CD137 agonist. An "activator" or "activating antibody" or "agonist" or "agonist antibody" is one that enhances or initiates signaling by the antigen to which it binds. In the context of the present invention, the term "CD137 agonist" encompasses the antibody of the present invention or antigen-binding fragment thereof that is capable to activate CD 137 signaling upon clustering of CD137-antigen-binding fragments thereof, e.g., wherein binding of at least two of said CD137-antigen-binding fragments allow for multimerization of the bound CD137 molecules and their activation. In some embodiments, agonist antibodies activate signaling without the presence of the natural ligand.

Antibodies of the invention include, but are not limited to, the humanized monoclonal antibodies isolated as described herein, including in the Examples. Examples of such anti-human CD137 antibodies are antibodies whose sequences are listed in Table 1. Additional details regarding the generation and characterization of the antibodies described herein are provided in the Examples.

The present invention provides antibodies that specifically bind to CD137 protein, said antibodies comprising a VH CDR having an amino acid sequence of any one of the VH CDRs listed in Table 1. In particular, the invention provides antibodies that specifically bind to CD137 protein, said antibodies comprising (or alternatively, consisting of) one, two, three, or more VH CDRs having an amino acid sequence of any of the VH CDRs listed in Table 1.

The present invention also provides antibodies and antigen-binding fragments thereof that specifically bind to CD137 protein, said antibodies or antigen-binding fragments thereof comprising a VL CDR having an amino acid sequence of any one of the VL CDRs listed in Table 1. In particular, the invention provides antibodies and antigen-binding fragments thereof that specifically bind to CD137 protein, said antibodies or antigen-binding fragments thereof comprising (or alternatively, consisting of) one, two, three or more VL CDRs having an amino acid sequence of any of the VL CDRs listed in Table 1.

Other antibodies and antigen-binding fragments thereof of the invention include amino acids that have been mutated, yet have at least 60, 70, 80, 90, 91, 92, 93, 94, 95, 96, 97, 98 or 99 percent identity in the CDR regions with the CDR regions depicted in the sequences described in Table 1. In one aspect, other antibodies and antigen-binding fragments thereof of the invention includes mutant amino acid sequences wherein no more than 1, 2, 3, 4 or 5 amino acids have been mutated in the CDR regions when compared with the CDR regions depicted in the sequence described in Table 1.

The terms "identical" or percent "identity", in the context of two or more nucleic acids or polypeptide sequences, refer to two or more sequences or subsequences that are the same. "Percent (%) amino acid sequence identity" and "homology" with respect to nucleic acid, a peptide, polypeptide or antibody sequence are defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2 or ALIGN software. Those skilled in the art can determine appropriate parameters for measuring alignment, including any algorithms needed to achieve maximal alignment over the full length of the sequences being compared.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, e.g., by the local homology algorithm of Smith and Waterman (1970) Adv. Appl. Math. 2:482c, by the homology alignment algorithm of Needleman and Wunsch, J. Mol. Biol. 48:443, 1970, by the search for similarity method of Pearson and Lipman, Proc. Nat'l. Acad. Sci. USA 85:2444, 1988, by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Brent et al., Current Protocols in Molecular Biology, John Wiley and Sons, Inc. (ringbou ed., 2003)).

Two examples of algorithms that are suitable for determining percent sequence identity and sequence similarity are the BLAST and BLAST 2.0 algorithms, which are described in Altschul et al., Nuc. Acids Res. 25:3389-3402, 1977; and Altschul et al., J. Mol. Biol. 215:403-410, 1990, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (N) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sci. USA 89: 10915, 1989) alignments (B) of 50, expectation (E) of 10, M=5, N=-4, and a comparison of both strands.

The BLAST algorithm also performs a statistical analysis of the similarity between two sequences (see, e.g., Karlin and Altschul, Proc. Natl. Acad. Sci. USA 90:5873- 5787, 1993). One measure of similarity provided by the BLAST algorithm is the smallest sum probability (P (N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller (Comput. Appl. Biosci., 4: 11-17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453, 1970) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, gamma-carboxyglutamate, and O-phosphoserine. The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

The present invention provides an isolated antibody or antigen-binding fragment thereof having a binding specificity for human CD137, which comprises: (a) a heavy chain variable region CDR1 comprising an amino acid sequence selected from any one of SEQ ID NOs: 1, 2, 5, 8, 30, 33 and 36; (b) a heavy chain variable region CDR2 comprising an amino acid sequence selected from any of SEQ ID NOs: 3, 6, 9, 31, 34 and 37; (c) a heavy chain variable region CDR3 comprising an amino acid sequence selected from any of SEQ ID NOs: 4, 7, 10, 32, 35 and 38; (d) a light chain variable region CDR1 comprising an amino acid sequence selected from any of SEQ ID NOs: 14, 17, 20, 40, 43, and 46; (e) a light chain variable region CDR2 comprising an amino acid sequence selected from any of SEQ ID NOs: 15, 18, 21, 41, 44, and 47; and (f) a light chain variable region CDR3 comprising an amino acid sequence selected from any of SEQ ID NOs: 16, 19, 22, 42, 45 and 48; wherein the antibody specifically binds human CD137. Suitably, the isolated antibody of the invention or antigen-binding fragment thereof having a binding specificity for human CD137 comprises: (a) a heavy chain variable region CDR1 comprising an amino acid sequence having at least 60, 70, 80, 90, 91, 92, 93, 94, 95, 96, 97, 98 or 99 percent identity to any one of SEQ ID NOs: 1, 2, 5, 8, 30, 33 and 36; (b) a heavy chain variable region CDR2 having at least 60, 70, 80, 90, 91, 92, 93, 94, 95, 96, 97, 98 or 99 percent identity to any of SEQ ID NOs: 3, 6, 9, 31, 34 and 37; (c) a heavy chain variable region CDR3 having at least 60, 70, 80, 90, 91, 92, 93, 94, 95, 96, 97, 98 or 99 percent identity to any of SEQ ID NOs: 4, 7, 10, 32, 35 and 38; (d) a light chain variable region CDR1 having at least 60, 70, 80, 90, 91, 92, 93, 94, 95, 96, 97, 98 or 99 percent identity to any of SEQ ID NOs: 14, 17, 20, 40, 43, and 46; (e) a light chain variable region CDR2 having at least 60, 70, 80, 90, 91, 92, 93, 94, 95, 96, 97, 98 or 99 percent identity to any of SEQ ID NOs: 15, 18, 21, 41, 44, and 47; and (f) a light chain variable region CDR3 having at least 60, 70, 80, 90, 91, 92, 93, 94, 95, 96, 97, 98 or 99 percent identity to any of SEQ ID NOs: 16, 19, 22, 42, 45 and 48.

In one embodiment, the antibody of the invention or antigen-binding fragment thereof having a binding specificity for human CD137 comprises: (a) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively; (b) HCDR1, HCDR2, and HCDR3 sequences of SEQ ID NOs: 2, 3, and 4, respectively, and LCDR1, LCDR2, and LCDR3 sequences of SEQ ID NOs: 14, 15, and 16, respectively; (c) HCDR1, HCDR2, and HCDR3 sequences of SEQ ID NOs: 5, 6, and 7, respectively, and LCDR1, LCDR2, and LCDR3 sequences of SEQ ID NOs: 17, 18, and 19, respectively; (d) HCDR1, HCDR2, and HCDR3 sequences of SEQ ID NOs: 8, 9, and 10, respectively, and LCDR1, LCDR2, and LCDR3 sequences of SEQ ID NOs: 20, 21, and 22, respectively; (e) HCDR1, HCDR2, and HCDR3 sequences of SEQ ID NOs: 30, 31, and 32, respectively, and LCDR1, LCDR2, and LCDR3 sequences of SEQ ID NOs: 40, 41, and 42, respectively; (f) HCDR1, HCDR2, and HCDR3 sequences of SEQ ID NOs: 33, 34, and 35, respectively, and LCDR1, LCDR2, and LCDR3 sequences of SEQ ID NOs: 43, 44, and 45, respectively; (g) HCDR1, HCDR2, and HCDR3 sequences of SEQ ID NOs: 36, 37, and 38, respectively, and LCDR1, LCDR2, and LCDR3 sequences of SEQ ID NOs: 46, 47, and 48, respectively.

Suitably, the antibody of the invention or antigen-binding fragment thereof having a binding specificity for human CD137 comprises: (a) HCDR1, HCDR2, and HCDR3 sequences having at least 60, 70, 80, 90, 91, 92, 93, 94, 95, 96, 97, 98 or 99 percent identity to SEQ ID NOs: 1, 3, and 4, respectively, and LCDR1, LCDR2, and LCDR3 sequences having at least 60, 70, 80, 90, 91, 92, 93, 94, 95, 96, 97, 98 or 99 percent identity to SEQ ID NOs: 14, 15, and 16, respectively; (b) HCDR1, HCDR2, and HCDR3 sequences having at least 60, 70, 80, 90, 91, 92, 93, 94, 95, 96, 97, 98 or 99 percent identity to SEQ ID NOs: 2, 3, and 4, respectively, and LCDR1, LCDR2, and LCDR3 sequences having at least 60, 70, 80, 90, 91, 92, 93, 94, 95, 96, 97, 98 or 99 percent identity to SEQ ID NOs: 14, 15, and 16, respectively; (c) HCDR1, HCDR2, and HCDR3 sequences having at least 60, 70, 80, 90, 91, 92, 93, 94, 95, 96, 97, 98 or 99 percent identity to SEQ ID NOs: 5, 6, and 7, respectively, and LCDR1, LCDR2, and LCDR3 sequences having at least 60, 70, 80, 90, 91, 92, 93, 94, 95, 96, 97, 98 or 99 percent identity to SEQ ID NOs: 17, 18, and 19, respectively; (d) HCDR1, HCDR2, and HCDR3 sequences having at least 60, 70, 80, 90, 91, 92, 93, 94, 95, 96, 97, 98 or 99 percent identity to SEQ ID NOs: 8, 9, and 10, respectively, and LCDR1, LCDR2, and LCDR3 sequences having at least 60, 70, 80, 90, 91, 92, 93, 94, 95, 96, 97, 98 or 99 percent identity to SEQ ID NOs: 20, 21, and 22, respectively; (e) HCDR1, HCDR2, and HCDR3 sequences having at least 60, 70, 80, 90, 91, 92, 93, 94, 95, 96, 97, 98 or 99 percent identity to SEQ ID NOs: 30, 31, and 32, respectively, and LCDR1, LCDR2, and LCDR3 sequences having at least 60, 70, 80, 90, 91, 92, 93, 94, 95, 96, 97, 98 or 99 percent identity to SEQ ID NOs: 40, 41, and 42, respectively; (f) HCDR1, HCDR2, and HCDR3 sequences having at least 60, 70, 80, 90, 91, 92, 93, 94, 95, 96, 97, 98 or 99 percent identity to SEQ ID NOs: 33, 34, and 35, respectively, and LCDR1, LCDR2, and LCDR3 sequences having at least 60, 70, 80, 90, 91, 92, 93, 94, 95, 96, 97, 98 or 99 percent identity to SEQ ID NOs: 43, 44, and 45, respectively; (g) HCDR1, HCDR2, and HCDR3 sequences having at least 60, 70, 80, 90, 91, 92, 93, 94, 95, 96, 97, 98 or 99 percent identity to SEQ ID NOs: 36, 37, and 38, respectively, and LCDR1, LCDR2, and LCDR3 sequences having at least 60, 70, 80, 90, 91, 92, 93, 94, 95, 96, 97, 98 or 99 percent identity to SEQ ID NOs: 46, 47, and 48, respectively.

In a further embodiment, the antibody of the invention or antigen-binding fragment thereof having a binding specificity for human CD137 comprises: (a) a HCDR1 comprising the amino acid sequence of SEQ ID NO: 1 or SEQ ID NO: 2; (b) a HCDR2 comprising the amino acid sequence of SEQ ID NO: 3; (c) a HCDR3 comprising the amino acid sequence of SEQ ID NO: 4; (d) a LCDR1 comprising the amino acid sequence of SEQ ID NOs: 14; (e) a LCDR2 comprising the amino acid sequence of SEQ ID NOs: 15; and (f) a LCDR3 comprising the amino acid sequence of SEQ ID NO: 16.

Suitably, the antibody of the invention or antigen-binding fragment thereof having a binding specificity for human CD137 comprises: (a) a HCDR1 comprising the amino acid sequence having at least 60, 70, 80, 90, 91, 92, 93, 94, 95, 96, 97, 98 or 99 percent identity to SEQ ID NO: 1 or SEQ ID NO: 2; (b) a HCDR2 comprising the amino acid sequence having at least 60, 70, 80, 90, 91, 92, 93, 94, 95, 96, 97, 98 or 99 percent identity to SEQ ID NO: 3; (c) a HCDR3 comprising the amino acid sequence having at least 60, 70, 80, 90, 91, 92, 93, 94, 95, 96, 97, 98 or 99 percent identity to SEQ ID NO: 4; (d) a LCDR1 comprising the amino acid sequence having at least 60, 70, 80, 90, 91, 92, 93, 94, 95, 96, 97, 98 or 99 percent identity to SEQ ID NOs: 14; (e) a LCDR2 comprising the amino acid sequence having at least 60, 70, 80, 90, 91, 92, 93, 94, 95, 96, 97, 98 or 99 percent identity to SEQ ID NOs: 15; and (f) a LCDR3 comprising the amino acid sequence of SEQ ID NO: 16.

In a further embodiment, the antibody of the invention or antigen-binding fragment thereof having a binding specificity for human CD137 comprises: (a) a HCDR1 comprising the amino acid sequence of SEQ ID NO: 30; (b) a HCDR2 comprising the amino acid sequence of SEQ ID NO: 31; (c) a HCDR3 comprising the amino acid sequence of SEQ ID NO: 32; (d) a LCDR1 comprising the amino acid sequence of SEQ ID NOs: 40; (e) a LCDR2 comprising the amino acid sequence of SEQ ID NOs: 41; and (f) a LCDR3 comprising the amino acid sequence of SEQ ID NO: 42.

Suitably, the antibody of the invention or antigen-binding fragment thereof having a binding specificity for human CD137 comprises: (a) a HCDR1 comprising the amino acid sequence having at least 60, 70, 80, 90, 91, 92, 93, 94, 95, 96, 97, 98 or 99 percent identity to SEQ ID NO: 30; (b) a HCDR2 comprising the amino acid sequence having at least 60, 70, 80, 90, 91, 92, 93, 94, 95, 96, 97, 98 or 99 percent identity to SEQ ID NO: 31; (c) a HCDR3 comprising the amino acid sequence having at least 60, 70, 80, 90, 91, 92, 93, 94, 95, 96, 97, 98 or 99 percent identity to SEQ ID NO: 32; (d) a LCDR1 comprising the amino acid sequence having at least 60, 70, 80, 90, 91, 92, 93, 94, 95, 96, 97, 98 or 99 percent identity to SEQ ID NOs: 40; (e) a LCDR2 comprising the amino acid sequence having at least 60, 70, 80, 90, 91, 92, 93, 94, 95, 96, 97, 98 or 99 percent identity to SEQ ID NOs: 41; and (f) a LCDR3 comprising the amino acid sequence having at least 60, 70, 80, 90, 91, 92, 93, 94, 95, 96, 97, 98 or 99 percent identity to SEQ ID NO: 42.

In a further embodiment, the present invention provides an isolated antibody or an antigen-binding fragment thereof that specifically binds CD137 (e.g., human CD137 protein), wherein said antibody or said antigen-binding fragment thereof comprises a VH domain and a VL domain. In the context of the present invention the terms "VH" (variable heavy chain), "VL" (variable light chain), "Vκ" and "Vλ" refer to families of antibody heavy and light chain sequences that are grouped according to sequence identity and homology. Methods for the determination of sequence homologies, for example by using a homology search matrix such as BLOSUM (Henikoff, S. & Henikoff, J. G., Proc. Natl. Acad. Sci. USA 89 (1992) 10915-10919), and methods for the grouping of sequences according to homologies are well known to one of ordinary skill in the art. For VH, Vκ and Vλ different subfamilies can be identified, as shown, for example, in Knappik et al., J. Mol. Biol. 296 (2000) 57-86, which groups VH in VH1A, VH1B and VH2 to VH6, Vκ in Vκ1 to Vκ4 and Vλ in Vλ1 to Vλ3. In vivo, antibody Vκ chains, Vλ chains, and VH chains are the result of the random rearrangement of germline κ chain V and J segments, germline λ chain V and J segments, and heavy chain V, D and J segments, respectively. To which subfamily a given antibody variable chain belongs is determined by the corresponding V segment, and in particular by the framework regions FR1 to FR3. Thus, any VH sequence that is characterized in the present application by a particular set of framework regions HFR1 to HFR3 only, may be combined with any HFR4 sequence, for example a HFR4 sequence taken from one of the heavy chain germline J segments, or a HFR4 sequence taken from a rearranged VH sequence.

Suitably, the present invention provides an isolated antibody or an antigen-binding fragment thereof that specifically binds CD137 (e.g., human CD137 protein), wherein said antibody or an antigen-binding fragment thereof comprises a VH4 or VH3 domain, preferably VH4 domain.

Suitably, the present invention provides an isolated antibody or an antigen-binding fragment thereof that specifically binds CD137 (e.g., human CD137 protein), wherein said antibody or an antigen-binding fragment thereof comprises Vκ frameworks FR1, FR2 and FR3, particularly Vκ1 or Vκ3 frameworks, preferably Vκ1 frameworks FR1 to 3, and a framework FR4, which is selected from a Vκ FR4, particularly Vκ1 FR4, Vκ3 FR4, and a Vλ FR4. Suitable Vλ FR4 are as set forth in SEQ ID NO: 53 to SEQ ID NO: 59. In one embodiment the present invention provides an isolated antibody or an antigen-binding fragment thereof that specifically binds CD137 (e.g., human CD137 protein), wherein said antibody or an antigen-binding fragment thereof comprises Vλ FR4 comprising the amino acid sequence having at least 60, 70, 80, 90 percent identity to comprising an amino acid sequence selected from any of SEQ ID NO: 53 to SEQ ID NO: 59, preferably to SEQ ID NO: 53.

Thus, in one embodiment, the present invention thus provides an antibody or antigen binding fragment thereof having a binding specificity for human CD137 comprising:
(i) the HCDR1, HCDR2, and HCDR3 sequences of:
   a. SEQ ID NOs: 1, 3, and 4, respectively, and the LCDR1, LCDR2, and LCDR3 sequences of SEQ ID NOs: 14, 15, and 16, respectively;
   b. SEQ ID NOs: 2, 3, and 4, respectively, and the LCDR1, LCDR2, and LCDR3 sequences of SEQ ID NOs: 14, 15, and 16, respectively; or
   c. SEQ ID NOs: 30, 31, and 32, respectively, and the LCDR1, LCDR2, and LCDR3 sequences of SEQ ID NOs: 40, 41, and 42, respectively
(ii) a VH3 or VH4 domain, preferably VH4 domain; and
(iii) a VL domain comprising a VL framework comprising Vκ frameworks FR1, FR2 and FR3, particularly Vκ1 or Vκ3 FR1 to FR3, preferably Vκ1 FR1 to FR3, and a framework FR4, which is selected from a Vκ FR4, particularly Vκ1 FR4, Vκ3 FR4, and a Vλ FR4, particularly Vλ FR4 comprising the amino acid sequence having at least 60, 70, 80, 90 percent identity to an amino acid sequence selected from any of SEQ ID NO: 53 to SEQ ID NO: 59, preferably Vλ FR4 is as set forth in SEQ ID NO: 53 to SEQ ID NO: 59, more preferably Vλ FR4 is as set forth in SEQ ID NO: 53.

In one embodiment, the present invention thus provides an antibody or antigen binding fragment thereof having a binding specificity for human CD137 and comprising a VL comprising:
(i) CDR domains CDR1, CDR2 and CDR3;
(ii) human Vκ framework regions FR1 to FR3, particularly human Vκ1 framework regions FR1 to FR3;
(iii) FR4, which is selected from (a) a human Vλ germ line sequence for FR4, particularly a Vλ germ line sequence selected from the list of: SEQ ID NO: 53 to 59, preferably SEQ ID NO: 53; and (b) a Vλ-based sequence, which has one or two mutations, particularly one mutation, compared to the closest human Vλ germ line sequence for FR4 comprising an amino acid sequence selected from any of SEQ ID NO: 53 to SEQ ID NO: 59, preferably SEQ ID NO: 53.

In a preferred embodiment, the present invention provides an antibody or antigen binding fragment thereof having a binding specificity for human CD137 and comprising:
(i) the HCDR1, HCDR2, and HCDR3 sequences of:
   a. SEQ ID NOs: 1, 3, and 4, respectively, and the LCDR1, LCDR2, and LCDR3 sequences of SEQ ID NOs: 14, 15, and 16, respectively;
   b. SEQ ID NOs: 2, 3, and 4, respectively, and the LCDR1, LCDR2, and LCDR3 sequences of SEQ ID NOs: 14, 15, and 16, respectively; or
   c. SEQ ID NOs: 30, 31, and 32, respectively, and the LCDR1, LCDR2, and LCDR3 sequences of SEQ ID NOs: 40, 41, and 42, respectively
(ii) a VH4 domain; and
(iii) a VL domain comprising a VL framework comprising Vκ1 frameworks FR1, FR2 and FR3, and a Vλ FR4 comprising the amino acid sequence having at least 60, 70, 80, 90 percent identity to comprising an amino acid sequence selected from any of SEQ ID NO: 53 to SEQ ID NO: 59, particularly Vλ FR4 as set forth in SEQ ID NO: 53 to SEQ ID NO: 59, preferably SEQ ID NO: 53.

The present invention provides an isolated antibody or an antigen-binding fragment thereof that specifically binds CD137 (e.g., human CD137 protein), wherein said antibody or antigen-binding fragment thereof comprises a VH domain listed in Table 1.

The invention also provides an isolated antibody or an antigen-binding fragment thereof that specifically binds to CD137, wherein said antibody or antigen-binding fragments thereof comprises (or alternatively, consisting of) a VH amino acid sequence listed in Table 1, wherein no more than about 10 amino acids in a framework sequence (for example, a sequence which is not a CDR) have been mutated (wherein a mutation is, as various non-limiting examples, an addition, substitution or deletion).

The invention also provides an isolated antibody or an antigen-binding fragment thereof that specifically binds to CD137, wherein said antibody or antigen-binding fragments thereof comprises (or alternatively, consisting of) a VH amino acid sequence listed in Table 1, wherein no more than about 20 amino acids in a framework sequence (for example, a sequence which is not a CDR) have been mutated (wherein a mutation is, as various non-limiting examples, an addition, substitution or deletion).

Other antibodies and antigen-binding fragments thereof of the invention include amino acids that have been mutated, yet have at least 60, 70, 80, 90, 91, 92, 93, 94, 95, 96, 97, 98 or 99 percent identity in the VH regions with the VH regions depicted in the sequences described in Table 1.

The present invention provides an isolated antibody or an antigen-binding fragment thereof that specifically binds to CD137 protein, said antibody or antigen-binding fragments thereof comprises a VL domain listed in Table 1.

The invention also provides an isolated antibody or an antigen-binding fragment thereof that specifically binds to CD137, wherein said antibody or antigen-binding fragments thereof comprises (or alternatively, consisting of) a VL amino acid sequence listed in Table 1, wherein no more than about 10 amino acids in a framework sequence (for example, a sequence which is not a CDR) have been mutated (wherein a mutation is, as various non-limiting examples, an addition, substitution or deletion).

The invention also provides an isolated antibody or an antigen-binding fragment thereof that specifically binds to CD137, wherein said antibody or antigen-binding fragment thereof comprises (or alternatively, consisting of) a VL amino acid sequence listed in Table 1, wherein no more than about 20 amino acids in a framework sequence (for example, a sequence which is not a CDR) have been mutated (wherein a mutation is, as various non-limiting examples, an addition, substitution or deletion).

Other antibodies and antigen-binding fragments thereof of the invention include amino acids that have been mutated, yet have at least 60, 70, 80, 90, 91, 92, 93, 94, 95, 96, 97, 98 or 99 percent identity in the VL regions with the VL regions depicted in the sequences described in Table 1.

The invention also provides an isolated antibody or an antigen-binding fragment thereof that specifically binds to CD137, wherein said antibody or antigen-binding fragment thereof comprises a heavy chain variable region comprising an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 11, 12, 13 and 39; and a light chain variable region comprising an amino acid sequence that is at least 60, 70, 80, 90, 91, 92, 93, 94, 95, 96, 97, 98 or 99 percent, preferably at least 90 percent, identical to the amino acid sequence selected from the group consisting of SEQ ID NOs: 23, 24, 25 and 49.

In one embodiment, the antibody of the invention or antigen-binding fragment thereof having a binding specificity for human CD137 comprises: a heavy chain variable region comprising an amino acid sequence selected from any of SEQ ID NOs: 11, 12, 13 and 39; and a light chain variable region comprising an amino acid sequence selected from any of SEQ ID NOs: 23, 24, 25 and 49.

In a further embodiment, the isolated antibody of the invention or antigen-binding fragment thereof having a binding specificity for human CD137 comprises: (a) a VH sequence of SEQ ID NO: 11 and a VL sequence of SEQ ID NO: 23; (b) a VH sequence of SEQ ID NO: 12 and a VL sequence of SEQ ID NO: 24; (c) a VH sequence of SEQ ID NO: 13 and a VL sequence of SEQ ID NO: 25; or (d) a VH sequence of SEQ ID NO: 39 and a VL sequence of SEQ ID NO: 49.

In one embodiment, the antibody of the invention or antigen-binding fragment thereof having a binding specificity for human CD137 comprises:
(a) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively, a VH sequence comprising an amino acid sequence that is at least 60, 70, 80, 90, 91, 92, 93, 94, 95, 96, 97, 98 or 99 percent, preferably at least 90 percent, identical to SEQ ID NO: 11, and a VL sequence comprising an amino acid sequence that is at least 60, 70, 80, 90, 91, 92, 93, 94, 95, 96, 97, 98 or 99 percent, preferably at least 90 percent, identical to SEQ ID NO: 23;
(b) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively, a VH sequence comprising an amino acid sequence that is at least 60, 70, 80, 90, 91, 92, 93, 94, 95, 96, 97, 98 or 99 percent, preferably at least 90 percent, identical to SEQ ID NO: 12, and a VL sequence comprising an amino acid sequence that is at least 60, 70, 80, 90, 91, 92, 93, 94, 95, 96, 97, 98 or 99 percent, preferably at least 90 percent, identical to SEQ ID NO: 24;
(c) HCDR1, HCDR2, and HCDR3 sequences of SEQ ID NOs: 2, 3, and 4, respectively, and LCDR1, LCDR2, and LCDR3 sequences of SEQ ID NOs: 14, 15, and 16, respectively, a VH sequence comprising an amino acid sequence that is at least 60, 70, 80, 90, 91, 92, 93, 94, 95, 96, 97, 98 or 99 percent, preferably at least 90 percent, identical to SEQ ID NO: 13, and a VL sequence comprising an amino acid sequence that is at least 60, 70, 80, 90, 91, 92, 93, 94, 95, 96, 97, 98 or 99 percent, preferably at least 90 percent, identical to SEQ ID NO: 25; or
(d) HCDR1, HCDR2, and HCDR3 sequences of SEQ ID NOs: 30, 31, and 32, respectively, and LCDR1, LCDR2, and LCDR3 sequences of SEQ ID NOs: 40, 41, and 42, respectively, a VH sequence comprising an amino acid sequence that is at least 60, 70, 80, 90, 91, 92, 93, 94, 95, 96, 97, 98 or 99 percent, preferably at least 90 percent, identical to SEQ ID NO: 39, and a VL sequence comprising an amino acid sequence that is at least 60, 70, 80, 90, 91, 92, 93, 94, 95, 96, 97, 98 or 99 percent, preferably at least 90 percent, identical to SEQ ID NO: 49.

In one embodiment, the antibody of the invention or antigen-binding fragment thereof having a binding specificity for human CD137
comprises:
(a) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively, a VH sequence of SEQ ID NO: 11 and a VL sequence of SEQ ID NO: 23;
(b) HCDR1, HCDR2, and HCDR3 sequences of SEQ ID NOs: 1, 3, and 4, respectively, and LCDR1, LCDR2, and LCDR3 sequences of SEQ ID NOs: 14, 15, and 16, respectively, a VH sequence of SEQ ID NO: 12 and a VL sequence of SEQ ID NO: 24;
(c) HCDR1, HCDR2, and HCDR3 sequences of SEQ ID NOs: 2, 3, and 4, respectively, and LCDR1, LCDR2, and LCDR3 sequences of SEQ ID NOs: 14, 15, and 16, respectively, a VH sequence of SEQ ID NO: 13 and a VL sequence of SEQ ID NO: 25; or
(d) HCDR1, HCDR2, and HCDR3 sequences of SEQ ID NOs: 30, 31, and 32, respectively, and LCDR1, LCDR2, and LCDR3 sequences of SEQ ID NOs: 40, 41, and 42, respectively, a VH sequence of SEQ ID NO: 39 and a VL sequence of SEQ ID NO: 49.

In one embodiment, an antibody that specifically binds to CD137 is an antibody that is described in Table 1. In one embodiment, an antibody that specifically binds to CD137 is as set forth in SEQ ID NO: 27, SEQ ID NO: 28, SEQ ID NO: 29, or SEQ ID NO: 51. In one embodiment, an antibody that specifically binds to CD137 is as set forth in SEQ ID NO: 29.

Other antibodies and antigen-binding fragments thereof of the invention include those wherein the amino acids or nucleic acids encoding the amino acids have been mutated, yet have at least 60, 70, 80, 90 or 95 percent identity to the sequences described in Table 1. In one embodiment, it includes mutant amino acid sequences wherein no more than 1, 2, 3, 4 or 5 amino acids have been mutated in the variable regions when compared with the variable regions depicted in the sequence described in Table 1, while retaining substantially the same therapeutic activity.

Given that each of these antibodies can bind to CD137 and that antigen-binding specificity is provided primarily by the CDR1, 2 and 3 regions, the VH CDR1, 2 and 3 sequences and VL CDR1, 2 and 3 sequences can be "mixed and matched" (i.e., CDRs from different antibodies can be mixed and match, although each antibody must contain a VH CDR1, 2 and 3 and a VL CDR1, 2 and 3 to create other CD137-binding binding molecules of the invention. Such "mixed and matched" CD137-binding antibodies can be tested using the binding assays known in the art and those described in the Examples (e.g., ELISAs). When VH CDR sequences are mixed and matched, the CDR1, CDR2 and/or CDR3 sequence from a particular VH sequence should be replaced with a structurally similar CDR sequence(s). Likewise, when VL CDR sequences are mixed and matched, the CDR1, CDR2 and/or CDR3 sequence from a particular VL sequence should be replaced with a structurally similar CDR sequence(s). It will be readily apparent to the ordinarily skilled artisan that novel VH and VL sequences can be created by mutating one or more VH and/or VL CDR region sequences with structurally similar sequences from the CDR sequences shown herein for monoclonal antibodies of the present invention.

In yet another embodiment, the present invention provides an antibody or an antigen-binding fragment thereof comprising amino acid sequences that are homologous to the sequences described in Table 1, and said antibody binds to CD137, and retains the desired functional properties of those antibodies described in Table 1.

For example, the invention provides an isolated monoclonal antibody (or a functional antigen-binding fragment thereof) comprising a heavy chain variable region and a light chain variable region, wherein the heavy chain variable region comprises an amino acid sequence that is at least 80 percent, at least 90 percent, or at least 95 percent identical to an amino acid sequence selected from the group consisting of SEQ ID NOs: 11, 12, 13 and 39; the light chain variable region comprises an amino acid sequence that is at least 80 percent, at least 90 percent, or at least 95 percent identical to an amino acid sequence selected from the group consisting of SEQ ID NOs: 23, 24, 25 and 49; wherein the antibody specifically binds to human CD137 protein.

In one embodiment, the VH and/or VL amino acid sequences may be 50 percent, 60 percent, 70 percent, 80 percent, 90 percent, 95 percent, 96 percent, 97 percent, 98 percent or 99 percent identical to the sequences set forth in Table 1. In one embodiment, the VH and/or VL amino acid sequences may be identical except an amino acid substitution in no more than 1, 2, 3, 4 or 5 amino acid positions.

In one embodiment, an antibody of the invention has a heavy chain variable region comprising CDR1, CDR2, and CDR3 sequences and a light chain variable region comprising CDR1, CDR2, and CDR3 sequences, wherein one or more of these CDR sequences have specified amino acid sequences based on the antibodies described herein or conservative modifications thereof, and wherein the antibodies retain the desired functional properties of the CD137-binding antibodies and antigen-binding fragments thereof of the invention.

The term "conservatively modified variant" or "conservative variants" applies to both amino acid and nucleic acid sequences. With respect to particular nucleic acid sequences, conservatively modified variants refer to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations", which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" or "conservative variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). In one embodiment, the term "conservative sequence modifications" are used to refer to amino acid modifications that do not significantly affect or alter the binding characteristics of the antibody containing the amino acid sequence.

Accordingly, the invention provides an isolated monoclonal antibody, or a functional antigen-binding fragment thereof, consisting of a heavy chain variable region comprising CDR1, CDR2, and CDR3 sequences and a light chain variable region comprising CDR1, CDR2, and CDR3 sequences, wherein: the heavy chain variable region CDR1 comprises an amino acid sequence selected from any of SEQ ID NOs: 1, 2, 5, 8, 30, 33 and 36, or conservative variants thereof; the heavy chain variable region CDR2 comprises an amino acid sequence selected from any of SEQ ID NOs: 3, 6, 9, 31, 34 and 37, or conservative variants thereof; the heavy chain variable region CDR3 comprises an amino acid sequence selected from any of SEQ ID NOs: 4, 7, 10, 32, 35 and 38, or conservative variants thereof; the light chain variable region CDR1 comprises an amino acid sequence selected from any of SEQ ID NOs: 14, 17, 20, 40, 43, and 46, or conservative variants thereof; the light chain variable region CDR2 comprises an amino acid sequence selected from any of SEQ ID NOs: 15, 18, 21, 41, 44, and 47, or conservative variants thereof; and the light chain variable region CDR3 comprises an amino acid sequence selected from any of SEQ ID NOs: 16, 19, 22, 42, 45 and 48, or conservative variants thereof; wherein the antibody or the antigen-binding fragment thereof specifically binds to CD137 and is capable of activating CD137 signaling with or without additional cross-linking.

In one embodiment, an antibody of the invention is optimized for expression in a mammalian cell has a heavy chain variable region and a light chain variable region, wherein one or more of these sequences have specified amino acid sequences based on the antibodies described herein or conservative modifications thereof, and wherein the antibodies retain the desired functional properties of the CD137-binding antibodies and antigen-binding fragments thereof of the invention. Accordingly, the invention provides an isolated monoclonal antibody optimized for expression in a mammalian cell comprising a heavy chain variable region and a light chain variable region wherein: the heavy chain variable region comprises an amino acid sequence selected from any of SEQ ID NOs: 11, 12, 13 and 39, and conservative modifications thereof; and the light chain variable region comprises an amino acid sequence selected from any of SEQ ID NOs: 23, 24, 25 and 49, and conservative modifications thereof; wherein the antibody specifically binds to CD137 and is capable of activating CD 137 signaling with or without additional cross-linking.

In one embodiment, an antibody of the invention is optimized for expression in a mammalian cell has a full length heavy chain sequence and a full length light chain sequence, wherein one or more of these sequences have specified amino acid sequences based on the antibodies described herein or conservative modifications thereof, and wherein the antibodies retain the desired functional properties of the CD137-binding antibodies and antigen-binding fragments thereof of the invention.

As used herein, the term, "optimized" means that a nucleotide sequence has been altered to encode an amino acid sequence using codons that are preferred in the production cell or organism, generally a eukaryotic cell, for example, a cell of Pichia, a Chinese Hamster Ovary cell (CHO) or a human cell. The optimized nucleotide sequence is engineered to retain completely or as much as possible the amino acid sequence originally encoded by the starting nucleotide sequence, which is also known as the "parental" sequence. The optimized sequences herein have been engineered to have codons that are preferred in mammalian cells. However, optimized expression of these sequences in other eukaryotic cells or prokaryotic cells is also envisioned herein. The amino acid sequences encoded by optimized nucleotide sequences are also referred to as optimized.

Another type of variable region modification is to mutate amino acid residues within the VH and/or VL CDR1, CDR2 and/or CDR3 regions to thereby improve one or more binding properties (e.g., affinity) of the antibody of interest, known as "affinity maturation." Site-directed mutagenesis or PCR-mediated mutagenesis can be performed to introduce the mutation (s) and the effect on antibody binding, or other functional property of interest, can be evaluated in in vitro or in vivo assays as described herein and provided in the Examples. Conservative modifications (as discussed above) can be introduced. The mutations may be amino acid substitutions, additions or deletions. Moreover, typically no more than one, two, three, four or five residues within a CDR region are altered.

An "affinity-matured" antibody is one with one or more alterations in one or more variable domains thereof that result in an improvement in the affinity of the antibody for antigen, compared to a parent antibody that does not possess those alteration(s). In one embodiment, an affinity-matured antibody has nanomolar or even picomolar affinities for the target antigen. Affinity-matured antibodies are produced by procedures known in the art. For example, Marks et al, Bio/Technology 10:779-783 (1992) describes affinity maturation by VH- and VL-domain shuffling. Random mutagenesis of HVR and/or framework residues is described by, for example: Barbas et al. Proc Nat. Acad. ScL USA 91:3809-3813 (1994); Schier et al. Gene 169:147-155 (1995); Jackson et al, J. Immunol. 154(7):3310- 9 (1995); and Hawkins et al, J. Mol. Biol. 226:889-896 (1992).

In one embodiment, an "affinity-matured" antibody of the invention comprises: a VH4 comprising I44V; F89V; Y105F mutations, in particular comprising an amino acid sequence according to SEQ ID NO: 12; and a VL comprising A51P mutation, in particular comprising an amino acid sequence according to SEQ ID NO: 24.

In another embodiment, an "affinity-matured" antibody of the invention comprises: a VH4 comprising V25A; I44V; V82K; F89V; Y105F mutations, in particular comprising an amino acid sequence according to SEQ ID NO: 13; and a VL comprising I2L; A51P mutations, in particular comprising an amino acid sequence according to SEQ ID NO: 25.

An antibody of the invention further can be prepared using an antibody having one or more of the VH and/or VL sequences shown herein as starting material to engineer a modified antibody, which modified antibody may have altered properties from the starting antibody. An antibody can be engineered by modifying one or more residues within one or both variable regions (i.e., VH and/or VL), for example within one or more CDR regions and/or within one or more framework regions. Additionally or alternatively, an antibody can be engineered by modifying residues within the constant region(s), for example to alter the effector function(s) of the antibody.

One type of variable region engineering that can be performed is CDR grafting. Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al., 1998 Nature 332:323-327; Jones, P. et al., 1986 Nature 321:522- 525; Queen, C. et al., 1989 Proc. Natl. Acad., U.S.A. 86: 10029-10033; U.S. Pat. No. 5,225,539 to Winter, and U.S. Pat. Nos. 5,530,101; 5,585,089; 5,693,762 and 6,180,370 to Queen et al.).

Such framework sequences can be obtained from public DNA databases or published references that include germine antibody gene sequences or rearranged antibody sequences. For example, germine DNA sequences for human heavy and light chain variable region genes can be found in the "VBase" human germline sequence database (available on the Internet at www.mrc-cpe.cam.ac.uk/vbase), as well as in Kabat, E. A., et al., 1991 Sequences of Proteins of Immunological Interest, Fifth Edition, U.S. Department of Health and Human Services, NIH Publication No. 91-3242; Tomlinson, I. M., et al., 1992 J. fol. Biol. 227:776-798; and Cox, J. P. L. et al., 1994 Eur. J Immunol. 24:827-836; the contents of each of which are expressly incorporated herein by reference. For example, germline DNA sequences for human heavy and light chain variable region genes and rearranged antibody sequences can be found in "IMGT" database (available on the Internet at www.imgt.org; see Lefranc, M.P. et al., 1999 Nucleic Acids Res. 27:209-212; the contents of each of which are expressly incorporated herein by reference).

An example of framework sequences for use in the antibodies and antigen- binding fragments thereof of the invention are those that are structurally similar to the framework sequences used by selected antibodies and antigen-binding fragments thereof of the invention, e.g., consensus sequences and/or framework sequences used by monoclonal antibodies of the invention. The VH CDR1, 2 and 3 sequences, and the VL CDR1, 2 and 3 sequences, can be grafted onto framework regions that have the identical sequence as that found in the germline immunoglobulin gene from which the framework sequence derive, or the CDR sequences can be grafted onto framework regions that contain one or more mutations as compared to the germline sequences. For example, it has been found that in certain instances it is beneficial to mutate residues within the framework regions to maintain or enhance the antigen binding ability of the antibody (see e.g., U.S. Pat. Nos. 5,530,101; 5,585,089; 5,693,762 and 6,180,370 to Queen et al).

A wide variety of antibody /immunoglobulin frameworks or scaffolds can be employed so long as the resulting polypeptide includes at least one binding region which specifically binds to CD137. Such frameworks or scaffolds include the 5 main idiotypes of human immunoglobulins, antigen-binding fragments thereof, and include immunoglobulins of other animal species, preferably having humanized aspects.

In one aspect, the invention pertains to a method of generating non-immunoglobulin based antibodies using non-immunoglobulin scaffolds onto which CDRs of the invention can be grafted. Known or future non-immunoglobulin frameworks and scaffolds may be employed, as long as they comprise a binding region specific for the target CD137 protein. Known non-immunoglobulin frameworks or scaffolds include, but are not limited to, fibronectin (Compound Therapeutics, Inc., Waltham, Mass.), ankyrin (Molecular Partners AG, Zurich, Switzerland), domain antibodies (Domantis, Ltd., Cambridge, Mass., and Ablynx nv, Zwijnaarde, Belgium), lipocalin (Pieris Proteolab AG, Freising, Germany), small modular immuno-pharmaceuticals (Trubion Pharmaceuticals Inc., Seattle, Wash.), maxybodies (Avidia, Inc., Mountain View, Calif), Protein A (Affibody AG, Sweden), and affilin (gamma-crystallin or ubiquitin) (Scil Proteins GmbH, Halle, Germany).

Suitably, the antibodies of the invention or antigen-binding fragment thereof specifically bind to CD137 and is characterized by one or more of the following parameters:
(i) binds to human CD137 with a dissociation constant (KD) of less than 50nM, particularly less than 10 nM, particularly less than 5 nM, particularly less than 1nM, particularly less than 500 pM, more particularly less than 100 pM, more particularly less than 50 pM;
(ii) binds to human CD137 with a K_{off} rate of 10⁻³ s⁻¹ or less, or 10⁻⁴ s⁻¹ or less, or 10⁻⁵ s⁻¹ or less as measured by SPR;
(iii) binds to human CD137 with a Kₒₙ rate of at least 10⁴ M⁻¹s⁻¹ or greater, at least 10⁵ M⁻¹ s⁻¹ or greater, at least 10⁶ M⁻¹ s⁻¹ or greater, as measured by SPR;
(iv)does not cross-compete with urelumab and utolimumab; and/or
(v) is cross-reactive with Macaca fascicularis (Cynomolgus) CD137;

The term "avidity" refers to an informative measure of the overall stability or strength of the antibody-antigen complex. It is controlled by three major factors: antibody epitope affinity; the valency of both the antigen and antibody; and the structural arrangement of the interacting parts. Ultimately these factors define the specificity of the antibody, that is, the likelihood that the particular antibody is binding to a precise antigen epitope.

As used herein, the term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the sum total of non-covalent interactions between a single binding site of a molecule (e.g., of an antibody) and its binding partner (e.g., an antigen). Unless indicated otherwise, as used herein, "binding affinity", "bind to", "binds to" or "binding to" refers to intrinsic binding affinity that reflects a 1:1 interaction between members of a binding pair (e.g., an antibody fragment and antigen). The affinity of a molecule X for its partner Y can generally be represented by the dissociation constant (K_{D}). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative and exemplary embodiments for measuring binding affinity, i.e. binding strength are described in the following.

The term "K_{assoc}", "Ka" or "Kₒₙ", as used herein, is intended to refer to the association rate of a particular antibody-antigen interaction, whereas the term "K_{dis}", "Kd" or "K_{off}", as used herein, is intended to refer to the dissociation rate of a particular antibody- antigen interaction. In one embodiment, the term "KD", as used herein, is intended to refer to the dissociation constant, which is obtained from the ratio of Kd to Ka (i.e. Kd/Ka) and is expressed as a molar concentration (M). The "KD" or "KD value" or "K_{D}" or "K_{D} value" according to this invention is in one embodiment measured by using surface-plasmon resonance assays using a MASS-1 SPR instrument (Sierra Sensors). To measure affinity, an antibody specific for the Fc region of rabbit IgGs (Bethyl Laboratories, Cat. No. A120-111A) is immobilized on a sensor chip (SPR-2 Affinity Sensor, High Capacity Amine, Sierra Sensors) using a standard amine-coupling procedure. Rabbit monoclonal antibodies in B-cell supernatants are captured by the immobilized anti-rabbit IgG antibody. A minimal IgG concentration in the B-cell supernatants is required to allow sufficient capture. After capturing of the monoclonal antibodies, human CD137 ECD (Peprotech, cat. 310-15-1MG) is injected into the flow cells for 3 min at a concentration of 90 nM, and dissociation of the protein from the IgG captured on the sensor chip was allowed to proceed for 5 min. After each injection cycle, surfaces are regenerated with two injections of 10 mM Glycine-HCl. The apparent dissociation (kd) and association (ka) rate constants and the apparent dissociation equilibrium constant (KD) are calculated with the MASS-1 analysis software (Analyzer, Sierra Sensors) using one-to-one Langmuir binding model and quality of the fits is monitored based on relative Chi² (Chi² normalized to the extrapolated maximal binding level of the analyte), which is a measure for the quality of the curve fitting. The smaller the value for the Chi² the more accurate is the fitting to the one-to-one Langmuir binding model. Results are deemed valid if the response units (RU) for ligand binding are at least 2% of the RUs for antibody capturing. Samples with RUs for ligand binding with less than 2% of the RUs for antibody capturing are considered to show no specific binding of CD137 to the captured antibody. The equilibrium dissociation constant (K_{D}) is calculated as the ratio k_{off}/kₒₙ. See, e.g., Chen et al, J. Mol. Biol. 293:865-881 (1999).

Suitably, the affinity of the antibody of the invention or antigen-binding fragment thereof to CD137 may be comparable to or higher than the affinity of CD137L to CD 137. Suitably, the affinity of the antibody of the invention or antigen-binding fragment thereof to CD137 may be comparable to or higher than the affinity of urelumab to CD137. It will be appreciated that the higher affinity of the CD137 binding domain may be particularly suitable for use in an antibody, wherein said antibody is monovalent for CD137. The binding affinity of an antibody or binding fragment thereof, may be determined, for example, by the dissociation constant (KD). A stronger affinity is represented by a lower KD, while a weaker affinity is represented by a higher KD.

Thus, in a suitable embodiment, the antibody of the invention or antigen-binding fragment thereof may have a KD of between 5 to 50000 pM, 5 to 40000 pM, 5 to 30000 pM, 5 to 20000 pM, 5 to 10000 pM, 5 to 5000 pM, 5 to 2500 pM, 5 to 1000 pM, 5 to 750 pM, 5 to 500 pM, 5 to 250 pM, 5 to 100 pM, 5 to 75 pM, 5 to 50 pM, 5 to 30 pM. In a further embodiment, the multispecific antibody of the invention or the CD137-BD of the invention binds to human CD137 with a KD of between 10nM and 10pM preferably between 10nM and 0.1nM, more preferably between 5nM and 1nM.

In a suitable embodiment, the antibody of the invention or antigen-binding fragment thereof may have a KD of less than approximately 50nM, less than approximately 45nM, less than approximately 40nM, less than approximately 35nM, less than approximately 30nM, less than approximately 25nM, less than 20nM, less than approximately 15nM, less than approximately 10nM, less than approximately 9nM, less than approximately 8nM, less than approximately 7nM, less than approximately 6nM, less than approximately 5nM, less than approximately 4nM, less than approximately 3nM, less than 2nM, less than 1nM, less than 0.5nM, less than 0.25nM, or less than 0.1nM, less than 50pM, less than 40pM, less than 30pM, less than 20pM. Suitably, the antibody of the invention or antigen-binding fragment thereof has a KD of less than 10nM. Preferably, the multispecific antibody of the invention or the CD137-BD of the invention binds to human CD137 with a KD of less than 5nM. Suitably, the antibody of the invention or antigen-binding fragment thereof has a KD of less than 1nM. Suitably, the antibody of the invention or antigen-binding fragment thereof has a KD of less than 50pM.

Suitably, the antibody of the invention or antigen-binding fragment thereof binds to human CD137 with a Kₒₙ rate of at least 10³ M⁻¹s⁻¹ or greater, at least 10⁴ M⁻¹s⁻¹ or greater, at least 5x10⁴ M⁻¹s⁻¹ or greater, at least 10⁵ M⁻¹s⁻¹ or greater, at least 5x10⁵ M⁻¹s⁻¹ or greater, at least 10⁶ M⁻¹ s⁻¹ or greater, at least 5x10⁶ M⁻¹ s⁻¹ or greater, at least 10⁷ M⁻¹ s⁻¹ or greater, at least 5x10⁷ M-¹s-¹ or greater as measured by surface plasmon resonance (SPR). Suitably, the antibody of the invention or antigen-binding fragment thereof has a Kₒₙ rate of at least 10⁴ M⁻¹s⁻¹ or greater, in particular at least 10⁵ M⁻¹s-¹ or greater, as measured by SPR.

Suitably, the antibody of the invention or antigen-binding fragment thereof binds to human CD137 with a K_{off} rate of 10⁻³ s⁻¹ or less, 3x10⁻³ s⁻¹ or less, 5x10⁻³ s⁻¹ or less, 10⁻⁴ s⁻¹ or less, 5x10⁻⁴ s⁻¹ or less, 10⁻⁵ s⁻¹ or less, 5x10⁻⁵ s⁻¹ or less, 10⁻⁶ s⁻¹ or less, or 10⁻⁷ s⁻¹ or less as measured by surface plasmon resonance (SPR). Suitably, the antibody of the invention or antigen-binding fragment thereof has a K_{off} rate of 10⁻⁴ s⁻¹ or less, in particular 10⁻⁵ s⁻¹ or less as measured by SPR.

Suitably, the antibody of the invention or antigen-binding fragment thereof a binding to human CD137 of at least 60% or greater, at least 70% or greater, at least 75% or greater, at least 80% of greater, at least 85% or greater, at least 90% or greater, at least 95% or greater, as measured with SPR.

In one embodiment, an antibody of the invention or antigen-binding fragment thereof does not cross-compete for binding with urelumab. The present invention provides an antibody or antigen-binding fragment thereof that binds to a different epitope than urelumab. Urelumab, also referred to as BMS-663513, is a fully humanized IgG4 mAb from Bristol-Myers Squibb, and is described in WO04/010947, US 6,887,673 and US 7,214,493, which are hereby incorporated into the present application by reference in their entirety.

In one embodiment, an antibody of the invention or antigen-binding fragment thereof does not cross-compete for binding with utomilumab. The present invention provides an antibody or antigen-binding fragment thereof that binds to a different epitope than utomilumab. Utomilumab, also referred to as PF-05082566, is a fully human IgG2 mAb from Pfizer, and is described in WO12/032433 and US 8,821,867, which is hereby incorporated into the present application by reference in its entirety.

In a further embodiment, an antibody of the invention or antigen-binding fragment thereof does not cross-compete for binding with urelumab and utomilumab. The present invention provides an antibody or antigen-binding fragment thereof that binds to a different epitope than urelumab and utomilumab.

The term "recognize" as used herein refers to an antibody antigen-binding fragment thereof that finds and interacts (e.g., binds) with its conformational epitope.

The terms "compete" or "cross-compete" and related terms are used interchangeably herein to mean the ability of an antibody or other binding agent to interfere with the binding of other antibodies or binding agents to CD137 in a standard competitive binding assay.

The ability or extent to which an antibody or other binding agent is able to interfere with the binding of another antibody or binding molecule to CD 137, and therefore whether it can be said to cross-compete according to the invention, can be determined using standard competition binding assays. One particularly suitable quantitative cross-competition assay uses a FACS- or an AlphaScreen-based approach to measure competition between the labelled (e.g. His tagged, biotinylated or radioactive labelled) an antibody or fragment thereof and the other an antibody or fragment thereof in terms of their binding to the target. In general, a cross-competing antibody or fragment thereof is for example one which will bind to the target in the cross-competition assay such that, during the assay and in the presence of a second antibody or fragment thereof, the recorded displacement of the immunoglobulin single variable domain or polypeptide according to the invention is up to 100% (e.g. in FACS based competition assay) of the maximum theoretical displacement (e.g. displacement by cold (e.g. unlabeled) antibody or fragment thereof that needs to be cross-blocked) by the to be tested potentially cross-blocking antibody or fragment thereof that is present in a given amount. Preferably, cross-competing antibodies or fragments thereof have a recorded displacement that is between 10% and 100%, more preferred between 50% to 100%.

The term "epitope" means a protein determinant capable of specific binding to an antibody. Epitopes usually consist of chemically active surface groupings of molecules such as amino acids or sugar side chains and usually have specific three dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein, and show a significantly reduced rate of HD exchange due to Fab binding. The conformation epitope contains, but is not limited to, the functional epitope.

The term "linear epitope" refers to an epitope with all of the points of interaction between the protein and the interacting molecule (such as an antibody) occurring linearly along the primary amino acid sequence of the protein (continuous).

The antibodies and antigen-binding fragments thereof of the present invention does not inhibit the binding of urelumab or utomiulumab to CD137 protein, which demonstrates that the antibody of the invention or antigen-binding fragment thereof cannot compete with urelumab or utomiulumab for binding to CD137; such an antibody may, according to non-limiting theory, bind to a different (e.g., a structurally different or spatially remote) epitope on CD137 as urelumab or utomiulumab.

The present invention also provides antibodies that bind to the same epitope as do the CD137-binding antibodies listed in Table 1. Additional antibodies can therefore be identified based on their ability to cross-compete (e.g., to competitively inhibit the binding of, in a statistically significant manner) with other antibodies and antigen-binding fragments thereof of the invention in CD137 binding assays.

The ability of a test antibody to inhibit the binding of antibodies and antigen-binding fragments thereof of the invention to CD137 protein demonstrates that the test antibody can compete with that antibody for binding to CD137; such an antibody may, according to non-limiting theory, bind to the same or a related (e.g., a structurally similar or spatially proximal) epitope on CD 137 as the antibody of the invention with which it competes. In a certain embodiment, the antibody that binds to the same epitope on CD 137 as the antibodies and antigen-binding fragments thereof of the invention is a human or humanized monoclonal antibody. Such human or humanized monoclonal antibodies can be prepared and isolated as described herein.

Once a desired epitope on an antigen is determined, it is possible to generate antibodies to that epitope, e.g., using the techniques described in the present invention. Alternatively, during the discovery process, the generation and characterization of antibodies may elucidate information about desirable epitopes. From this information, it is then possible to competitively screen antibodies for binding to the same epitope. An approach to achieve this is to conduct cross-competition studies to find antibodies that competitively bind with one another, e.g., the antibodies compete for binding to the antigen. A high throughput process for "binning" antibodies based upon their cross-competition is described in International Patent Application No. WO 2003/48731. As will be appreciated by one of skill in the art, practically anything to which an antibody can specifically bind could be an epitope. An epitope can comprise those residues to which the antibody binds.

Regions of a given polypeptide that include an epitope can be identified using any number of epitope mapping techniques, well known in the art. See, e.g., Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al., (1984) Proc. Natl. Acad. Sci. USA 8:3998-4002; Geysen et al., (1985) Proc. Natl. Acad. Sci. USA 82:78-182; Geysen et al., (1986) Mol. Immunol. 23:709-715. Similarly, conformational epitopes are readily identified by determining spatial conformation of amino acids CD137 such as by, e.g., hydrogen/deuterium exchange, x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al., (1981) Proc. Natl. Acad. Sci USA 78:3824-3828; for determining antigenicity profiles, and the Kyte-Doolittle technique, Kyte et al., (1982) J.MoI. Biol. 157: 105-132; for hydropathy plots.

Suitably, the isolated antibody of the present invention or antigen-binding fragment thereof is selected from the group consisting of: a monoclonal antibody, a chimeric antibody, an IgG antibody, a Fab, an Fv, a scFv, dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology).

Suitably, the isolated antibody of the invention or antigen-binding fragment thereof is an Fv. Suitably, the isolated antibody of the invention or antigen-binding fragment thereof is scFv antibody fragment. "Single-chain Fv" or "scFv" or "sFv" antibody fragments comprise the VH and VL domains of an antibody, wherein these domains are present in a single polypeptide chain. Generally, the Fv polypeptide further comprises a polypeptide linker between the VH and VL domains which enables the sFv to form the desired structure for target binding. "Single-chain Fv" or "scFv" antibody fragments comprise the VH and VL domains of antibody, wherein these domains are present in a single polypeptide chain. Generally, the scFv polypeptides further comprises a polypeptide linker between the VH and VL domains which enables the scFv to form the desired structure for antigen binding (see, for example, Plückthun, The pharmacology of Monoclonal Antibodies, vol. 113, Rosenburg and Moore eds., (Springer-Verlag, New York, 1994), pp. 269-315). In particular embodiments, said functional fragment is an scFv format comprising the linker according to SEQ ID NO: 26. In a further embodiment, the isolated antibody of the invention or antigen-binding fragment thereof is a single-chain variable fragment (scFv) as shown in SEQ ID NO:27, SEQ ID NO: 28, SEQ ID NO: 29 or SEQ ID NO: 51. In one embodiment, the isolated antibody of the invention or antigen-binding fragment thereof is a single-chain variable fragment (scFv) as shown in SEQ ID NO: 28. In one embodiment, the isolated antibody of the invention or antigen-binding fragment thereof is a single-chain variable fragment (scFv) as shown in SEQ ID NO: 29.

Suitably, the isolated antibody of the invention or antigen-binding fragment thereof is IgG antibody fragment. The term "isotype" refers to the antibody class (e.g., IgM, IgE, IgG such as IgG1 or IgG4) that is provided by the heavy chain constant region genes. Isotype also includes modified versions of one of these classes, where modifications have been made to after the Fc function, for example, to enhance or reduce effector functions or binding to Fc receptors. In one embodiment, the isolated antibody of the invention or antigen-binding fragment thereof is IgG selected from the group consisting of an IgG1, an IgG2, an IgG3 and an IgG4, preferably IgG4.

In another particular embodiment, the isolated antibody of the invention or antigen-binding fragment thereof is a multispecific molecule, e.g., bispecific molecule, trispecific molecule, tetraspecific, pentaspecific, hexaspecific molecule, comprising a CD137 antigen-binding fragment of the antibody of the invention.

The term "multispecific molecule" or "multispecific antibody" as used herein, refers to an antibody that binds to two or more different epitopes on at least two or more different targets (e.g., CD137 and another target different from CD137). The term "multispecific molecule" includes bispecific, trispecific, tetraspecific, pentaspecific and hexaspecific antibodies. The term "bispecific antibody" as used herein, refers to an antibody that binds to two different epitopes on two different targets. The term "trispecific antibody" as used herein, refers to an antibody that binds to three different epitopes on three different targets.

An antibody of the invention, or antigen-binding regions thereof, can be derivatized or linked to another functional molecule, e.g., another peptide or protein (e.g., another antibody or ligand for a receptor) to generate a multispecific molecule that binds to at least two binding sites and/or different target molecules. The antibody of the invention may in fact be derivatized or linked to more than one other functional molecule to generate multispecific molecules that bind to more than two different binding sites and/or target molecules. To create a multispecific molecule of the invention, an antibody of the invention can be functionally linked (e.g., by chemical coupling, genetic fusion, noncovalent association or otherwise) to one or more other binding molecules, such as another antibody, antibody fragment, peptide or binding mimetic, such that a multispecific molecule results.

Accordingly, the present invention includes multispecific molecules comprising at least one first binding specificity for CD137 and a second binding specificity for a second target epitope. For example, the second target epitope is present on another target molecule different from CD 137.

Bivalent CD137 antibodies were shown to be generally weak in their ability to induce the signaling in the absence of an exogenous clustering. To illustrate, anti-CD137 antibody utomilumab is only capable to activate CD 137 signaling when either cross-linked to anti-human F(ab')2 secondary antibody or immobilized to tissue culture plastic (Fisher at al., Cancer Immunol Immunother 61:1721-1733 (2012)). Studies in rodent agonistic antibodies to CD40 (TNFRSF5), another member of TNFRSF, have suggested that the exogenous clustering can be partially achieved through the interaction with Fcγ-receptor (Li F, Ravetch JV, Science 333(6045):1030-10 (2011); White AL, et al., J Immunol 187(4):1754-1763 (2011)). The interaction with Fcγ-receptor can however deplete the CD137-expressing cells through effector mechanisms. The current bivalent antibodies targeting CD137 are thus either ineffective agonists or lead to depleting of CD137-positive cells. Suitably, second binding specificity of the multispecific molecule is capable of providing additional cross-linking of the CD137-binding antibody of the present invention or antigen-binding fragment thereof. Accordingly, the present invention includes multispecific molecules comprising at least one first binding specificity for CD137 and a second binding specificity for a second target epitope. For example, the second target epitope is another epitope of CD 13 7 different from the first target epitope. The multispecific molecule can further include a third binding specificity, in addition to the first and second target epitope.

In a further embodiment, the present invention includes multispecific molecules monovalent, bivalent or multivalent for CD137 specificity, preferably monovalent.

In another particular embodiment of the present invention, the isolated antibody of the present invention or antigen-binding fragment thereof is a monovalent or multivalent for CD137 specificity molecule, e.g., bivalent, trivalent, tetravalent, pentavalent, hexavalent.

The term "monovalent molecule" or "monovalent antibody", as used herein, refers to an antibody that binds to a single epitope on a target molecule, such as CD137.

The term "bivalent antibody" as used herein, refers to an antibody that binds to two epitopes on at least two identical target molecules, such as CD137 target molecules. A "bivalent antibody" also refers to an antibody that binds to two different epitopes on at least two identical target molecules, such as CD137 target molecules. The bivalent antibody may crosslink the target CD137 molecules to one another.

The term "multivalent antibody" refers to a single binding molecule with more than one valency, where "valency" is described as the number of antigen-binding moieties that binds to epitopes on identical target molecules. As such, the single binding molecule can bind to more than one binding site on a target molecule. Examples of multivalent antibodies include, but are not limited to bivalent antibodies, trivalent antibodies, tetravalent antibodies, pentavalent antibodies, and the like.

Suitable, the isolated antibody of the present invention or antigen-binding fragment thereof is a multispecific molecule, e.g., bispecific molecule, and / or a multivalent molecule, e.g., monovalent for CD137 specificity molecule, bivalent for CD137 specificity molecule, which is an antibody format selected from any suitable multispecific, e.g. bispecific, format known in the art, including, by way of non-limiting example, formats based on a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)2) or bibody (Fab-(scFv)1), Fab,, Fab-Fv2, Morrison (IgG CH₃-scFv fusion (Morrison L) or IgG CL-scFv fusion (Morrison H)), triabody (scDb-scFv), bispecific Fab2, di-miniantibody, tetrabody, scFv-Fc-scFv fusion, scFv-HSA-scFv fusion, di-diabody, DVD-Ig, COVD, IgG-scFab, scFab-dsscFv, Fv2-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs) (bispecific IgGs prepared by the KiH technology); an Fv, scFv, scDb, tandem-di-scFv, tandem tri-scFv, Fab-(scFv)2, Fab-(scFv)1, Fab, Fab-Fv2, COVD fused to the N- and/or the C-terminus of either chain of a heterodimeric Fc domain or any other heterodimerization domain, a MATCH (described in WO2016/0202457; Egan T., et al., mAbs 9 (2017) 68-84) and DuoBodies(bispecific IgGs prepared by the Duobody technology) (MAbs. 2017 Feb/Mar;9(2):182-212. doi: 10.1080/19420862.2016.1268307).

The term "diabodies" refers to antibody fragments with two antigen-binding sites, which fragments comprise a VH connected to VL in the same polypeptide chain (VH-VL). By using a linker that is too short to allow pairing between the two domains on the same chain, the domains are forced to pair with the complementary domains of another chain to create two antigen-binding sites. Diabodies may be bivalent or bispecific. Diabodies are described more fully in, for example, EP404097, WO1993/01161, Hudson et al., Nat. Med. 9:129-134 (2003), and Hollinger et al., Proc. Natl. Acad. Sci. USA 90: 6444-6448 (1993). Triabodies and tetrabodies are also described in Hudson et al., Nat. Med. 9:129-134 (2003).

The bispecific scDb, in particular the bispecific monomeric scDb, particularly comprises two variable heavy chain domains (VH) or fragments thereof and two variable light chain domains (VL) or fragments thereof connected by linkers L1, L2 and L3 in the order VHA-L1-VLB-L2-VHB-L3-VLA, VHA-L1-VHB-L2-VLB-L3-VLA, VLA-L1-VLB-L2-VHB-L3-VHA, VLA-L1-VHB-L2-VLB-L3-VHA, VHB-L1-VLA-L2-VHA-L3-VLB, VHB-L1-VHA-L2-VLA-L3-VLB, VLB-L1-VLA-L2-VHA-L3-VHB or VLB-L1-VHA-L2-VLA-L3-VHB, wherein the VLA and VHA domains jointly form the antigen binding site for the first antigen, and VLB and VHB jointly form the antigen binding site for the second antigen.

The linker L1 particularly is a peptide of 2-10 amino acids, more particularly 3-7 amino acids, and most particularly 5 amino acids, and linker L3 particularly is a peptide of 1-10 amino acids, more particularly 2-7 amino acids, and most particularly 5 amino acids. The middle linker L2 particularly is a peptide of 10-40 amino acids, more particularly 15-30 amino acids, and most particularly 20-25 amino acids.

In one embodiment of the present invention, the isolated antibody or antigen-binding fragment thereof is a multispecific and/or multivalent antibody in a MATCH format described in WO2016/0202457; Egan T., et al., mAbs 9 (2017) 68-84.

Multispecific and/or multivalent molecules of the present invention can be produced using any convenient antibody manufacturing method known in the art (see, e.g., Fischer, N. & Leger, O., Pathobiology 74 (2007) 3-14 with regard to the production of bispecific constructs; Hornig, N. & Färber-Schwarz, A., Methods Mol. Biol. 907 (2012)713-727, and WO 99/57150 with regard to bispecific diabodies and tandem scFvs). Specific examples of suitable methods for the preparation of the bispecific construct of the present invention further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, e.g., Zhu et al., Cancer Lett. 86 (1994) 127-134); and Suresh et al., Methods Enzymol. 121 (1986) 210-228).

Other antibodies which can be employed in the multispecific and in the multvalent molecules of the invention are murine, chimeric and humanized monoclonal antibodies.

The multispecific molecules of the present invention can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N-succinimidyl-3- (2-pyridyldithio)propionate (SPDP), and sulfosuccinimidyl 4- (N-maleimidomethyl)cyclohaxane-1-carboxylate (sulfo-SMCC) (see e.g., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83), and Glennie et al., 1987 J. Immunol. 139: 2367-2375). Conjugating agents are SATA and sulfo-SMCC, both available from Pierce Chemical Co. (Rockford, 111.).

When the binding specificities are antibodies, they can be conjugated by sulfhydryl bonding of the C-terminus hinge regions of the two heavy chains. In a particularly embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb X mAb, mAb X Fab, Fab X F (ab')2 or ligand X Fab fusion protein. A multispecific molecule of the invention can be a single chain molecule comprising one single chain antibody and a binding determinant, or a single chain multispecific molecule comprising two binding determinants. Multispecific molecules may comprise at least two single chain molecules. Methods for preparing multispecific molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the bispecific molecules to their specific targets can be confirmed by, for example, enzyme-linked immunosorbent assay (ELISA), radioimmunoassay (REA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest.

In a further aspect, the invention provides a nucleic acid encoding the antibody of the invention or antigen-binding fragment thereof. The present invention also provides nucleic acid sequences that encode CDRs, VH, VL, the full length heavy chain, and the full length light chain of the antibodies and antigen- binding fragments thereof that specifically bind to CD137 protein. Such nucleic acid sequences can be optimized for expression in mammalian cells.

The term "nucleic acid" is used herein interchangeably with the term "polynucleotide" and refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The invention provides substantially purified nucleic acid molecules which encode polypeptides comprising segments or domains of the CD137-binding antibody chains described above. When expressed from appropriate expression vectors, polypeptides encoded by these nucleic acid molecules are capable of exhibiting CD137 antigen binding capacity.

Also provided in the invention are polynucleotides which encode at least one CDR region and usually all three CDR regions from the heavy or light chain of the CD137-binding antibody set forth in Table 1. Some other polynucleotides encode all or substantially all of the variable region sequence of the heavy chain and/or the light chain of the CD137-binding antibody set forth in Table 1. Because of the degeneracy of the code, a variety of nucleic acid sequences will encode each of the immunoglobulin amino acid sequences.

The polynucleotide sequences can be produced by de novo solid-phase DNA synthesis or by PCR mutagenesis of an existing sequence (e.g., sequences as described in the Examples below) encoding a CD137-binding antibody or its binding fragment. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetra. Lett., 22: 1859, 1981; and the solid support method of U.S. Pat. No. 4,458,066. Introducing mutations to a polynucleotide sequence by PCR can be performed as described in, e.g., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the invention are expression vectors and host cells for producing the CD137-binding antibodies described above.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses and adeno- associated viruses), which serve equivalent functions.

The term "operably linked" refers to a functional relationship between two or more polynucleotide (e.g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis-acting. However, some transcriptional regulatory sequences, such as enhancers, need not be physically contiguous or located in close proximity to the coding sequences whose transcription they enhance.

Various expression vectors can be employed to express the polynucleotides encoding the CD137-binding antibody chains or binding fragments. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, e.g., Harrington et al., Nat Genet. 15:345, 1997). For example, nonviral vectors useful for expression of the CD137-binding polynucleotides and polypeptides in mammalian (e.g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adenoassociated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (e.g., enhancers) that are operably linked to the polynucleotides encoding a CD137-binding antibody chain antigen-binding fragment. In one embodiment, an inducible promoter is employed to prevent expression of inserted sequences except under inducing conditions. Inducible promoters include, e.g., arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of a CD137-binding antibody chain antigen-binding fragment. These elements typically include an ATG initiation codon and adjacent ribosome binding site or other sequences. In addition, the efficiency of expression may be enhanced by the inclusion of enhancers appropriate to the cell system in use (see, e.g., Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

The expression vectors may also provide a secretion signal sequence position to form a fusion protein with polypeptides encoded by inserted CD137-binding antibody sequences. More often, the inserted CD137-binding antibody sequences are linked to signal sequences before inclusion in the vector. Vectors to be used to receive sequences encoding CD137-binding antibody light and heavy chain variable domains sometimes also encode constant regions or parts thereof. Such vectors allow expression of the variable regions as fusion proteins with the constant regions thereby leading to production of intact antibodies and antigen-binding fragments thereof. Typically, such constant regions are human.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the CD137-binding antibody chains can be either prokaryotic or eukaryotic. E. coli is one prokaryotic host useful for cloning and expressing the polynucleotides of the present invention. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express CD137-binding polypeptides of the invention. Insect cells in combination with baculovirus vectors can also be used.

In one embodiment, mammalian host cells are used to express and produce the CD137-binding polypeptides of the present invention. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, e.g., Winnacker, FROM GENES TO CLONES, VCH Publishers, N.Y., N.Y., 1987. Expression vectors for mammalian host cells can include expression control sequences, such as an origin of replication, a promoter, and an enhancer (see, e.g., Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP poIIII promoter, the constitutive MPS V promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, et al., supra). Other methods include, e.g., electroporation, calcium phosphate treatment, liposome-mediated transformation, injection and microinjection, ballistic methods, virosomes, immunoliposomes, polycatiomnucleic acid conjugates, naked DNA, artificial virions, fusion to the herpes virus structural protein VP22 (Elliot and O'Hare, Cell 88:223, 1997), agent-enhanced uptake of DNA, and ex vivo transduction. For long-term, high-yield production of recombinant proteins, stable expression will often be desired. For example, cell lines which stably express CD137-binding antibody chains or binding fragments can be prepared using expression vectors of the invention which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present invention thus provides a method of producing the antibody of the invention or antigen-binding fragment thereof, wherein said method comprises the step of culturing a host cell expressing a nucleic acid encoding the antibody of the invention or antigen-binding fragment thereof.

In a further aspect, the present invention relates to a pharmaceutical composition comprising the antibody or antigen-binding fragment thereof of the present invention, and a pharmaceutically acceptable carrier. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

A pharmaceutical composition of the present invention can be administered by a variety of methods known in the art. The route and/or mode of administration vary depending upon the desired results. Administration can be intravenous, intramuscular, intraperitoneal, or subcutaneous, or administered proximal to the site of the target. The pharmaceutically acceptable carrier should be suitable for intravenous, intramuscular, subcutaneous, parenteral, spinal or epidermal administration (e.g., by injection or infusion). Depending on the route of administration, the active compound, i.e., antibody, and multispecific molecule, may be coated in a material to protect the compound from the action of acids and other natural conditions that may inactivate the compound.

Pharmaceutical compositions of the invention can be prepared in accordance with methods well known and routinely practiced in the art. See, e.g., Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the CD137-binding antibody is employed in the pharmaceutical compositions of the invention. The CD137-binding antibodies are formulated into pharmaceutically acceptable dosage forms by conventional methods known to those of skill in the art. Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the present invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

Antibody is usually administered on multiple occasions. Intervals between single dosages can be weekly, monthly or yearly. Intervals can also be irregular as indicated by measuring blood levels of CD137-binding antibody in the patient. Alternatively, antibody can be administered as a sustained release formulation, in which case less frequent administration is required. Dosage and frequency vary depending on the half-life of the antibody in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In one aspect, the present invention relates to the antibody of the present invention or antigen-binding fragment thereof, or the multispecific molecule of the present invention, or the composition of the present invention for use as a medicament.

In another aspect, the present invention relates to the antibody of the present invention or antigen-binding fragment thereof, or the multispecific molecule of the present invention, or the composition of the present invention for use in a manufacture of a medicament.

In one aspect, the present invention relates to the antibody of the present invention or antigen-binding fragment thereof, or the multispecific molecule of the present invention, or the composition of the present invention for use in treating a cancer in a subject in need thereof.

In another aspect, the present invention relates to use of the antibody of the present invention or antigen-binding fragment thereof, or the multispecific molecule of the present invention, or the composition of the present invention to treat a cancer in a subject in need thereof.

In a further aspect, the present invention relates to use of the antibody of the present invention or antigen-binding fragment thereof, or the multispecific molecule of the present invention, or the composition of the present in the manufacture of a medicament for treatment of a cancer, in a subject in need thereof.

In one aspect, the present invention provides a method of treating a cancer in a subject in need thereof comprising administering to the subject a therapeutically effective amount of the antibody of the invention or antigen-binding fragment thereof, the multispecific molecule of the invention, or the composition of the invention.

The term "subject" includes human and non-human animals. Non-human animals include all vertebrates, e.g., mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, e.g., in a human, and includes: (a) inhibiting the disease, i.e., arresting its development; and (c) relieving the disease, i.e., causing regression of the disease.

The term "therapeutically effective amount" or "efficacious amount" refers to the amount of an agent that, when administered to a mammal or other subject for treating a disease, is sufficient to effect such treatment for the disease. The "therapeutically effective amount" will vary depending on the agent, the disease and its severity and the age, weight, etc., of the subject to be treated.

The term "cancer" refers to a disease characterized by the rapid and uncontrolled growth of aberrant cells. Cancer cells can spread locally or through the bloodstream and lymphatic system to other parts of the body. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

**TABLE 1. Examples of CD137 antibodies of the present invention.**

| **SEQ ID NUMBER** | **Ab region** | **Sequence** |
|---|---|---|
| **38-02-A04** | | |
| SEQ ID NO: 1 (AHo) | HCDR1 | VSGFSFSNSYW |
| | (38-02-A04 sc01) | |
| | (38-02-A04 sc05 IF) | |
| SEQ ID NO: 2 (AHo) | HCDR1 | ASGFSFSNSYW |
| | (38-02-A04 sc06 Full) | |
| SEQ ID NO: 3 (AHo) | HCDR2 | TFVGSSDSTYYANWAKGR |
| | (38-02-A04 sc01) | |
| | (38-02-A04 sc05 IF) | |
| | (38-02-A04 sc06 Full) | |
| SEQ ID NO: 4 (AHo) | HCDR3 | HPSDAVYGYANN |
| | (38-02-A04 sc01) | |
| | (38-02-A04 sc05 IF) | |
| | (38-02-A04 sc06 Full) | |
| SEQ ID NO: 5 (Kabat) | HCDR1 | NSYWIC |
| | (38-02-A04 sc01) | |
| | (38-02-A04 sc05 IF) | |
| | (38-02-A04 sc06 Full) | |
| SEQ ID NO: 6 (Kabat) | HCDR2 | CTFVGSSDSTYYANWAKG |
| | (38-02-A04 sc01) | |
| | (38-02-A04 sc05 IF) | |
| | (38-02-A04 sc06 Full) | |
| SEQ ID NO: 7 (Kabat) | HCDR3 | HPSDAVYGYANNL |
| | (38-02-A04 sc01) | |
| | (38-02-A04 sc05 IF) | |
| | (38-02-A04 sc06 Full) | |
| SEQ ID NO: 8 (Chothia) | HCDR1 | GFSFSNSY |
| | (38-02-A04 sc01) | |
| | (38-02-A04 sc05 IF) | |
| | (38-02-A04 sc06 Full) | |
| SEQ ID NO: 9 (Chothia) | HCDR2 | FVGSSDS |
| | (38-02-A04 sc01) | |
| | (38-02-A04 sc05 IF) | |
| | (38-02-A04 sc06 Full) | |
| SEQ ID NO: 10 (Chothia) | HCDR3 | HPSDAVYGYANNL |
| | (38-02-A04 sc01) | |
| | (38-02-A04 sc05 IF) | |
| | (38-02-A04 sc06 Full) | |
| SEQ ID NO: 11 | VH | QVQLQESGPGLVKPSETLSLTCKVSG FSFSNSYWICWIRQPPGKGLEWIGCTF |
| | (VH4) | VGSSDSTYYANWAKGRVTISVDSSK NQFSLKLSSVTAADTAVYYCARHPS DAVYGYANNLWGQGTLVTVSS |
| | (38-02-A04 sc01) | |
| SEQ ID NO: 12 | VH | QVQLQESGPGLVKPSETLSLTCKVSG FSFSNSYWICWVRQPPGKGLEWIGCT FVGSSDSTYYANWAKGRVTISVDSSK NQVSLKLSSVTAADTAVYFCARHPS DAVYGYANNLWGQGTLVTVSS |
| | (VH4) | |
| | (38-02-A04 sc05 IF) | |
| | Mutations VH: I44V; F89V; Y105F. | |
| SEQ ID NO: 13 | VH | QVQLQESGPGLVKPSETLSLTCKASG FSFSNSYWICWVRQPPGKGLEWIGCT FVGSSDSTYYANWAKGRVTISKDSSK NQVSLKLSSVTAADTAVYFCARHPS DAVYGYANNLWGQGTLVTVSS |
| | (VH4) | |
| | (38-02-A04 sc06 Full) | |
| | Mutations VH: V25A; I44V; V82K; F89V; Y105F | |
| SEQ ID NO: 14 (AHo) | LCDR1 | ASQSINNV |
| | (38-02-A04 sc01) | |
| | (38-02-A04 sc05 IF) | |
| | (38-02-A04 sc06 Full) | |
| SEQ ID NO: 15 (AHo) | LCDR2 | RASTLASGVPSR |
| | (38-02-A04 sc01) | |
| | (38-02-A04 sc05 IF) | |
| | (38-02-A04 sc06 Full) | |
| SEQ ID NO: 16 (AHo) | LCDR3 | SYGNYG |
| | (38-02-A04 sc01) | |
| | (38-02-A04 sc05 IF) | |
| | (38-02-A04 sc06 Full) | |
| SEQ ID NO: 17 (Kabat) | LCDR1 | QASQSINNVLA |
| | (38-02-A04 sc01) | |
| | (38-02-A04 sc05 IF) | |
| | (38-02-A04 sc06 Full) | |
| SEQ ID NO: 18 (Kabat) | LCDR2 | RASTLAS |
| | (38-02-A04 sc01) | |
| | (38-02-A04 sc05 IF) | |
| | (38-02-A04 sc06 Full) | |
| SEQ ID NO: 19 (Kabat) | LCDR3 | QSSYGNYGD |
| | (38-02-A04 sc01) | |
| | (38-02-A04 sc05 IF) | |
| | (38-02-A04 sc06 Full) | |
| SEQ ID NO: 20 (Chothia) | LCDR1 | QASQSINNVLA |
| | (38-02-A04 sc01) | |
| | (38-02-A04 sc05 IF) | |
| | (38-02-A04 sc06 Full) | |
| SEQ ID NO: 21 (Chothia) | LCDR2 | RASTLAS |
| | (38-02-A04 sc01) | |
| | (38-02-A04 sc05 IF) | |
| | (38-02-A04 sc06 Full) | |
| SEQ ID NO: 22 (Chothia) | LCDR3 | QSSYGNYGD |
| | (38-02-A04 sc01) | |
| | (38-02-A04 sc05 IF) | |
| | (38-02-A04 sc06 Full) | |
| SEQ ID NO: 23 | VL | DIQMTQSPSSLSASVGDRVTITCQASQ SINNVLAWYQQKPGKAPKLLIYRAST LASGVPSRFSGSGSGTDFTLTISSLQPE DFATYYCQSSYGNYGDFGTGTKVTV LG |
| | (Vk1-sk17) | |
| | (38-02-A04 sc01) | |
| SEQ ID NO: 24 | VL | DIQMTQSPSSLSASVGDRVTITCQASQ SINNVLAWYQQKPGKPPKLLIYRAST LASGVPSRFSGSGSGTDFTLTISSLQPE DFATYYCQSSYGNYGDFGTGTKVTV LG |
| | (Vk1-sk17) | |
| | (38-02-A04 sc05 IF) | |
| | Mutations VL: A51P | |
| SEQ ID NO: 25 | VL | DLQMTQSPSSLSASVGDRVTITCQAS QSINNVLAWYQQKPGKPPKLLIYRAS TLASGVPSRFSGSGSGTDFTLTISSLQP EDFATYYCQSSYGNYGDFGTGTKVT VLG |
| | (Vk1-sk17) | |
| | (38-02-A04 sc06 Full) | |
| | Mutations VL: I2L; A51P | |
| SEQ ID NO: 26 | Linker | GGGGSGGGGSGGGGSGGGGS |
| SEQ ID NO: 27 | scFv (VL-linker-VH) | DIQMTQSPSSLSASVGDRVTITCQASQ SINNVLAWYQQKPGKAPKLLIYRAST LASGVPSRFSGSGSGTDFTLTISSLQPE DFATYYCQSSYGNYGDFGTGTKVTV LGGGGGSGGGGSGGGGSGGGGSQV QLQESGPGLVKPSETLSLTCKVSGFSF SNSYWICWIRQPPGKGLEWIGCTFVG SSDSTYYANWAKGRVTISVDSSKNQF SLKLSSVTAADTAVYYCARHPSDAV YGYANNLWGQGTLVTVSS |
| | (38-02-A04 sc01) | |
| SEQ ID NO: 28 | scFv (VL-linker-VH) | DIQMTQSPSSLSASVGDRVTITCQASQ SINNVLAWYQQKPGKPPKLLIYRAST LASGVPSRFSGSGSGTDFTLTISSLQPE DFATYYCQSSYGNYGDFGTGTKVTV LGGGGGSGGGGSGGGGSGGGGSQV |
| | (38-02-A04 sc05 IF) | QLQESGPGLVKPSETLSLTCKVSGFSF SNSYWICWVRQPPGKGLEWIGCTFV GSSDSTYYANWAKGRVTISVDSSKN QVSLKLSSVTAADTAVYFCARHPSD AVYGYANNLWGQGTLVTVSS |
| SEQ ID NO: 29 | scFv (VL-linker-VH) | DLQMTQSPSSLSASVGDRVTITCQAS QSINNVLAWYQQKPGKPPKLLIYRAS TLASGVPSRFSGSGSGTDFTLTISSLQP EDFATYYCQSSYGNYGDFGTGTKVT VLGGGGGSGGGGSGGGGSGGGGSQ VQLQESGPGLVKPSETLSLTCKASGF SFSNSYWICWVRQPPGKGLEWIGCTF VGSSDSTYYANWAKGRVTISKDSSK NQVSLKLSSVTAADTAVYFCARHPS DAVYGYANNLWGQGTLVTVSS |
| | (38-02-A04 sc06 Full) | |
| 38-27-C05 sc01 | | |
| SEQ ID NO: 30 (AHo) | HCDR1 | VSGFSFNNDYD |
| SEQ ID NO: 31 (AHo) | HCDR2 | IDTGDGSTYYASWAKGR |
| SEQ ID NO: 32 (AHo) | HCDR3 | EAASSSGYGMGYFD |
| SEQ ID NO: 33 (Kabat) | HCDR1 | NDYDMC |
| SEQ ID NO: 34 (Kabat) | HCDR2 | CIDTGDGSTYYASWAKG |
| SEQ ID NO: 35 (Kabat) | HCDR3 | EAASSSGYGMGYFDL |
| SEQ ID NO: 36 (Chothia) | HCDR1 | GFSFNNDY |
| SEQ ID NO: 37 (Chothia) | HCDR2 | DTGDGS |
| SEQ ID NO: 38 (Chothia) | HCDR3 | EAASSSGYGMGYFDL |
| SEQ ID NO: 39 | VH | |
| | (VH4) | QVQLQESGPGLVKPSETLSLTCKVSG FSFNNDYDMCWIRQPPGKGLEWIGCI DTGDGSTYYASWAKGRVTISVDSSK NQFSLKLSSVTAADTAVYYCAREAA SSSGYGMGYFDLWGQGTLVTVSS |
| SEQ ID NO: 40 (AHo) | LCDR1 | SSQSVYDNNW |
| SEQ ID NO: 41 (AHo) | LCDR2 | RASNLASGVPSR |
| SEQ ID NO: 42 (AHo) | LCDR3 | TYLSSNWYW |
| SEQ ID NO: 43 (Kabat) | LCDR1 | QSSQSVYDNNWLA |
| SEQ ID NO: 44 (Kabat) | LCDR2 | RASNLAS |
| SEQ ID NO: 45 (Kabat) | LCDR3 | QGTYLSSNWYWA |
| SEQ ID NO: 46 (Chothia) | LCDR1 | QSSQSVYDNNWLA |
| SEQ ID NO: 47 (Chothia) | LCDR2 | RASNLAS |
| SEQ ID NO: 48 (Chothia) | LCDR3 | QGTYLSSNWYWA |
| SEQ ID NO: 49 | VL | DIQMTQSPSSLSASVGDRVTITCQSSQ SVYDNNWLAWYQQKPGKAPKLLIY RASNLASGVPSRFSGSGSGTDFTLTIS SLQPEDFATYYCQGTYLSSNWYWAF GTGTKVTVLG |
| | (Vk1-sk17) | |
| SEQ ID NO: 50 | Linker | GGGGSGGGGSGGGGSGGGGS |
| SEQ ID NO: 51 | scFv (VL-linker-VH) | DIQMTQSPSSLSASVGDRVTITCQSSQ SVYDNNWLAWYQQKPGKAPKLLIY RASNLASGVPSRFSGSGSGTDFTLTIS SLQPEDFATYYCQGTYLSSNWYWAF GTGTKVTVLGGGGGSGGGGSGGGGS GGGGSQVQLQESGPGLVKPSETLSLT CKVSGFSFNNDYDMCWIRQPPGKGL EWIGCIDTGDGSTYYASWAKGRVTIS VDSSKNQFSLKLSSVTAADTAVYYC AREAASSSGYGMGYFDLWGQGTLVT VSS |

**TABLE 2. Other sequences related to the present invention.**

| **SEQ ID NUMBER** | **Ab region** | **Sequence** |
|---|---|---|
| SEQ ID NO: 52 | Human CD137 | MGNSCYNIVATLLLVLNFERTRSLQDPC SNCPAGTFCDNNRNQICSPCPPNSFSSAG GQRTCDICRQCKGVFRTRKECSSTSNAE CDCTPGFHCLGAGCSMCEQDCKQGQEL TKKGCKDCCFGTFNDQKRGICRPWTNC SLDGKSVLVNGTKERDVVCGPSPADLS PGASSVTPPAPAREPGHSPQIISFFLALTS TALLFLLFFLTLRFSVVKRGRKKLLYIFK QPFMRPVQTTQEEDGCSCRFPEEEEGGC EL |
| SEQ ID NO: 53 | Vλ germline-based FR4 | FGTGTKVTVLG |
| | (Sk17) | |
| SEQ ID NO: 54 | Vλ germline-based FR4 | FGGGTKLTVLG |
| | (Sk12) | |
| SEQ ID NO: 55 | Vλ germline-based FR4 | FGGGTQLIILG |
| SEQ ID NO: 56 | Vλ germline-based FR4 | FGEGTELTVLG |
| SEQ ID NO: 57 | Vλ germline-based FR4 | FGSGTKVTVLG |
| SEQ ID NO: 58 | Vλ germline-based FR4 | FGGGTQLTVLG |
| SEQ ID NO: 59 | Vλ germline-based FR4 | FGGGTQLTALG |

**TABLE 3. Examples of multispecific molecules comprising the antibody of the invention or antigen-binding fragment thereof.**

| **SEQ ID NUMBER** | **Ab Format** | **Sequence** |
|---|---|---|
| **PRO885** | | |
| 38-02-A04 sc01 scDb-i/33-03-G02 sc01 scDb-o | | |
| SEQ ID NO: 60 | scDb | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFSNSYWIC WIRQPPGKGLEWIGCTFVGSSDSTY YANWAKGRVTISVDSSKNQFSLKLS SVTAADTAVYYCARHPSDAVYGYA NNLWGQGTLVTVSSGGGGSGGGG SGGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSQV QLQESGPGLVKPSETLSLTCKVSGFS FSSGYDMCWIRQPPGKGLEWIGCV VAGSVDITYYASWAKGRVTISVDSS KNQFSLKLSSVTAADTAVYYCARK DAYSDAFNLWGQGTLVTVSS |
| **PRO951** | | |
| 38-27-C05 sc02 scDb-i/33-03-G02 sc01 scDb-o | | |
| SEQ ID NO: 61 | scDb | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSEVQLVESGGGL VQPGGSLRLSCAASGFSFNNDYDM CWVRQAPGKGLEWIGCIDTGDGST YYASWAKGRFTISRDNSKNTVYLQ MNSLRAEDTAVYYCAREAASSSGY GMGYFDLWGQGTLVTVSSGGGGS |
| | | GGGGSGGGGSGGGGSIQMTQSPSS LSASVGDRVTITCQSSQSVYDNNWL AWYQQKPGKAPKLLIYRASNLASG VPSRFSGSGSGTDFTLTISSLQPEDFA TYYCQGTYLSSNWYWAFGTGTKVT VLGGGGGSQVQLQESGPGLVKPSE TLSLTCKVSGFSFSSGYDMCWIRQP PGKGLEWIGCVVAGSVDITYYASW AKGRVTISVDSSKNQFSLKLSSVTA ADTAVYYCARKDAYSDAFNLWGQ GTLVTVSS |
| **PRO1123** | | |
| 38-02-A04 sc05 IF scDb-i/33_03_G02 sc01 scDb-o | | |
| SEQ ID NO: 62 | scDb | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFSNSYWIC WVRQPPGKGLEWIGCTFVGSSDSTY YANWAKGRVTISVDSSKNQVSLKL SSVTAADTAVYFCARHPSDAVYGY ANNLWGQGTLVTVSSGGGGSGGG GSGGGGSGGGGSIQMTQSPSSLSA SVGDRVTITCQASQSINNVLAWYQQ KPGKPPKLLIYRASTLASGVPSRFSG SGSGTDFTLTISSLQPEDFATYYCQS SYGNYGDFGTGTKVTVLGGGGGSQ VQLQESGPGLVKPSETLSLTCKVSG FSFSSGYDMCWIRQPPGKGLEWIGC VVAGSVDITYYASWAKGRVTISVDS SKNQFSLKLSSVTAADTAVYYCAR KDAYSDAFNLWGQGTLVTVSS |
| **PRO1124** | | |
| 38-02-A04 sc06 Full scDb-i/33_03_G02 sc01 scDb-o | | |
| SEQ ID NO: 63 | scDb | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKASGFSFSNSYWIC WVRQPPGKGLEWIGCTFVGSSDSTY YANWAKGRVTISKDSSKNQVSLKL SSVTAADTAVYFCARHPSDAVYGY ANNLWGQGTLVTVSSGGGGSGGG GSGGGGSGGGGSLQMTQSPSSLSA SVGDRVTITCQASQSINNVLAWYQQ |
| | | KPGKPPKLLIYRASTLASGVPSRFSG SGSGTDFTLTISSLQPEDFATYYCQS SYGNYGDFGTGTKVTVLGGGGGSQ VQLQESGPGLVKPSETLSLTCKVSG FSFSSGYDMCWIRQPPGKGLEWIGC VVAGSVDITYYASWAKGRVTISVDS SKNQFSLKLSSVTAADTAVYYCAR KDAYSDAFNLWGQGTLVTVSS |
| **PR01125** | | |
| 38-02-A04 sc01 scDb-i/33_03_G02 sc02 IF scDb-o | | |
| SEQ ID NO: 64 | scDb | DIQMTQSPSSLSASVGDRVTITCQAS QSINDYLAWYQQKPGKSPKLLIYKA STLASGVPSRFSGSGSGTDFTLTISSL QPEDFATYYCQQGYIITDIDNVFGTG TKVTVLGGGGGSQVQLQESGPGLV KPSETLSLTCKVSGFSFSNSYWICWI RQPPGKGLEWIGCTFVGSSDSTYYA NWAKGRVTISVDSSKNQFSLKLSSV TAADTAVYYCARHPSDAVYGYAN NLWGQGTLVTVSSGGGGSGGGGS GGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSQV QLQESGPGLVKPSETLSLTCKVSGFS FSSGYDMCWVRQPPGKGLEWIACV VAGSVDITYYASWAKGRVTISVDSS KNQFSLKLSSVTAADTAVYFCARK DAYSDAFNLWGQGTLVTVSS |
| **PRO1126** | | |
| 38-02-A04 sc01 scDb-i/33_03_G02 sc03 Full scDb-o | | |
| SEQ ID NO: 65 | scDb | DFQLTQSPSSLSASVGDRVTITCQAS QSINDYLAWYQQKPGKSPKLLIYKA STLASGVPSRFSGSGSGTDFTLTISSL QPEDFATYYCQQGYIITDIDNVFGTG TKVTVLGGGGGSQVQLQESGPGLV KPSETLSLTCKVSGFSFSNSYWICWI RQPPGKGLEWIGCTFVGSSDSTYYA NWAKGRVTISVDSSKNQFSLKLSSV TAADTAVYYCARHPSDAVYGYAN NLWGQGTLVTVSSGGGGSGGGGS GGGGSGGGGSIQMTQSPSSLSASV GDRVTITCQASQSINNVLAWYQQKP |
| | | GKAPKLLIYRASTLASGVPSRFSGSG SGTDFTLTISSLQPEDFATYYCQSSY GNYGDFGTGTKVTVLGGGGGSQSQ LQESGPGLVKPSETLSLTCKASGFSF SSGYDMCWVRQPPGKGLEWIACVV AGSVDITYYASWAKGRVTISKDSSK NQVSLKLSSVTAADTAVYFCARKD AYSDAFNLWGQGTLVTVSS |
| **PRO1134** | | |
| 38-02-A04 sc01 scDb-i/33_03_G02 sc07 GL VH3 scDb-o | | |
| SEQ ID NO: 66 | scDb | DIQMTQSPSSLSASVGDAVTITCQAS QSINDYLAWYQQKPGKSPKLLIYKA STLASGVPSRFSGSGSGTDFTLTISSL QPEDFATYYCQQGYIITDIDNVFGTG TKVTVLGGGGGSQVQLQESGPGLV KPSETLSLTCKVSGFSFSNSYWICWI RQPPGKGLEWIGCTFVGSSDSTYYA NWAKGRVTISVDSSKNQFSLKLSSV TAADTAVYYCARHPSDAVYGYAN NLWGQGTLVTVSSGGGGSGGGGS GGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSEV QLVESGGGLVQPGGSLRLSCAASGF SFSSGYDMCWVRQAPGKGLEWVG CVVAGSVDITYYASWAKGRFTISRD NSKNTVYLQMNSLRAEDTATYYCA RKDAYSDAFNLWGPGTLVTVSS |
| **PRO963 (= PRO1051)** | | |
| 38_02_A04 sc01 scDb-i/33-03-G02 sc01 scDb-o/19-01-H04-sc03 scFv | | |
| SEQ ID NO: 67 | scTriabody | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFSNSYWIC WIRQPPGKGLEWIGCTFVGSSDSTY YANWAKGRVTISVDSSKNQFSLKLS SVTAADTAVYYCARHPSDAVYGYA NNLWGQGTLVTVSSGGGGSGGGG SGGGGSGGGGSIQMTQSPSSLSASV GDRVTITCQASQSINNVLAWYQQKP |
| | | GKAPKLLIYRASTLASGVPSRFSGSG SGTDFTLTISSLQPEDFATYYCQSSY GNYGDFGTGTKVTVLGGGGGSQV QLQESGPGLVKPSETLSLTCKVSGFS FSSGYDMCWIRQPPGKGLEWIGCV VAGSVDITYYASWAKGRVTISVDSS KNQFSLKLSSVTAADTAVYYCARK DAYSDAFNLWGQGTLVTVSSGGG GSGGGGSIQMTQSPSSLSASVGDRV TITCQSSESVYSNNQLSWYQQKPGQ PPKLLIYDASDLASGVPSRFSGSGSG TDFTLTISSLQPEDFATYYCAGGFSS SSDTAFGGGTKLTVLGGGGGSGGG GSGGGGSGGGGSEVQLVESGGGL VQPGGSLRLSCAASGFSLSSNAMG WVRQAPGKGLEYIGIISVGGFTYYA SWAKGRFTISRDNSKNTVYLQMNS LRAEDTATYFCARDRHGGDSSGAF YLWGQGTLVTVSS |
| **PRO966 (= PRO1052)** | | |
| 38_27_C05 sc01 scDb-i/33-03-G02 sc01 scDb-o/19-01-H04-sc03 scFv | | |
| SEQ ID NO: 68 | scTriabody | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFNNDYDMC WIRQPPGKGLEWIGCIDTGDGSTYY ASWAKGRVTISVDSSKNQFSLKLSS VTAADTAVYYCAREAASSSGYGMG YFDLWGQGTLVTVSSGGGGSGGG GSGGGGSGGGGSIQMTQSPSSLSA SVGDRVTITCQSSQSVYDNNWLAW YQQKPGKAPKLLIYRASNLASGVPS RFSGSGSGTDFTLTISSLQPEDFATY YCQGTYLSSNWYWAFGTGTKVTVL GGGGGSQVQLQESGPGLVKPSETL SLTCKVSGFSFSSGYDMCWIRQPPG KGLEWIGCVVAGSVDITYYASWAK GRVTISVDSSKNQFSLKLSSVTAAD TAVYYCARKDAYSDAFNLWGQGT LVTVSSGGGGSGGGGSIQMTQSPS SLSASVGDRVTITCQSSESVYSNNQL SWYQQKPGQPPKLLIYDASDLASGV PSRFSGSGSGTDFTLTISSLQPEDFAT YYCAGGFSSSSDTAFGGGTKLTVLG GGGGSGGGGSGGGGSGGGGSEV |
| | | QLVESGGGLVQPGGSLRLSCAASGF SLSSNAMGWVRQAPGKGLEYIGIIS VGGFTYYASWAKGRFTISRDNSKNT VYLQMNSLRAEDTATYFCARDRHG GDSSGAFYLWGQGTLVTVSS |
| **PRO1057** | | |
| 38_02_A04 sc01 scDb-i/33-03-G02 sc01 scDb-o/mxr HSA (23-13-A01-sc03, sk17sh4) | | |
| SEQ ID NO: 69 | scTriabody | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGGGGGSQVQLQESGPGL VKPSETLSLTCKVSGFSFSNSYWIC WIRQPPGKGLEWIGCTFVGSSDSTY YANWAKGRVTISVDSSKNQFSLKLS SVTAADTAVYYCARHPSDAVYGYA NNLWGQGTLVTVSSGGGGSGGGG SGGGGSGGGGSIQMTQSPSSLSASV GDRVTITCQASQSINNVLAWYQQKP GKAPKLLIYRASTLASGVPSRFSGSG SGTDFTLTISSLQPEDFATYYCQSSY GNYGDFGTGTKVTVLGGGGGSQV QLQESGPGLVKPSETLSLTCKVSGFS FSSGYDMCWIRQPPGKGLEWIGCV VAGSVDITYYASWAKGRVTISVDSS KNQFSLKLSSVTAADTAVYYCARK DAYSDAFNLWGQGTLVTVSSGGG GSGGGGSVVMTQSPSSLSASVGDR VTITCQASQIISSRSAWYQQKPGQPP KLLIYQASKLASGVPSRFSGSGSGTD FTLTISSLQPEDFATYYCQCTYIDSN FGAFGGGTKLTVLGGGGGSGGGG SGGGGSGGGGSEVQLVESGGGLV QPGGSLRLSCAASGFSFSSSYWICW VRQAPGKGLEWVGCVFTGDGTTYY ASWAKGRFTISRDNSKNTVYLQMN SLRAEDTATYFCARPVSVYYYGMD LWGQGTLVTVSS |
| **PRO1058** | | |
| 38_27_C05 sc01 scDb-i/33-03-G02 sc01 scDb-o/mxr HSA (23-13-A01-sc03, sk17sh4) | | |
| SEQ ID NO: 70 | scTriabody | DIQMTQSPSSLSASVGDRVTITCQAS QSINDYLAWYQQKPGKAPKLLIYK |
| | | ASTLASGVPSRFSGSGSGTDFTLTISS LQPEDFATYYCQQGYIITDIDNVFGT GTKVTVLGGGGGSQVQLQESGPGL VKPSETLSLTCKVSGFSFNNDYDMC WIRQPPGKGLEWIGCIDTGDGSTYY ASWAKGRVTISVDSSKNQFSLKLSS VTAADTAVYYCAREAASSSGYGMG YFDLWGQGTLVTVSSGGGGSGGG GSGGGGSGGGGSIQMTQSPSSLSA SVGDRVTITCQSSQSVYDNNWLAW YQQKPGKAPKLLIYRASNLASGVPS RFSGSGSGTDFTLTISSLQPEDFATY YCQGTYLSSNWYWAFGTGTKVTVL GGGGSQVQLQESGPGLVKPSETL SLTCKVSGFSFSSGYDMCWIRQPPG KGLEWIGCVVAGSVDITYYASWAK GRVTISVDSSKNQFSLKLSSVTAAD TAVYYCARKDAYSDAFNLWGQGT LVTVSSGGGGSGGGGSVVMTQSPS SLSASVGDRVTITCQASQIISSRSAW YQQKPGQPPKLLIYQASKLASGVPS RFSGSGSGTDFTLTISSLQPEDFATY YCQCTYIDSNFGAFGGGTKLTVLGG GGGSGGGGSGGGGSGGGGSEVQL VESGGGLVQPGGSLRLSCAASGFSF SSSYWICWVRQAPGKGLEWVGCVF TGDGTTYYASWAKGRFTISRDNSK NTVYLQMNSLRAEDTATYFCARPV SVYYYGMDLWGQGTLVTVSS |
| **PRO1059** | | |
| 33-03-G02 IgG1 LC with 38_02_A04 sc01 scFV, PD-L1/CD137(scFv) silent Morrison | | |
| SEQ ID NO: 71 | Morrison-L Light chain | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGTVAAPSVFIFPPSDEQLK SGTASVVCLLNNFYPREAKVQWKV DNALQSGNSQESVTEQDSKDSTYSL SSTLTLSKADYEKHKVYACEVTHQ GLSSPVTKSFNRGECGGGGSGGGG SIQMTQSPSSLSASVGDRVTITCQAS QSINNVLAWYQQKPGKAPKLLIYRA STLASGVPSRFSGSGSGTDFTLTISSL QPEDFATYYCQSSYGNYGDFGTGT KVTVLGGGGGSGGGGSGGGGSG GGGSQVQLQESGPGLVKPSETLSLT |
| | | CKVSGFSFSNSYWICWIRQPPGKGL EWIGCTFVGSSDSTYYANWAKGRV TISVDSSKNQFSLKLSSVTAADTAV YYCARHPSDAVYGYANNLWGQGT LVTVSS |
| SEQ ID NO: 72 | Morrison-L Heavy chain | QVQLQESGPGLVKPSETLSLTCKVS GFSFSSGYDMCWIRQPPGKGLEWIG CVVAGSVDITYYASWAKGRVTISV DSSKNQFSLKLSSVTAADTAVYYCA RKDAYSDAFNLWGQGTLVTVSSAS TKGPSVFPLAPSSKSTSGGTAALGCL VKDYFPEPVTVSWNSGALTSGVHTF PAVLQSSGLYSLSSVVTVPSSSLGTQ TYICNVNHKPSNTKVDKRVEPKSCD KTHTCPPCPAPEAAGGPSVFLFPPKP KDTLMISRTPEVTCVVVDVSHEDPE VKFNWYVDGVEVHNAKTKPREEQ YNSTYRVVSVLTVLHQDWLNGKEY KCKVSNKALGAPIEKTISKAKGQPR EPQVYTLPPSREEMTKNQVSLTCLV KGFYPSDIAVEWESNGQPENNYKTT PPVLDSDGSFFLYSKLTVDKSRWQQ GNVFSCSVMHEALHNHYTQKSLSL SPGK |
| **PRO1060** | | |
| 33-03-G02 IgG1 HC with 38_02_A04 sc01 scFV, PD-L1/CD137(scFv) silent Morrison | | |
| SEQ ID NO: 73 | Morrison-H Light chain | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGTVAAPSVFIFPPSDEQLK SGTASVVCLLNNFYPREAKVQWKV DNALQSGNSQESVTEQDSKDSTYSL SSTLTLSKADYEKHKVYACEVTHQ GLSSPVTKSFNRGEC |
| SEQ ID NO: 74 | Morrison-H Heavy chain | QVQLQESGPGLVKPSETLSLTCKVS GFSFSSGYDMCWIRQPPGKGLEWIG CVVAGSVDITYYASWAKGRVTISV DSSKNQFSLKLSSVTAADTAVYYCA RKDAYSDAFNLWGQGTLVTVSSAS TKGPSVFPLAPSSKSTSGGTAALGCL VKDYFPEPVTVSWNSGALTSGVHTF PAVLQSSGLYSLSSVVTVPSSSLGTQ TYICNVNHKPSNTKVDKRVEPKSCD |
| | | KTHTCPPCPAPEAAGGPSVFLFPPKP KDTLMISRTPEVTCVVVDVSHEDPE VKFNWYVDGVEVHNAKTKPREEQ YNSTYRVVSVLTVLHQDWLNGKEY KCKVSNKALGAPIEKTISKAKGQPR EPQVYTLPPSREEMTKNQVSLTCLV KGFYPSDIAVEWESNGQPENNYKTT PPVLDSDGSFFLYSKLTVDKSRWQQ GNVFSCSVMHEALHNHYTQKSLSL SPGKGGGGSGGGGSIQMTQSPSSLS ASVGDRVTITCQASQSINNVLAWYQ QKPGKAPKLLIYRASTLASGVPSRFS GSGSGTDFTLTISSLQPEDFATYYCQ SSYGNYGDFGTGTKVTVLGGGGGS GGGGSGGGGSGGGGSQVQLQESG PGLVKPSETLSLTCKVSGFSFSNSY WICWIRQPPGKGLEWIGCTFVGSSD STYYANWAKGRVTISVDSSKNQFSL KLSSVTAADTAVYYCARHPSDAVY GYANNLWGQGTLVTVSS |
| **PRO1061** | | |
| 33-03-G02 sc01 IgG1 LC with 38_27_C05 sc01 scFv, PD-L1/CD137(scFv) silent Morrison | | |
| SEQ ID NO: 75 | Morrison-L Light chain | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGTVAAPSVFIFPPSDEQLK SGTASVVCLLNNFYPREAKVQWKV DNALQSGNSQESVTEQDSKDSTYSL SSTLTLSKADYEKHKVYACEVTHQ GLSSPVTKSFNRGECGGGGSGGGG SIQMTQSPSSLSASVGDRVTITCQAS QSINNVLAWYQQKPGKAPKLLIYRA STLASGVPSRFSGSGSGTDFTLTISSL QPEDFATYYCQSSYGNYGDFGTGT KVTVLGGGGGSGGGGSGGGGSG GGGSQVQLQESGPGLVKPSETLSLT CKVSGFSFSNSYWICWIRQPPGKGL EWIGCTFVGSSDSTYYANWAKGRV TISVDSSKNQFSLKLSSVTAADTAV YYCARHPSDAVYGYANNLWGQGT LVTVSS |
| SEQ ID NO: 76 | Morrison-L Heavy chain | QVQLQESGPGLVKPSETLSLTCKVS GFSFSSGYDMCWIRQPPGKGLEWIG CVVAGSVDITYYASWAKGRVTISV |
| | | DSSKNQFSLKLSSVTAADTAVYYCA RKDAYSDAFNLWGQGTLVTVSSAS TKGPSVFPLAPSSKSTSGGTAALGCL VKDYFPEPVTVSWNSGALTSGVHTF PAVLQSSGLYSLSSVVTVPSSSLGTQ TYICNVNHKPSNTKVDKRVEPKSCD KTHTCPPCPAPEAAGGPSVFLFPPKP KDTLMISRTPEVTCVVVDVSHEDPE VKFNWYVDGVEVHNAKTKPREEQ YNSTYRVVSVLTVLHQDWLNGKEY KCKVSNKALGAPIEKTISKAKGQPR EPQVYTLPPSREEMTKNQVSLTCLV KGFYPSDIAVEWESNGQPENNYKTT PPVLDSDGSFFLYSKLTVDKSRWQQ GNVFSCSVMHEALHNHYTQKSLSL SPGK |
| **PRO1062** | | |
| 33-03-G02 sc01 IgG1 HC with 38_27_C05 sc01 scFv, PD-L1/CD137(scFv) silent Morrison | | |
| SEQ ID NO: 77 | Morrison-H Light chain | DIQMTQSPSSLSASVGDRVTITCQAS QSINDYLAWYQQKPGKAPKLLIYK ASTLASGVPSRFSGSGSGTDFTLTISS LQPEDFATYYCQQGYIITDIDNVFGT GTKVTVLGTVAAPSVFIFPPSDEQLK SGTASVVCLLNNFYPREAKVQWKV DNALQSGNSQESVTEQDSKDSTYSL SSTLTLSKADYEKHKVYACEVTHQ GLSSPVTKSFNRGEC |
| SEQ ID NO: 78 | Morrison-H Heavy chain | QVQLQESGPGLVKPSETLSLTCKVS GFSFSSGYDMCWIRQPPGKGLEWIG CVVAGSVDITYYASWAKGRVTISV DSSKNQFSLKLSSVTAADTAVYYCA RKDAYSDAFNLWGQGTLVTVSSAS TKGPSVFPLAPSSKSTSGGTAALGCL VKDYFPEPVTVSWNSGALTSGVHTF PAVLQSSGLYSLSSVVTVPSSSLGTQ TYICNVNHKPSNTKVDKRVEPKSCD KTHTCPPCPAPEAAGGPSVFLFPPKP KDTLMISRTPEVTCVVVDVSHEDPE VKFNWYVDGVEVHNAKTKPREEQ YNSTYRVVSVLTVLHQDWLNGKEY KCKVSNKALGAPIEKTISKAKGQPR EPQVYTLPPSREEMTKNQVSLTCLV KGFYPSDIAVEWESNGQPENNYKTT PPVLDSDGSFFLYSKLTVDKSRWQQ GNVFSCSVMHEALHNHYTQKSLSL |
| | | SPGKGGGGSGGGGSIQMTQSPSSLS ASVGDRVTITCQASQSINNVLAWYQ QKPGKAPKLLIYRASTLASGVPSRFS GSGSGTDFTLTISSLQPEDFATYYCQ SSYGNYGDFGTGTKVTVLGGGGGS GGGGSGGGGSGGGGSQVQLQESG PGLVKPSETLSLTCKVSGFSFSNSY WICWIRQPPGKGLEWIGCTFVGSSD STYYANWAKGRVTISVDSSKNQFSL KLSSVTAADTAVYYCARHPSDAVY GYANNLWGQGTLVTVSS |

Throughout the text of this application, should there be a discrepancy between the text of the specification (e.g., Tables 1 to 3) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all subcombinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

### Examples

### NOVEL ANTIBODIES DIRECTED AGAINST HUMAN CD137

### Example 1: Generation of rabbit antibodies directed against human CD137.

Rabbits have been immunized with recombinantly produced and purified human CD137 extracellular domain (Peprotech, cat. 310-15-1MG). During the immunization, the strength of the humoral immune response against the antigen was qualitatively assessed by determining the maximal dilution (titer) for the serum of each rabbit that still produced detectable binding of the polyclonal serum antibodies to the antigen. Serum antibody titers against the immobilized antigen (recombinant human CD 137 ECD) were assessed using an enzyme-linked immunosorbent assay (ELISA). All six rabbits immunized showed very high titers of at least 1:702' 175 dilution of the serum. Serum from the same rabbits before the first antigen injection were used as background control.

### Example 2: Hit Identification and selection.

Within the Hit identification procedure, a flow-cytometry-based sorting procedure was developed that specifically detects and allows for the isolation of high-affinity human CD137 ECD binding B-cells. To identify CD137 binding B-cells, CD137 ECD was labeled with the fluorescent dye R-Phycoerythrin (RPE). Since the CD137L binding site as well as the binding site of an anti-CD137 antibody on the labeled CD137 could potentially be blocked by the bulky RPE label, accessibility of the epitopes was confirmed by flow-cytometry. CD137L ECD fused to the Fc part of a human IgG1, Urelumab, rabbit polyclonal anti-human CD137 or goat polyclonal anti-human CD137 were captured on protein G beads, and binding of R-PE labeled CD137 was confirmed by flow-cytometry. The fluorescence intensity is proportional to the amount of labeled CD137 bound to CD137L immobilized on the beads. Binding of CD137 to CD137L and anti-CD137 antibodies was found while no binding of RPE-labeled CD137 to Infliximab was detected.

### Sorting:

A total of 1.44E+07 lymphocytes derived from all six rabbits were analyzed in the sorting campaign. Out of the 1.44E+07 cells analyzed, in total 2816 B-cells expressing CD137-specific antibodies (IgG) were isolated. A total of 718 hits were identified.

### Screening:

The results obtained during the screening phase are based on assays performed with non-purified antibodies from culture supernatants of antibody secreting cells (ASC), as the scale of the high-throughput culture does not allow for purification of the individual rabbit antibodies. Such supernatants allow to rank large numbers of antibodies relative to each other, however do not provide absolute values except for binding affinity. During the course of at least four weeks, supernatants from every individually cultured clone were collected. At the end of the cultivation period, the rabbit monoclonal antibodies in each cell culture supernatant were characterized in a high-throughput ELISA for binding to recombinant human CD137 ECD. CD137-binding supernatants were further characterized for binding kinetics to human and cynomolgus CD137. In addition, neutralization potential of the CD137 interaction to CD137L as well as to Urelumab was determined by competition ELISA. Binding to membranous CD137 expressed on stable transduced Jurkat cells was also assessed. Mouse CD137 binding potential of the supernatants was analyzed by direct ELISA and binding kinetics were determine only for the positive supernatants.

### Direct ELISA

The aim of the primary screening is to identify B-cell clones that produce antibodies binding to CD137 ECD. For this purpose, cell culture supernatants of 2'816 B-cell clones were screened for the presence of antibodies to human CD137 by ELISA. The ELISA method used assesses the "quantity" of antibodies of the IgG subtype bound to recombinant human CD137, gives however no information about the affinity or the concentration of the antibodies.

Method: ELISA plates were coated by adding 50 µl of PBS containing 250 ng/ml human CD137 (Peprotech, cat. 310-15-1MG) overnight at 4°C. Next day, plates were washed three times in overflow mode with 300 µl wash buffer (PBS, 0.005% Tween 20) per wells and 270 µl of blocking buffer (PBS, 1% BSA, 0.2% Tween 20) were added to each well for 1h at RT without shaking. Then, plates were washed three times in overflow mode with 300 µl wash buffer and 50 µl of each supernatant was added, plates were incubated 1.5h at RT under gentle agitation. After 3 washes in overflow mode with 300 µl wash buffer, 50 µl of a HRP-coupled goat-anti-rabbit IgG antibody 1:5'000 diluted in blocking buffer was added to each well. After 1h incubation at RT on a nutating mixer, plates were washed three times in overflow mode with 300 µl wash buffer per well prior to the addition of 50 µl TMB (3,3',5,5'-tetramethylbenzidine). After 5 to 10 minutes development the enzymatic reaction was stopped by addition of 50 µl of 1M HCl per well and plate was read at 450 nm using 690 nm as a reference wavelength.

Results: In this assay, supernatants from 718 B-cell clones produced a signal that was clearly above background. All 718 hits identified in this primary screening, were subjected to the measurement of binding kinetics by SPR (secondary screening).

### Affinity to hCD137 by SPR

The 718 B-cell clones qualified as positive by direct ELISA, were then entering secondary screens. One of them is the determination of the affinity to human recombinant CD 137 ECD by surface plasmon resonance (SPR). In contrast to the ELISA used during the primary screening, the method used here assesses the kinetics of target binding as a function of time. This allows determination of the rate constants for association (kₐ) and dissociation (k_{d}) of the antibody from its target. The ratio k_{d}/kₐ provides the equilibrium dissociation constant (K_{D}), which reflects the affinity - the binding strength - of an antibody to its target. The lower the value for K_{D}, the stronger is the interaction.

Method: Binding affinities of antibodies towards human CD137 were measured by SPR using a MASS-1 SPR instrument (Sierra Sensors). For affinity screening, an antibody specific for the Fc region of rabbit IgGs (Bethyl Laboratories, Cat. No. A120-111A) was immobilized on a sensor chip (MASS-1 Affinity Sensor, High Capacity Amine, Sierra Sensors) using a standard amine-coupling procedure. Rabbit monoclonal antibodies in B-cell supernatants were captured by the immobilized anti-rabbit IgG antibody. A minimal IgG concentration in the B-cell supernatants is required to allow sufficient capture. After capturing of the monoclonal antibodies, human CD137 ECD (Peprotech, cat. 310-15-1MG) was injected into the flow cells for 3 min at a concentration of 90 nM and dissociation of the protein from the IgG captured on the sensor chip was allowed to proceed for 5 min. After each injection cycle, surfaces were regenerated with two injections of 10 mM Glycine-HCl. The apparent dissociation (k_{d}) and association (kₐ) rate constants and the apparent dissociation equilibrium constant (K_{D}) were calculated with the MASS-1 analysis software (Analyzer, Sierra Sensors) using one-to-one Langmuir binding model and quality of the fits was monitored based on relative Chi² (Chi² normalized to the extrapolated maximal binding level of the analyte), which is a measure for the quality of the curve fitting. The smaller the value for the Chi² the more accurate is the fitting to the one-to-one Langmuir binding model. For most of the hits the relative Chi² value was below 15%. Results were deemed valid if the response units (RU) for ligand binding were at least 2% of the RUs for antibody capturing. Samples with RUs for ligand binding with less than 2% of the RUs for antibody capturing were considered to show no specific binding of CD137 to the captured antibody.

Results: For 31 antibodies, no binding was observed. The affinities for the other 686 clones that could be measured showed K_{D} ranging from 3.77E-13 M to 8.92E-07 M. For 16 clones, a negative k_{d} was detected and the k_{d} was set to <1E-06.

### CD137/CD137L competition ELISA

The 718 hits were then tested for their ability to neutralize the CD137/CD137L interaction in a competition ELISA. The commercial inhibitory polyclonal anti-CD 137 goat antibody (Antibodies online, cat. ABIN636609) served as a reference.

Method: ELISA plates were coated by adding 50 µl of PBS containing 50 ng/ml CD137 Fc chimera (R&D Systems, cat. 838-4B-100) overnight at 4°C. Next day, plates were washed three times in overflow mode with 450 µl wash buffer (PBS, 0.005% Tween 20) per wells and 300 µl of blocking buffer (PBS with 1% BSA and 0.2 Tween 20) were added to each well for 1h at RT on a nutating mixer. Then, the positive control (neutralizing goat anti-CD137 antibody) was diluted in 100% negative supernatant of sort 38 and 50 µl of the neutralizing antibody was added to the corresponding wells of the binding plate. In addition, 50 µl of the supernatant of the positive hits were transferred to the binding plate and incubated for 1h at RT with shaking. Next, ELISA plates were washed 3 times in overflow mode with 450 µl wash buffer per well and 50 µl of 20 ng/ml biotinylated recombinant human CD137 ligand (Acro Biosystem, cat. 41L-H5257) diluted in blocking buffer was added to the wells. After 1h of incubation at RT with shaking, the ELISA plates were washed 3 times in overflow mode with 450 µl wash buffer per well. Then, 50 µl of 10 ng/ml streptavidin-poly-HRP diluted in blocking buffer was added to each wells of the ELISA plates. After 1h incubation at RT, plates were washed three times with 450 µl wash buffer and developed for 5 to 10 minutes after addition of 50 µl TMB. Finally, the enzymatic reaction was stopped by addition of 50 µl of 1M HCl and plate was read at 450 nm using 690 nm as a reference wavelength.

Results: In total, 718 clones were tested and 260 clones were identified as neutralizing (more than 30% inhibition as compared to reference molecule) and 458 clones as non-neutralizing (less than 30% of inhibtion) the CD137/CD137L interaction.

### Species specificity by SPR: cyno

Binding kinetics to cynomolgus CD137 were also determined for the 718 hits identified in the initial screening ELISA using the same SPR setup as described for the binding to human CD137, but replacing human CD137 ECD by cynomolgus monkey CD137 ECD (Acro Biosystem, cat. 41B-C52H4). For 121 antibodies, no binding could be measured. The affinities for the other 597 clones that could be measured showed K_{D} ranging from 8.69E-13 M to 2.64E-07 M.

### Urelumab competition ELISA

The 718 hits were then tested for their ability to compete with Urelumab for binding to human CD137 in a competition ELISA in order to identify clones sharing the same or an overlapping epitope as Urelumab.

Method: ELISA plates were coated by adding 50 µl of PBS containing 2 µg/ml Urelumab (produced by Evitria, Schlieren, Switzerland) overnight at 4°C. Next day, plates were washed three times in overflow mode with 450 µl wash buffer (PBS, 0.005% Tween 20) per wells and 300 µl of blocking buffer (PBS with 1% BSA and 0.2 Tween 20) were added to each well for 1h at RT on a nutating mixer. Then, Urelumab was diluted in 95% negative supernatant of sort 38 spiked and pre-incubated for 1h with 5% biotinylated CD137 ECD (Peprotech, cat. 310-15-1MG) at 7.5 ng/ml and was added to the corresponding wells of the binding plate. In addition, 55 µl of the supernatant of the positive hits were also spiked and pre-incubated for 1h with 5% biotinylated CD137 ECD at 7.5 ng/ml and transferred to the binding plate and incubated for 1h at RT with shaking. Next, the ELISA plates were washed 3 times in overflow mode with 450 µl wash buffer per well. Then, 50 µl of 10 ng/ml streptavidin-poly-HRP diluted in blocking buffer was added to each well of the ELISA plate. After 1h incubation at RT, plates were washed three times with 450 µl wash buffer and developed for 5 to 10 minutes after addition of 50 µl TMB. Finally, the enzymatic reaction was stopped by addition of 50 µl of 1M HCl and plate was read at 450 nm using 690 nm as a reference wavelength.

Results: In total, 718 clones were tested and 136 clones were identified as Urelumab competitors (more than 30% inhibition as compared to Urelumab) and 582 clones as non-competitors.

### Cell based binding assay by FC: human CD137

The 718 hits were then tested for their ability to bind cellular human CD137. For this assay, stable-transfected Jurkat cells expressing CD137 constitutively were generated and used.

Method: Jurkat wild type (control cells that do not express CD137) and Jurkat CD137 cells (clone C6, 1) were harvested and cell number was determined. Cell suspensions were centrifuged for 5min at 400xg and 40µl of cell suspensions (40'000 cells) diluted in PBS-EB (1x DPBS, 2% BCS H.I., 2mM EDTA) were added to designated wells in a non-binding plate. Supernatant from positive hits of sort 38 were directly transferred into 96 well plate according to plate layout. Positive control samples (Urelumab) were diluted in PBS-EB and transferred to the plate, final samples were of 95% negative supernatant of sort 38. After incubation at 4°C for 1h, plates washed 3 times using 100µl of PBS-EB. Then, cell pellets were re-suspended with 50µl secondary antibody solution at a concentration of 2µg/ml (for B-cell clones: goat-anti-rabbit IgG labeled with AF647; for Urelumab: goat-anti-human IgG labeled with PE) and incubated for 1h at 4°C. Next, cells were washed again three times using 100µl of PBS-EB. The cell pellets were then re-suspended with 50µl PBS-EB and analyzed with NovoCyte 2060 flow cytometer device. Fluorescence intensity of PE and AF647 for 20'000 events was recorded for each sample and the geometric mean of fluorescence intensity MFI was calculated. The data were first corrected for unspecific antibody binding (blank and Jurkat wild type cell binding) and then normalized to binding levels obtained for Urelumab.

Results: In total, 718 clones were tested and 525 clones were identified as cellular CD137 binder (more than 5% binding relative to Urelumab) and 193 clones as non-binders.

### Direct ELISA mouse CD137

As a first step to identify mouse cross-reactive CD137 binder, a direct ELISA against the mouse CD137 was performed. For this purpose, cell culture supernatants of 2'816 B-cell clones were screened for the presence of antibodies to mouse CD137 by ELISA. The ELISA method used assesses the "quantity" of antibodies of the IgG subtype bound to recombinant mouse CD 137, gives however no information about the affinity or the concentration of the antibodies.

Method: ELISA plates were coated by adding 50 µl of PBS containing 250 ng/ml mouse CD137 (Acro Biosystem, cat. 41B-M52H7) overnight at 4°C. Next day, plates were washed three times in overflow mode with 300 µl wash buffer (PBS, 0.005% Tween 20) per wells and 270 µl of blocking buffer (PBS, 1% BSA, 0.2% Tween 20) were added to each well for 1h at RT without shaking. Then, plates were washed three times in overflow mode with 300 µl wash buffer and 50 µl of each supernatant was added, plates were incubated 1.5h at RT under gentle agitation. After 3 washes in overflow mode with 300 µl wash buffer, 50 µl of a HRP-coupled goat-anti-rabbit IgG antibody 1:5'000 diluted in blocking buffer was added to each well. After 1h incubation at RT on a nutating mixer, plates were washed three times in overflow mode with 300 µl wash buffer per well prior to the addition of 50 µl TMB. After 5 to 10 minutes development the enzymatic reaction was stopped by addition of 50 µl of 1M HCl per well and plate was read at 450 nm using 690 nm as a reference wavelength. In this assay, supernatants from 85 B-cell clones produced a signal that was clearly above background (>0.1 OD).

Results: All 85 hits identified in this primary screening, were subjected to the measurement of binding kinetics by SPR (secondary screening).

### Species specificity by SPR: mouse

Binding kinetics to mouse CD137 were also determined using the same setup as described for the binding to the human CD137, but in this case human CD137 ECD was replaced by mouse CD137 ECD (Acro Biosystem, cat. 41B-M52H7). The 85 monoclonal rabbit antibodies qualified as positive by direct ELISA using mouse CD137, were measured by SPR. For 28 antibodies, no binding could be measured. The affinities for the other 57 clones that could be measured showed K_{D} ranging from 9.28E-12 M to 6.16E-07 M.

### Selection of Screening Hits

Based on pharmacologic properties of monoclonal antibodies in B-cell supernatant a number of clones were selected for hit confirmation analysis. Pharmacologic properties of monoclonal antibodies of final clones in B-cell supernatant are presented in Table 4. In a next step, selected clones were used for RNA isolation and RT-PCR to amplify the sequence of rabbit antibody light and heavy chain variable regions (38-02-A04 and 38-27-C05).

**TABLE 4 Pharmacodynamic properties of monoclonal antibodies in B-cell supernatants: 38-02-A04.**

| | **Affinity to human CD137 (SPR)** | | | **Affinity to cyno CD137 (SPR)** | | | **Affinity ratio** | **Affinity to mouse CD137 (SPR)** | | | **Affinity ratio** | **Urelumab competition ELISA** | **Binding to cellular human CD137** |
|---|---|---|---|---|---|---|---|---|---|---|---|---|---|
| **Clone ID** | **kₐ [M⁻¹ s⁻¹]** | **k_{d} [s⁻¹]** | **K_{D} [M]** | **kₐ [M⁻¹s⁻¹]** | **k_{d} [s⁻¹]** | **K_{D} [M]** | **cyno CD137 / human CD137** | **kₐ [M⁻¹s⁻¹]** | **k_{d} [s⁻¹]** | **K_{D} [M]** | **mouse CD137 / human CD137** | **% inhibition** | **% relative binding to control** |
| **38-02-A04** | 4.38E+05 | 1.00E-06 | 2.29E-12 | 1.26E+05 | 1.00E-06 | 7.94E-12 | 3.46 | 8.89E+04 | 1.00E-06 | 1.13E-11 | 0.20 | 17.02 | 92.31 |
| **38-27-C05** | 5.96E+05 | 2.86E-04 | 4.80E-10 | 2.38E+05 | 1.96E-04 | 8.26E-10 | 1.72 | 1.08E+05 | 2.43E-04 | 2.25E-09 | 0.21 | 3.22 | 87.44 |

In a next step, selected clones were used for RNA isolation and RT-PCR to amplify the sequence of rabbit antibody light and heavy chain variable regions (38-02-A04 and 38-27-C05).

### Example 4: Selection of clones for humanization

Based on data obtained during hit screening, 2 mouse cross-reactive CD137 binder 38-02-A04 and 38-27-C05 were humanized by grafting the CDRs on VH4 based framework and implemented into a scDb format together with an anti-PD-L1 domain. In addition, CDR regions of the selected clones were also grafted on VH3 based framework and produced in a scFv format. In order to achieve the best affinity and potency, further optimization with 2 different structural grafts (STR and IF grafting) was done for one clone which displayed the best affinity to human CD137 as scDb, namely 38-02-A04.

### THE MULTISPECIFIC MOLECULES COMPRISING THE ANTIBODY OF THE

### INVENTION OR ANTIGEN-BINDING FRAGMENT THEREOF

The exemplary multispecific molecules comprising the antibody of the invention or antigen-binding fragment thereof are included in Table 3.

### Example 1: Affinities to PD-L1, CD137, HSA and MSA.

### Methods:

Affinity to PD-L1 of the different species was determined by SPR measurements using a Biacore T200 device (GE Healthcare). An antibody specific for the Fc region of human IgGs was immobilized on a sensor chip (CM5 sensor chip, GE Healthcare) by amine-coupling. For all formats, with the exception of the Fc containing Morrison formats, PD-L1-Fc chimeric protein from different species were captured by the immobilized antibody. Three-fold serial dilutions of the molecules specific for PD-L1 (0.12-90 nM) were injected into the flow cells for three minutes and dissociation was monitored for 10 minutes. After each injection cycle, surfaces were regenerated with one injection of a 3 M MgCl₂ solution. The apparent dissociation (k_{d}) and association (kₐ) rate constants and the apparent dissociation equilibrium constant (KD) were calculated using one-to-one Langmuir binding model. Affinity to CD137 of the different species was determined using the identical setup as for PD-L1 with the exception that CD137-Fc chimeric protein from different species were captured by the immobilized antibody.

The Fc containing formats were directly captured by the antibody specific for the Fc region of human IgGs. Two-fold serial dilutions of PD-L1 extracellular domain or CD137 extracellular domain ranging from 90 to 0.35 nM were tested for binding to the IgG captured on the biosensor chip. After each injection cycle, surfaces were regenerated with one injection of a 3 M MgCl2 solution.

Affinity of molecules to serum albumin (SA) of the different species was determined by SPR measurements using a Biacore T200 device (GE Healthcare). SA was directly coupled to a CM5 sensor chip (GE Healthcare) using amine coupling chemistry. After performing a regeneration scouting and surface performance test to find best assay conditions, a dose response was measured and obtained binding curves were double-referenced (empty reference channel and zero analyte injection) and fitted using the 1:1 Langmuir model to retrieve kinetic parameters. The assay was run in a 1 X PBS-Tween buffer at pH 5.5.

### Results:

The measurements of the binding kinetics for the humanized constructs show a difference in binding affinity for PD-L1 when comparing the CDR and structural grafts of clone 33-03-G02 the STR graft shows a 20-fold improvement in affinity compared to the CDR graft of the same clone (PRO885 versus PRO1126 in Table 6). The CDR graft derived from clone 37-20-B03 (PRO997) shows approximately two fold higher affinity when compared to the STR graft of clone 33-03-G02. The binding affinities for the CDR graft of 33-03-G02 are similar to the binding affinity of the parental scFv when they are combined into different multispecific formats (compare PRO830 to PRO885, PRO951, PRO1123, PRO1124, PRO963, PRO966, PRO1057, PRO1058, PRO1059 and PRO1060 in Table 6). The scFv derived from both clones show nearly identical affinity to human and cynomolgus monkey PD-L1 (see PRO977 and PRO830 in Table 6).

The measurement of binding kinetics for the CDR grafts of the two CD137 specific humanized constructs derived from clone 38-02-A04 and 38-27-C05 show nearly identical affinities (compare PRO885 and PRO951 in Table 6). For clone 38-02-04 the described structural residues engrafted in the framework regions led to an improvement of affinity of more than 200-fold (compare PRO885 and PRO1124 in Table 6). In addition for constructs derived from clon 38-02-04 binding to mouse CD137 was observed, however with very much reduced affinity.

Binding of triabodies to serum albumin (SA) was confirmed by SPR. The triabodies containing the SA binding domain derived from clone 19-01-H04 show high affinity binding to human serum albumin, while no binding was observed for rodent SA. For the triabodies containing clone 23-13-A01 binding was observed to human SA, in addition the molecules bind with reduced affinity to rodent SA (see Table 6).

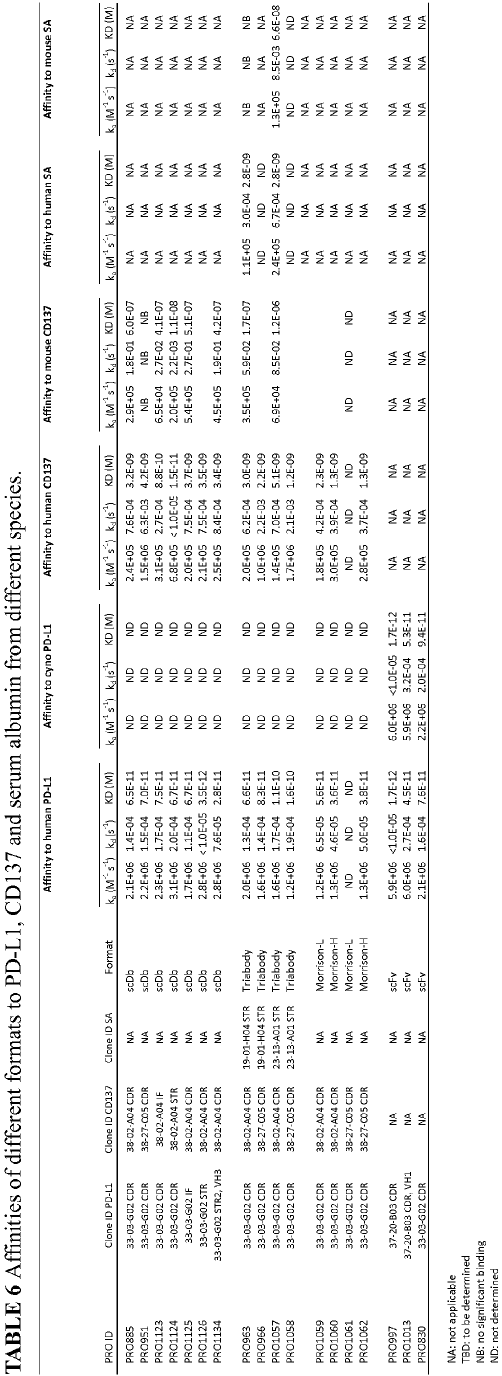

### Example 2: Blockade of the PD-L1/PD-1 interaction in a cell-based reporter gene assay using CHO cells expressing PD-L1 and a TCR activator molecule, and Jurkat cells expressing PD-1 and containing a luciferase gene under the NFAT response element.

### Methods

In the bioluminescent reporter gene assay, engineered Jurkat T cells stably expressing NFAT (nuclear factor of activated T-cells)-luciferase reporter and human PD-1 act as effector T cells. Cells stably expressing human PD-L1 and a T cell receptor (TCR) activator act as antigen presenting cells. Co-cultivating the two cell lines induces activation of the Jurkat NFAT pathway via crosslinking of TCR activator/TCR complex. Upon engagement of PD-L1 expressing cells, PD-1 signaling in PD-1 effector T cells inhibits T-cell function, and results in NFAT pathway inhibition. Blockade of PD-1 and PD-L1 receptor interaction leads to re-activation of the NFAT pathway.

35'000 CHO/PD-L1/TCR activator (BPS Bioscience) cells in 100 µl of cell culture medium (DMEM/F12, 10% FCS) were added to the inner wells of a white cell culture plate and incubated for 16-20h at 37°C and 5% CO₂. Next day, 95 µl of cell culture medium was removed from each well and 50 µl of 2-fold concentrated serial dilutions of the respective molecules to be tested (from 3000 to 0.46 ng/ml), including the reference Avelumab, were added. Then, 50 µl of effector Jurkat cells expressing PD-1 (BPS Bioscience) diluted at 400'000 cell/ml in assay buffer (RPMI1640 with 10% FCS) were added to each well and plates were incubated 6h at 37°C and 5% CO₂. Finally, 50 µL luciferase substrate (BPS Bioscience) prepared according to manufacturer's protocol, was added per well and plates were incubated 30 min in the dark, luminescence was measured using Topcount.

### Results

In order to assess the influence of the CDR set and framework selection on potency to neutralize the PD-L1 binding to PD-1, three anti-PD-L1 scFvs were tested in the NFAT reporter gene cell-based assay. PRO830 comprises the CDR set of clone 33-03-G02 grafted on a VH4 framework and PRO997 and PRO1013 comprise the CDR set of clone 37-20-B03 grafted on either a VH4 or a VH1 framework, respectively. PRO830 has the lowest potency of the three scFvs tested with an IC₅₀ value of 42.88 ng/ml, and has similar potency as Avelumab with an IC₅₀ value of 34.09 ng/ml. PRO997 is the most potent molecule. Potency of the same CDR set was about 2-fold higher when grafted on a VH4 framework than on VH1 framework. IC₅₀ values were 11,12 ng/ml versus 21.29 ng/ml, respectively. (Figure 1 and Table 7)

Neutralization potency of the PD-L1 binding to PD-1 was determined for bi-specific molecules possessing the 33-03-G02 PD-L1 domain before (CDR graft) and after (structural graft) domain optimization. The CDR graft (PRO885) was compared to a structural graft (PRO1126). The domain optimization improved the neutralization potency by a factor of three with IC₅₀ values being 137.2 ng/ml for PRO885 and 48.15 ng/ml for PRO1126. (Figure 2 and Table 7).

Potency to neutralize the PD-L1/PD-1 interaction was also assessed for two tri-specific molecules possessing the anti-PD-L1 domain of the CDR graft of clone 33-03-G02 and two different human serum albumin binding domain, for half-life extension. The HSA domain of PRO1057 is also binding mouse serum albumin. Experiments were performed in presence of 25 mg/ml HSA. Neutralization potency (IC₅₀ = 665.1 ng/ml) was lower than for Avelumab. (Figure 3 and Table 7)

Another format which would extend the half-life in serum, the so-called Morrison format, was tested in the cell based potency reporter gene assay. In this format, one specificity is carried by the IgG moiety (bi-valency) and two scFvs with specificities to the second target are linked by flexible peptide linkers either to the heavy chain (HC) or light chain (LC) of the IgG. All Morrison molecules tested carried the anti-PD-L1 domain of the CDR graft of clone 33-03-G02 on both IgG arms. The two constructs PRO1059 and PRO1060 differ by the fusion of two anti-CD137 scFvs either on the heavy chain (HC) or to the (LC),. PRO1062 has the same architecture as PRO1060 with a different CD137 domain. Neutralization potencies of all molecules were similar (Figure 4 and Table 7).

**TABLE 7 Neutralization of PD-L1 PD-1 interaction in the NF-AT reporter gene assay.**

| | | | | | **Neutralization of PD-Llin NF-AT Potency assay** | | |
|---|---|---|---|---|---|---|---|
| PRO ID | Clone ID PD-L1 | Clone ID CD137 | Clone ID SA | Format | IC₅₀ (ng/ml) | rel. IC₅₀^{*} | HSA |
| PRO885 | 33-03-G02 CDR | 38-02-A04 CDR | NA | scDb | 137.20 | 0.28 | no |
| PRO951 | 33-03-G02 CDR | 38-27-C05 CDR | NA | scDb | 88.50 | 0.47 | no |
| | | | | | | | |
| PRO963 | 33-03-G02 CDR | 38-02-A04 CDR | 19-01-H04 STR | Triabody | 274.80 | 0.25 | yes |
| PRO1057 | 33-03-G02 CDR | 38-02-A04 CDR | 23-13-A01 STR | Triabody | 665.10 | 0.10 | yes |
| | | | | | | | |
| PRO1059 | 33-03-G02 CDR | 38-02-A04 CDR | NA | Morrison-L | 93.76 | 0.52 | no |
| PRO1060 | 33-03-G02 CDR | 38-02-A04 CDR | NA | Morrison-H | 132.70 | 0.44 | no |
| PRO1062 | 33-03-G02 CDR | 38-27-C05 CDR | NA | Morrison-H | 96.55 | 0.68 | no |
| | | | | | | | |
| PRO997 | 37-20-B03 CDR | NA | NA | scFv | 11.12 | 3.07 | no |
| PRO1013 | 37-20-B03 CDR, VH1 | NA | NA | scFv | 21.29 | 1.60 | no |
| PRO830 | 33-03-G02 CDR | NA | NA | scFv | 42.88 | 0.73 | no |

| | | | | | | | |
|---|---|---|---|---|---|---|---|
| NA: not applicable ^{*}: IC_{50, Avelumab} (ng/ml)/IC_{50, test molecule}(ng/ml) | | | | | | | |

### Example 3: Blockade of the interaction of PD-L1 with PD-1 and B7.1 using competition ELISA.

These assays were performed to assess the ability of PD-L1 inhibitors to block the interaction between PD-L1 and PD-1 or PD-L1 and B.71. Different formats including scFvs, scDbs, scDb-scFv and Morrison were analyzed in the competition ELISA and compared to the reference IgG Avelumab.

### PD-L1/PD-1 competition ELISA

### Method

ELISA microplates coated overnight at 4°C with 4 µg/ml human PD-1 were washed three times with 450 µl wash buffer per well. Plates were blocked for 1 hour at room temperature by adding 300 µl of PBS with 1%BSA and 0.2% tween (dilution buffer) to each well. Inhibitors were serially diluted in 3-fold steps to final concentrations ranging from 300 to 0.005 ng/ml in dilution buffer containing 1 ng/ml biotinylated human PD-L1. The mixtures were pre-incubated for 1 hour at room temperature under gentle agitation on a rotating mixer (21 rpm) and added to the microplates after 3 wash cycles with 450 µl wash buffer per well. Plates were incubated for 1.5 hours at room temperature under gentle agitation, then 10 ng/ml streptavidin-polyHRP40 was added to each microplate well after three washes with 450 µl of wash buffer per well. After 1h incubation at RT, plates were washed three times with 450 µl wash buffer and TMB substrate solution was added. The enzymatic reaction was stopped after 6 minutes by addition of 1M HCl and absorbance was measured at 450 nm using 690 nm as a reference wavelength. For calculation of IC₅₀ values, a four-parameter logistic (4PL) curve fit was performed in Graph Pad Prism using reference subtracted values.

### Results

As illustrated in Figure 5, all PD-L1 inhibitors blocked the interaction of PD-1 with PD-L1 when tested in the competition ELISA. The scFv PRO830 blocked the interaction with similar potency while PRO997 and PRO1013 exhibited significantly lower IC₅₀ values than Avelumab and are thus more potent inhibitors. When combined into multispecific formats, i.e. scDbs or Morrisons, all molecules conserved their inhibiting properties. PRO885 was less potent than Avelumab whereas a lower IC₅₀ value was determined for PRO1126 comprising an improved anti-PD-L1 domain. The Morrison formats were slightly less potent when compared to Avelumab. The neutralizing effect of PRO1057 was also shown in presence of human serum albumin, where IC₅₀ values were approximately two-fold higher.

### PD-L1/B7.1 competition ELISA

### Method

ELISA microplates coated overnight at 4°C with 4 µg/ml human B7.1 were washed three times with 450 µl wash buffer per well. Plates were blocked for 1 hour at room temperature by adding 300 µl of PBS with 1%BSA and 0.2% tween (dilution buffer) to each well. Inhibitors were serially diluted in 3-fold steps to final concentrations ranging from 900 to 0.015 ng/ml in dilution buffer containing 40 ng/ml biotinylated PD-L1. The mixtures were pre-incubated for 1 hour at room temperature under gentle agitation on a rotating mixer (21 rpm) and added to the microplates after 3 wash cycles with 450 µl wash buffer per well. Plates were incubated for 1.5 hours at room temperature under gentle agitation, then 10 ng/ml streptavidin-polyHRP40 was added to each microplate well after three washes with 450 µl of wash buffer per well. After 1h incubation at RT, plates were washed three times with 450 µl wash buffer and TMB substrate solution was added. The enzymatic reaction was stopped after 6 minutes by addition of 1M HCl and absorbance was measured at 450 nm using 690 nm as a reference wavelength. For calculation of IC₅₀ values, a four-parameter logistic (4PL) curve fit was performed in Graph Pad Prism using reference subtracted values.

### Results

Except for PRO1126, all PD-L1 inhibitors were also tested for their ability to block the interaction of PD-1 with B7.1. PRO830 showed similar potency than Avelumab whereas lower IC₅₀ values were determined for PRO997 and PRO1013. All scDbs and Morrisons also inhibited the interaction between PD-L1 and B.7-1. The scDb PRO885 exhibited similar potency than Avelumab whereas the IC₅₀ values for the Morrisons were about 2 -3.4 fold lower.

### Example 4: No inhibition of CD137 and CD137 neutralization by humanized anti-CD137 domains.

### Methods:

To show that PRO885 does not interfere with the binding of CD137 ligand (CD137L) to CD137, a competitive ELISA was employed. The commercial inhibitory polyclonal anti-CD137 goat antibody (Antibodies online, Cat# ABIN636609) served as a reference. In brief, CD137 was coated on the ELISA plate overnight and serial dilutions of PRO885 were added to the ELISA plate. Afterwards, biotinylated CD137L was added and bound ligand was detected by addition of Streptavidin-HRP. Finally, the HRP substrate TMB was added. After development for 5 min, the reaction was stopped with 1 M HCl solution. The absorbance was measured at 450 nm and 690 nm as reference.

### Results:

The titration curves obtained for PRO885 containing the CD137 domain derived from clone 38-02-A04 are represented in Figure 7A and the binding curves obtained for PRO951 containing the CD137 domain derived from clone 38-27-C05 are represented in Figure 7B. While the reference antibody completely prevented binding of CD137L to CD137, PRO885 and PRO951 did not significantly inhibit the CD137L binding to CD137 and therefore were defined as non-neutralizing.

**TABLE 8 Blockade of the interaction of PD-L1 with PD-1 and B7.1 using competition ELISA.**

| | | | | | **Blocking of PD-L1/ PD-1 interaction** | | **Blocking of PD-L1/ B7.1 interaction** | |
|---|---|---|---|---|---|---|---|---|
| PRO ID | Clone ID PD-L1 | Clone ID CD137 | Clone ID SA | Format | IC₅₀ (ng/ml) | rel. IC₅₀^{*} | IC₅₀ (ng/ml) | rel. IC₅₀^{*} |
| PRO885 | 33-03-G02 CDR | 38-02-A04 CDR | NA | scDb | 8.35 | 0.17 | 12.2 | 0.59 |
| PRO951 | 33-03-G02 CDR | 38-27-C05 CDR | NA | scDb | 9.50 | 0.15 | 9.30 | 0.78 |
| PRO1126 | 33-03-G02 STR | 38-02-A04 CDR | NA | scDb | 1.28 | 1.59 | TBD | TBD |
| | | | | | | | | |
| PRO1057 | 33-03-G02 CDR | 38-02-A04 CDR | 23-13-A01 STR | Triabody | 8.61 | 0.20 | 16.29 | 0.53 |
| | | | | | | | | |
| PRO1059 | 33-03-G02 CDR | 38-02-A04 CDR | NA | Morrison-L | 4.54 | 0.37 | 28.98 | 0.30 |
| PRO1060 | 33-03-G02 CDR | 38-02-A04 CDR | NA | Morrison-H | 5.67 | 0.30 | 17.42 | 0.49 |
| PRO1062 | 33-03-G02 CDR | 38-27-C05 CDR | NA | Morrison-H | 11.33 | 0.32 | 19.53 | 0.51 |
| | | | | | | | | |
| PRO997 | 37-20-B03 CDR | NA | NA | scFv | 0.50 | 4.16 | 6.359 | 2.34 |
| PRO1013 | 37-20-B03 CDR, VH1 | NA | NA | scFv | 0.57 | 3.67 | 4.05 | 3.68 |
| PRO830 | 33-03-G02 CDR | NA | NA | scFv | 3.40 | 0.61 | 12.87 | 1.16 |

| | | | | | | | | |
|---|---|---|---|---|---|---|---|---|
| NA: not applicable ^{*}: IC_{50, Avelumab} (ng/ml)/IC_{50, test molecule}(ng/ml) | | | | | | | | |

### Example 5: Epitope binning of 38-02-A04 and 38-27-C05 against Urelumab and Utolimumab.

### Methods:

The binding epitopes on CD137 of the proteins PRO885 (scDb containing 38-02-A04 CDR graft), PRO951 (scDb containing 38-27-C05 CDR graft) and the competitor molecules Urelumab (BMS) and Utomilumab Pfizer) were compared in a SPR epitope binning assay using a MASS-1 device (Sierra Sensors). A sandwich setup was chosen to examine if the molecules block one another's binding to CD137. Therefore, PRO885 and PRO951 were immobilized on high capacity amine sensor chips (HCA, Sierra Sensors). Then, 90 nM of the antigen CD137 was captured on the scDbs, followed immediately by an injection of 22.5 nM of the second antibody (PRO885, PRO951, Urelumab and Utolimumab).

The capture levels of CD137 on each protein and the second binder response levels were determined (response units, RU). By calculating the theoretical maximum response (Rmax), which depends on the molecular weights of the involved proteins and the capture levels, the relative binding level (%) of the proteins on the captured antigen were determined. If the molecules bind overlapping or similar epitopes on CD137, no binding of the antibody injected over the captured CD137 should be observed. Consequently, when binding of the antibody is observed the two antibody pairs bind non-overlapping epitopes.

### Results:

When PRO885 was immobilized on a sensor chip, all 3 antibodies PRO951, Urelumab and Utomilumab showed binding to CD137 captured by PRO885. As expected, no binding was observed for PRO885 that was used a control (Figure 8 and Figure 9). When PRO951 was immobilized on the sensor chip, Urelumab and PRO885 showed binding while Utomilumab and PRO951 that was used as control did not show any significant binding (Figure 8 and Figure 10). These results show that PRO885 derived from clone 38-02-A04 binds to different epitopes on CD137 than Urelumab, Utolimumab and PRO951 derived from 38-27-C05. In contrast, RO951 bind to an epitope that is overlapping with Utolimumab but not with Urelumab and PRO885.

### Example 6: Assessment of the CD137 agonistic effect of anti-PD-L1xCD137 molecules by using a cell-based assay of transgenic NFkB Jurkat reporter cell line expressing CD137.

### Introduction

In this assay the activation of CD137 signaling in Jurkat cells was assessed. The activitiy of CD137 signaling is reported by measurement of Luciferase expression which is driven by CD137 induced NF-kB activation in a Jurkat reporter cell line. The expression of Luciferase directly correlates with the activity of CD137. Moreover, clustering of CD137 which is required for activation of the signal pathway is facilitated via then formation of an immunological synapse between the Jurkat cells and a PD-L1 expressing cell line. Therefore, PD-L1 expression is needed for clustering and activation of CD 137 on the reporter cell line.

### Methods

PD-L1 expressing CHO (clone A2) and HCC827 cells unstimulated or stimulated for 24h with 10 ng/ml IFNy to increase PD-L1 expression were seeded at 25'000 cells per well on 96-well culture plates. As a negative control, CHO WT cells without PD-L1 expression were seeded at the same cell density. Then, serial dilutions of the anti-PD-L1xCD137 molecules as well as the competitor Urelumab were prepared and added to the cells. Next, Jurkat reporter cells were prepared in assay medium containing HSA at 25 mg/ml or without and added at a cell density of 40'000 cells per well. Luciferase expression was detected by addition of Luciferase reagent and was read by a luminescence reader 6 or 24h after addition of Jurkat cells. Data were analyzed by normalization the relative luminescence units (RLU) of the test samples to the RLU measured for Urelumab yielding values of the relative activation of CD137 signaling.

### Results

### I. Test of PRO885 and PRO951 using CHO-PD-L1 cells:

As shown in Figure 11, PRO885 and PRO951 activated CD137 signaling more efficient in the presence of PD-L1 expressing CHO cells than Urelumab. PRO885 showed the best potency and highest signal of activation (PRO885, EC₅₀ = 11.72 ng/ml, PRO951: EC₅₀ = 33.68 ng/ml; Urelumab: EC₅₀ = 79.11 ng/ml, Table 9). In the absence of PD-L1, neither PRO885 nor PRO951 could activate CD137 in reporter cells while Urelumab showed activation of CD 13 7 signaling independently of PD-L1.

**TABLE 9 EC50 values for anti-PD-L1xCD137 molecules using CHO-PD-L1 cells.**

| | Urelumab | PRO885 | PRO951 |
|---|---|---|---|
| Bottom | -0.4628 | -8.1 | -4.066 |
| Top | 101.5 | 491.2 | 411.4 |
| EC50 in ng/ml | 79.11 | 11.72 | 33.68 |
| R square | 0.995 | 0.9922 | 0.9899 |

### II. Test of STR-grafted scDbs using CHO-PD-L1 cells:

As shown in Figure 12 and Table 10, the anti-PD-L1xCD137 scDb molecules stimulated CD137 signaling more efficiently than Urelumab. In contrast to Urelumab, the stimulatory effect was only seen for the scDb when PD-L1 expressing target cells were present. All scDbs showed identical potency to stimulate Nf-kB reporter gene activation in presence of CHO cells expressing PD_L1 at high levels. Next, the same molecules were tested in the presence of cells expressing a lower amount of PD-L1.

### III. Test of STR-grafted scDb using HCC827 cells without IFNy:

As shown in Figure 13 and Table 11, the anti-PD-L1xCD137 scDb molecules stimulated CD137 signaling more efficiently than Urelumab. The scDbs with affinity improved CD137 domain (STR grafts of 38-02-A04, PRO1120 and PRO1124) showed an improved potency in CD137 activation when compared to the CDR grafted CD137 domain (for instance, PRO885, EC₅₀ = 13.02 ng/ml, PRO1124: EC₅₀ = 5.62 ng/ml, Table 11). Of note, increased affinity to PD-L1 as it was found for the STR graft of the PD-L1 domain (Pro1126) also resulted in increased potency when compared to the parental molecule PRO885 (PRO885, EC₅₀ = 13.02 ng/ml, PRO1126: EC₅₀ = 6.97 ng/ml, Table 11). At high concentrations, the STR grafted scDb showed a tendency of decreasing signal of activation. This was more pronounced for molecules having a STR grafted CD137 domain (PRO1120 and PRO1126). Interestingly, when the STR graft of the PD-L1 domain was combined with the CDR graft of CD137 the signal decrease at high concentrations was not observed (compare PRO885 and PRO1124 in Figure 13). Thus, potency increased slightly with increasing affinity to CD137 and PDL1. A signal decrease at high concentrations (bell-shaped curve) was more pronounced with increasing affinity to CD 137, while increased affinity to PDL1 did not contribute to this effect. Thus, rather the ratio between affinity to CD137 and PD-L1, than the absolute affinities of each domain seem critical to extend the concentration window of maximal activity.

| | | **Affinity to human CD137: SPR data** | | | | **Affinity to mouse CD137: SPR data** | | | | | **Affinity to human PD-L1: SPR data** | | | | | |
|---|---|---|---|---|---|---|---|---|---|---|---|---|---|---|---|---|
| **PRO Number** | **Protein description** | **kₐ (M⁻¹s⁻¹]** | **k_{d} [s⁻¹]** | **K_{D} [M]** | **Binding level normalized to theoretical Rmax (%)** | | **kₐ [M⁻¹s⁻¹]** | **k_{d} [s⁻¹]** | **K_{D} [M]** | **Binding level normalized to theoretical Rmax (%)** | | **kₐ [M⁻¹s⁻¹]** | **k_{d} [s⁻¹]** | **K_{D} [M]** | **Binding level normalized to theoretical Rmax (%)** | |
| PRO1118 | **38-02-A04** sc01 scDb-i/33_02_G02 sc01 scDb-o | 2.75E+05 | 7,23E-04 | 2.63E-09 | 79.8 | | 8.29E+04 | 2.27E-04 | 2.73E-09 | 11.5 | | 1.34E+06 | < 1.00E-05 | < 7.46E-12 | 79.2 | |
| | | | | | | | | | | | | | | | | |
| PRO119 | **38-02-A04** sc05 IF scDb-i/33_02_G02 sc01 scDb-o | 3.81E+05 | 2.76E-04 | 7.24E-10 | 79.1 | | 1.40E+05 | 2.48E-02 | 1.77E-07 | 81.0 | | 1.50E+06 | < 1.00E-05 | < 6.67E-12 | 77.5 | |
| | | | | | | | | | | | | | | | | |
| PRO1120 | 38-02-A04 sc06 Full scDb-i/33_02_G02 sc01 scDb-o | 5.78E+05 | <1.00E-05 | < 1.73E-11 | 74.7 | | 2.12E+05 | 3.25E-03 | 1.54E-08 | 87.5 | | 1.27E+06 | <1.00E-05 | < 7.87E-12 | 76.8 | |

### IV. Test of STR-grafted scDb using HCC827 cells with IFNγ:

Stimulation of HCC827 with IFNy led to increased signal of activation without changing the potency of the scDb molecules. Of note, the drop of signal at high concentration of the tested scDb was less obvious in this setting suggesting a correlation with PD-L1 expresion (Figure 14 and Table 12).

### V. Test of long half-life molecules using CHO-PD-L1 cells:

As shown in Figures 17 and 18 and Tables 13 and 14, tested long half-life anti-PD-L1xCD137 molecules stimulated CD137 signaling to the same extend as Urelumab did. There was a difference when the Morrision formats (PRO1060, PRO1062) were compared to the scDb-scFv formats (PRO1057, PRO1058). While the Morrison formats showed a higher potency, the maximum signal of activation was substantially increased when the scDb-scFv were tested. Of note, after 24h of incubation PRO1057 showed a remarkable high signal of activation. All tested long half-life molecules activated CD137 signaling only in the presence of PD-L1 expressing cells. Interestingly, despite similar affinities to both targets, PRO1057 showed a much higher maximal signal than PRO1058. And further, the monovalent scDb-scFv PRO1057 showed stronger activation than the respective bivalent Morrison format PRO1060.

### VI. Test of long half-life molecules using HCC827 cells without and with IFNy

As shown in Figure 17 and Table 15, tested long half-life anti-PD-L1xCD137 molecules stimulated CD137 signaling to the same extend as Urelumab in the presence of cells expressing lower amounts of PD-L1. The maximum activation of tested molecules was further increased when the target cells were stimulated by IFNy suggesting a direct correlation of CD 13 7 activation with the levels of PD-L1 expression on the target cells. As already stated above, PRO1057 showed a higher level of reporter gene activation when compared to the Morrison formats.

**TABLE 10 EC₅₀ values for anti-PD-L1xCD137 molecules using CHO-PD-L1 cells.**

| | Urelumab | PRO885 | PRO1118 | PRO1119 |
|---|---|---|---|---|
| Bottom | -0.5169 | -6.408 | -2.387 | 2.039 |
| Top | 99.99 | 249.1 | 245.3 | 238.9 |
| EC50 in ng/ml | 79.47 | 5.135 | 4.596 | 4.22 |
| R square | 0.9637 | 0.9708 | 0.9761 | 0.9726 |

| | Urelumab | PRO885 | PRO1120 | PRO1123 |
|---|---|---|---|---|
| Bottom | -0.6273 | 0.5392 | 6.497 | -1.816 |
| Top | 100 | 222.1 | 201.1 | 219.2 |
| EC50 in ng/ml | 104.2 | 4.856 | 4.689 | 5.753 |
| R square | 0.9677 | 0.9769 | 0.963 | 0.9568 |

| | Urelumab | PRO885 | PRO1124 | **PRO1126** |
|---|---|---|---|---|
| Bottom | -0.499 | -11.51 | 2.552 | -2.922 |
| Top | 100 | 253.8 | 228.6 | 242.1 |
| EC50 in ng/ml | 86.51 | 6.299 | 3.681 | 5.997 |
| R square | 0.979 | 0.9714 | 0.9561 | 0.9467 |

**TABLE 11 EC₅₀ values for anti-PD-L1xCD137 molecules using HCC827 cells without IFNγ.**

| | Urelumab | PRO885 | PRO1118 | PRO1119 |
|---|---|---|---|---|
| Bottom | -2.035 | -0.5477 | -0.3666 | 0.1789 |
| Top | 100 | 96.45 | 81.98 | 74.91 |
| EC50 in ng/ml | 212.5 | 11.5 | 8.559 | 5.045 |
| R square | 0.9815 | 0.9852 | 0.995 | 0.9725 |

| | Urelumab | PRO885 | PRO1120 | PRO1123 |
|---|---|---|---|---|
| Bottom | -1.986 | -0.2298 | 0.0807 | -0.8367 |
| Top | 99.96 | 111.1 | 126.5 | 123.2 |
| EC50 in ng/ml | 109.9 | 13.06 | 6.685 | 16.12 |
| R square | 0.9895 | 0.9759 | 0.9609 | 0.96 |

| | Urelumab | PRO885 | PRO1124 | PRO1126 |
|---|---|---|---|---|
| Bottom | -1.384 | -0.3237 | -1.084 | -8.28 |
| Top | 99.98 | 118.7 | 113.2 | 135.1 |
| EC50 in ng/ml | 111.7 | 13.02 | 5.616 | 6.966 |
| R square | 0.9941 | 0.9816 | 0.9875 | 0.9577 |

**TABLE 12 EC₅₀ values for STR grafted scDb using HCC827 cells stimulated with IFNγ.**

| | Urelumab | PRO885 | PRO1118 | PRO1119 |
|---|---|---|---|---|
| Bottom | -1.208 | -1.446 | -1.564 | 0.1399 |
| Top | 100 | 167.4 | 146.5 | 139.9 |
| EC50 in ng/ml | 114.2 | 9.266 | 7.965 | 4.855 |
| R square | 0.9939 | 0.9803 | 0.996 | 0.9767 |

| | Urelumab | PRO885 | PRO1120 | PRO1123 |
|---|---|---|---|---|
| Bottom | -1.009 | -2.023 | 0.1813 | -3.584 |
| Top | 99.98 | 134.2 | 117.3 | 154.4 |
| EC50 in ng/ml | 144.8 | 8.883 | 5.15 | 11.87 |
| R square | 0.9811 | 0.9795 | 0.9554 | 0.9883 |

| | Urelumab | PRO885 | PRO1124 | PRO1126 |
|---|---|---|---|---|
| Bottom | -1.28 | -0.7258 | 1.572 | -1.561 |
| Top | 100 | 165.8 | 188.5 | 207.7 |
| EC50 in ng/ml | 108.2 | 9.229 | 4.833 | 5.48 |
| R square | 0.9976 | 0.9764 | 0.9906 | 0.9825 |

**TABLE 13 EC₅₀ values for long half-life molecules using PD-L1 expressing CHO cells (6h).**

| | Urelumab | PRO885 | PRO1060 | PRO1062 |
|---|---|---|---|---|
| Bottom | -0.09762 | 6.019 | -5.304 | -1.947 |
| Top | 99.96 | 337.5 | 220.4 | 199.1 |
| EC50 in ng/ml | 56.45 | 7.843 | 56.42 | 52.16 |
| R square | 0.9948 | 0.9474 | 0.9874 | 0.993 |

| | Urelumab + HSA | PRO885 + HSA | PRO1057 + HSA | PRO1058 + HSA |
|---|---|---|---|---|
| Bottom | -0.8837 | 6.69 | -7.721 | -0.5282 |
| Top | 106.3 | 492.8 | 462.9 | 40.06 |
| EC50 in ng/ml | 77.72 | 7.916 | 111.5 | 70.93 |
| R square | 0.9957 | 0.9457 | 0.9943 | 0.6132 |

**TABLE 14 EC₅₀ values for long half-life molecules using PD-L1 expressing CHO cells (24h).**

| | Urelumab | PRO885 | PRO1060 | PRO1062 |
|---|---|---|---|---|
| Bottom | 0.2207 | -11.83 | -5.151 | -3.914 |
| Top | 88.59 | 196.1 | 141.1 | 130.5 |
| EC50 in ng/ml | 46.63 | 13.75 | 80.85 | 86.87 |
| R square | 0.9739 | 0.9869 | 0.991 | 0.9737 |

| | Urelumab + HSA | PRO885 + HSA | PRO1057 + HSA | PRO1058 + HSA |
|---|---|---|---|---|
| Bottom | -0.3454 | -5.805 | -7.71 | -0.4289 |
| Top | 90.45 | 258.5 | 744.9 | 20.53 |
| EC50 in ng/ml | 78.17 | 14.85 | 792.4 | 121.4 |
| R square | 0.9893 | 0.9812 | 0.9955 | 0.6294 |

**TABLE 15 EC₅₀ values for long half-life molecules using HCC827 cells stimulated with IFNγ.**

| | HCC827 without IFNy stimulation | | | |
|---|---|---|---|---|
| | Urelumab | PRO885 | PRO1057 (+HSA) | PRO1060 |
| Bottom | -0.7119 | -5.806 | 0.08644 | -0.6993 |
| Top | 85.16 | 136.6 | 63.18 | 43.56 |
| EC50 in ng/ml | 47.49 | 9.518 | 76.61 | 13.57 |
| R square | 0.975 | 0.9838 | 0.9478 | 0.9255 |

| | HCC827 with IFNy stimulation | | | |
|---|---|---|---|---|
| | Urelumab | PRO885 | PRO1057 (+HSA) | PRO1060 |
| Bottom | -2.649 | -3.386 | -3.323 | -1.177 |
| Top | 91.62 | 140.2 | 136.6 | 83.61 |
| EC50 in ng/ml | 52.77 | 7.515 | 115 | 17.51 |
| R square | 0.9743 | 0.9901 | 0.9911 | 0.972 |

### Example 7: Assessment of T cell stimulatory effect of concomitant PD-L1 blockade and CD137 stimulation in a cell-based assay using human PBMC and transgenic CHO cells expressing PD-L1.

### Method

CHO-A2 cells expressing PD-L1 were seeded at three different densities, ranging from 50000 to 200000 cells per well on 96-well culture plates pre-coated with an anti-human CD3 antibody. The plates were incubated overnight at 37 °C, 5% CO₂. On the next day, peripheral blood mononuclear cells (PBMC) were isolated from fresh human whole blood by means of density gradient centrifugation. 100000 PBMCs per well were added to the 96-well plate, followed by the addition of the anti-PD-L1xCD137 scDb PRO885 at concentrations of 500, 50 and 5 ng/ml. After 76 hours of incubation, cell supernatants were harvested. Human interleukin-2 (IL-2) levels in the culture supernatants were quantified using the IL-2 human ELISA MAX assay from Biolegend, according to kit instructions. IL-2 concentrations were interpolated from a IL-2 standard curve, back-calculated and plotted against PRO885 concentrations for calculation of EC50 values.

### Results

**TABLE 17 Induction of IL-2 secretion by T cells upon treatment with PRO885.**

| ug/ml of anti-CD3 antibody | 2 | | | 1 | | |
|---|---|---|---|---|---|---|
| CHO-A1 cells/well | 50000 | 100000 | 200000 | 50000 | 100000 | 200000 |
| EC50 (ng/ml) | 52.01 | 41.85 | 30.05 | 12.85 | 86.22 | 80.62 |

As shown in Figure 18, IL-2 was secreted by T cells following concomitant blockade of PD-1/PD-L1 interaction and stimulation of CD137 by the addition of the bispecific molecule PRO885. Secreted IL-2 levels increased with augmenting anti-CD3 antibody and CHO-A2 cell-densities, and increasing PRO885 concentrations. In the absence of anti-CD3 antibodies, IL-2 levels were comparable to basal IL-2 secretion. PRO885 only activated T-cells co-stimulated by an anti-CD3 antibody. This finding demonstrates that PRO885 only stimulates activated T-cells and suggests that *in vivo* PRO885 would specifically stimulate tumor specific T-cells.

**Table 16: NF-kB reporter gene activation by PD-L1 x CD137 multispecific constructs.**

| | | | | | **Activation of NF-kB reporter gene CHO-PD-L1** | | | | **Activation of NF-kB reporter gene HCC827 -IFNg** | | | **Activation of NF-kB reporter gene HCC827 +IFNg** | | | |
|---|---|---|---|---|---|---|---|---|---|---|---|---|---|---|---|
| PRO ID | Clone ID PD-L1 | Clone ID CD137 | Clone ID SA | Format | IC₅₀ (ng/ml) | rel. IC₅₀ & | max. activation (%) | HSA | IC₅₀ (ng/ml) | rel. IC₅₀ & | max. activation (%) | IC₅₀ (ng/ml) | rel. IC₅₀ & | max. activation (%) | HSA |
| PRO885 | 33-03-G02 CDR | 38-02-A04 CDR | NA | scDb | 11.72 | 6.75 | 499.42 | no | 13.06 | 8.42 | 111.10 | 8.88 | 16.30 | 134.20 | no |
| PRO951 | 33-03-G02 CDR | 38-27-C05 CDR | NA | scDb | 33.68 | 2.35 | 431.70 | no | ND | ND | ND | ND | ND | ND | ND |
| PRO1123 | 33-03-G02 CDR | 38-02-A04 IF | NA | scDb | 5.75 | 18.11 | 219.20 | no | 16.12 | 6.82 | 123.20 | 11.87 | 12.20 | 154.40 | no |
| PRO1124 | 33-03-G02 CDR | 38-02-A04 STR | NA | scDb | 3.68 | 23.50 | 228.60 | no | 5.62 | 19.89 | 113.20 | 4.83 | 22.39 | 188.50 | no |
| PRO1126 | 33-03-G02 STR | 38-02-A04 CDR | NA | scDb | 6.00 | 14.43 | 242.10 | no | 6.97 | 16.04 | 135.10 | 5.48 | 19.74 | 207.70 | no |
| | | | | | | | | | | | | | | | |
| PRO963 | 33-03-G02 CDR | 38-02-A04 CDR | 19-01-H04 STR | Triabody | ND | ND | 368.67 | yes, 24h | ND | ND | ND | ND | ND | ND | ND |
| PRO1057 | 33-03-G02 CDR | 38-02-A04 CDR | 23-13-A01 STR | Triabody | 792.40 | 0.10 | 662.79 | yes, 24h | 76.61 | 0.62 | 68.99 | 115.00 | 0.46 | 135.34 | yes, 24h |
| PRO1058 | 33-03-G02 CDR | 38-27-C05 CDR | 23-13-A01 STR | Triabody | 121.40 | 0.64 | 36.23 | yes, 24h | ND | ND | ND | ND | ND | ND | ND |
| | | | | | | | | | | | | | | | |
| PRO1059 | 33-03-G02 CDR | 38-02**-A04** CDR | NA | Morrison-L | 289.10 | 0.09 | 189.09 | no, 24h | ND | ND | ND | ND | ND | ND | ND |
| PRO1060 | 33-03-G02 CDR | 38-02-A04 CDR | NA | Morrison-H | 80.85 | 0.58 | 144.13 | no, 24h | 13.57 | 3.50 | 54.94 | 17.51 | 3.01 | 97.04 | no, 24h |
| PRO1061 | 33-03-G02 CDR | 38-27-C05 CDR | NA | Morrison-L | ND | ND | ND | ND | ND | ND | ND | ND | ND | ND | ND |
| PRO1062 | 33-03-G02 CDR | 38-27-C05 CDR | NA | Morrison-H | 86.87 | 0.54 | 133.52 | no, 24h | ND | ND | ND | ND | ND | ND | ND |

| | | | | | | | | | | | | | | | |
|---|---|---|---|---|---|---|---|---|---|---|---|---|---|---|---|
| NA: not applicable ND: not determined &: IC_{50,Urelumab} (ng/ml)/IC_{50,test molecule}(ng/ml) | | | | | | | | | | | | | | | |

### Example 8: Assessment of the stimulatory effect of concomitant PD-L1 blockade and CD137 stimulation in a cell-based assay using human PBMC stimulated with superantigen SEA.

In this experiment, the synergistic effect of PD-1/ PD-L1 inhibition and CD137 agonism was assessed. The assay used peripheral blood mononuclear cells (PBMC) that were stimulated with the superantigen Staphylococcal Enterotoxin A (SEA) in order to induce expression of PD-L1 on antigen-presenting cells (APC) and T cells respectively and CD137 on T-cells. By applying anti-PD-L1xCD137 molecules two T-cell regulatory signaling pathways were targeted concomitantly: inhibition of the inhibitory PD-1/PD-L1 pathway as well as activation of the CD137 pathway via formation of an immunological synapse mediated by the bispecific anti-PD-L1xCD137 molecule (PRO885). The activation of T-cells was assessed by the secretion of Interleukin-2 (IL-2) and compared to the effect mediated by PD-L1 inhibition mediated by the benchmarking reference antibody Avelumab. In addition, the anti-PD-L1 scFv, PRO997, was tested and compared to Avelumab in the same experimental setup.

### Method

Peripheral blood mononuclear cells (PBMC) were isolated from fresh human whole blood by means of density gradient centrifugation. Then, PBMC were depleted for NK cells using anti-CD56 antibody and the MACS cell separation kit (Miltenyi Biotec). Next, 100'000 PBMCs per well were added to the 96-well plate, followed by the addition of serial dilutions of PRO885, PRO997 and Avelumab in assay buffer containing SEA at a concentration of 10 ng/ml. After 96 hours of incubation at 37°C and 5% CO₂, cell supernatants were harvested and human Interleukin-2 (IL-2) levels in the culture supernatants were quantified using the IL-2 human ELISA MAX assay from BioLegend according to kit instructions. IL-2 concentrations were interpolated from a IL-2 standard curve, back-calculated and plotted against Avelumab and PRO885 concentrations for calculation of EC50 values.

### Results

**TABLE 18: EC50 values for PRO885 and PRO997 in PBMC assay using SEA stimulation.**

| | Avelumab | PRO885 |
|---|---|---|
| Bottom | 2479 | 7463 |
| Top | 8687 | 20663 |
| EC50 in ng/ml | 69.89 | 39.92 |
| R square | 0.8589 | 0.9052 |

| | Avelumab | PRO997 |
|---|---|---|
| Bottom | 2117 | 3226 |
| Top | 8588 | 9480 |
| EC50 in ng/ml | 90.18 | 40.86 |
| R square | 0.8783 | 0.867 |

As shown in Figure 19, IL-2 was secreted by T-cells following concomitant blockade of PD-1/PD-L1 interaction and stimulation of CD137 by the addition of the bispecific molecule PRO885. When compared to Avelumab, PRO885 showed higher T cell activation and better potency (PRO885, EC₅₀ = 39.92 ng/ml; Avelumab, EC₅₀ = 69.89 ng/ml, Table 18). This finding demonstrates that the bispecific anti-PD-L1xCD137 scDb PRO885 is able to induce stronger T cell stimulaton than mere PD-L1 blockade by Avelumab. Moreover, the high-affinity anti-PD-L1 scFv PRO997 was found to be more potent in stimulation of T-cells than Avelumab (PRO997, EC₅₀ = 40.86 ng/ml; Avelumab, EC₅₀ = 90.18 ng/ml, Table 18).

### Example 9: Assessment of the anti-tumor efficacy of PD-L1 blockade and concomitant localized stimulation of CD137 in the human cell line-derived lung cancer xenograft model HCC827.

Anti-tumor activity of the multispecific antibody of the present invention will be compared to anti-PD-L1 and anti-CD137 therapy in human HCC827 NSCLC xenografts using the immunodeficient NOG mice strain from Taconic and allogeneic human peripheral blood mononuclear cells. Engrafted human T lymphocytes show xeno-reactivity against foreign major histocompatibility (MHC) class I and II and other antigens from mice cells. As a result, T lymphocytes cause an inflammatory infiltrate in different organs that leads to death of the animals after several weeks, a process known as xenograft-versus-host disease (xGVHD). Treatment with immunomodulatory antibodies such as anti-PD-L1 and anti-CD137 was shown to exacerbate xGVHD (Sanmamed MF et al. Nivolumab and Urelumab enhance antitumor activity of human T lymphocytes engrafted in Rag2-/-IL2Rgnull immunodeficient mice. Cancer Res 2015;75(17):3466-3478). Effects of PRO1057 (scDb-scFv) and PRO1060 (IgG-scFv with CD137 scFv fused to the C-terminus of the heavy chain of the IgG) on tumor volume will be compared to treatment with the IgG1 containing the same PD-L1 specific variable domain as the multispecific antibody of the present invention (e.g., PRO1137) and with the IgG4 with the same CD137 specific variable domain (PRO1138). To provide further evidence of localized antitumor immune response, frequency of tumor infiltrating lymphocytes such as CD8+, CD4+ and regulatory T cells will be analyzed by flow cytometry. To explore modulation of the immune system systemically following anti-CD137/anti-PD-L1 treatment the frequency of CD4+ and CD8+ T cells in liver and spleen will be analyzed by flow cytometry. Moreover, systemic IFNg levels will be analyzed using a quantitative ELISA method.

### Study set-up and treatment schedule

Female NOG mice will receive unilateral injections of 5x10⁶ HCC827 cells. Cells will be injected in a mixture of 50% cell suspension in PBS and 50% matrigel in a total injection volume of 100 µl. After injection of tumor cells into NOG mice and successful tumor engraftment (median group tumor volume of 80-100 mm³), mice will be substituted with Sx10⁶ human PBMCs by intravenous injection. On the day of randomization, four mice of each group will be reconstituted with PBMCs of donor A and another four mice with PBMCs of donor B. Treatment will start 1-2 hours after the injection of PBMCs and will be applied as follows.

| **group ID** | **compound** | **total daily dose [mg]** | **dosing days** | **route** | **no. of mice** |
|---|---|---|---|---|---|
| 1 | **Vehicle** | na | 0,3,7,10 | ip | 8 |
| 2 | **PRO1057 low dose** | 0.08 | 0,3,7,10 | ip | 8 |
| 3 | **PRO1057 medium dose** | 0.4 | 0,3,7,10 | ip | 8 |
| 4 | **PRO1057 high dose** | 2 | 0,3,7,10 | ip | 8 |
| 5 | **PRO1137** | 0.2 | 0,3,7,10 | ip | 8 |
| 6 | **PRO1138** | 0.2 | 0,3,7,10 | ip | 8 |
| 7 | **PRO1060** | 0.2 | 0,3,7,10 | ip | 8 |

Body weights and tumor volume by caliper measurement will be performed twice weekly. Animals will be terminated at defined time-points depending on the study results.
Option A: Four animals per group will be terminated at an earlier time-point compared to the rest of the group (e.g. day 21) while the remaining animals will be terminated one week later (e.g. day 28) aiming at the maximum possible observation time which is limited by the onset of xGvHD.
Option B: All animals will be terminated at the 'same' time-point (between day 21 and day 28) with sample collection and processing of the first half of each group being performed on the first day and of the second half of each group on the following day for capacity reasons.

For both options, animals reconstituted with PBMCs from the two different donors will be equally represented in the two sampling cohorts.

Tumors, spleens and livers from all animals will be collected at the end of the study and will be processed for flow cytometry where the following human markers will be analyzed: Live/Dead, CD4, CD8, CD25, FOXP3, TIM3 and Granzyme B. Serum samples will be analyzed for IFNg levels by ELISA using the DuoSet® ELISA Development System from R&D Systems according to the manufacturer's instructions.

### Example 10: Assessment of the anti-tumor efficacy of PD-L1 blockade and concomitant localized stimulation of CD137 in a syngeneic MC38 colon cancer model.

In addition, anti-tumor activity of the multispecific antibody of the present invention will be tested in a MC38 colon carcinoma model in syngeneic C57BL/6 mice with an intact immune system. This model has been used by others to show enhanced antitumor activity by combination treatment with CD137 agonists and PD-1/PD-L1 antagonists (Chen S et al. Combination of 4-1BB agonist and PD-1 antagonist promotes antitumor effector/memory CD8 T cells in a poorly immunogenic tumor model. Cancer Immunol Res 2014;3(2):149-160 and Rodriguez-Ruiz ME et al. Abscopal effects of radiotherapy are enhanced by combined immunostimulatory mAbs and are dependent on CD8 T cells and crosspriming. Cancer Res 2016;76(20):5994-6005).

Since both, the anti-CD137 domain and the anti-PD-L1 domain of of the multispecific antibody of the present invention are not cross-reactive to mouse PD-L1 an engineered human CD137 knock-in model established by CrownBio will be used. In this model, the extracellular and transmembrane domain of mouse CD137 was replaced by the respective sequence of human CD137 in the C57BL/6 mice background using the CRISPR/Cas9 system. In addition, a modified MC38 tumor cell line expressing human PD-L1 under control of a CMV promoter instead of mouse PD-L1 will be used. Effects of the multispecific antibody of the present invention on tumor volume will be compared to combination treatment with the humanized IgG1 containing the same PD-L1 specific variable domain as ND021 and with the humanized IgG4 with the same CD137 specific variable domain. To provide further evidence of localized antitumor immune response, frequency of tumor infiltrating lymphocytes such as CD8+, CD4+ and regulatory T cells will be analyzed by flow cytometry. To explore modulation of the immune system systemically following anti-CD137/anti-PD-L1 treatment, the frequency of CD4+ and CD8+ T cells in liver and spleen will be analyzed by flow cytometry and possibly immunohistochemistry. Moreover, systemic IFNg levels could be analyzed using a quantitative ELISA method. To further characterize the safety profile of the anti-CD 137/anti-PD-L1 combination therapy, clinical chemistry pathology parameters associated primarily with liver toxicity (observed for anti-CD 137 therapy in the clinic), such as increased levels of alanine aminotransferase, glutamate dehydrogenase and aspartate aminotransferase could be assessed.

## Claims

1. An isolated antibody or antigen-binding fragment thereof having a binding specificity for human CD137, which comprises: (a) a heavy chain variable region CDR1 comprising an amino acid sequence selected from any one of SEQ ID NOs: 1, 2, 5 and 8; (b) a heavy chain variable region CDR2 comprising an amino acid sequence selected from any of SEQ ID NOs: 3, 6 and 9; (c) a heavy chain variable region CDR3 comprising an amino acid sequence selected from any of SEQ ID NOs: 4, 7 and 10; (d) a light chain variable region CDR1 comprising an amino acid sequence selected from any of SEQ ID NOs: 14, 17 and 20; (e) a light chain variable region CDR2 comprising an amino acid sequence selected from any of SEQ ID NOs: 15, 18 and 21; and (f) a light chain variable region CDR3 comprising an amino acid sequence selected from any of SEQ ID NOs: 16, 19 and 22.

2. The isolated antibody or antigen-binding fragment thereof of claim 1, comprising: (a) a HCDR1 comprising the amino acid sequence of SEQ ID NO: 1 or SEQ ID NO: 2; (b) a HCDR2 comprising the amino acid sequence of SEQ ID NO: 3; (c) a HCDR3 comprising the amino acid sequence of SEQ ID NO: 4; (d) a LCDR1 comprising the amino acid sequence of SEQ ID NOs: 14; (e) a LCDR2 comprising the amino acid sequence of SEQ ID NOs: 15; and (f) a LCDR3 comprising the amino acid sequence of SEQ ID NO: 16.

3. The antibody or antigen-binding fragment thereof of any one of the preceding claims, wherein the antibody or antigen-binding fragment thereof comprises a heavy chain variable region comprising an amino acid sequence that is at least 90 percent identical to the amino acid sequence selected from the group consisting of SEQ ID NOs: 11, 12 and 13; and a light chain variable region comprising an amino acid sequence that is at least 90 percent identical to the amino acid sequence selected from the group consisting of SEQ ID NOs: 23, 24 and 25.

4. The isolated antibody or antigen-binding fragment thereof of claim 2, comprising: (a) a HCDR1 comprising the amino acid sequence of SEQ ID NO: 1, a VH sequence of SEQ ID NO: 11 and a VL sequence of SEQ ID NO: 23; (b) a HCDR1 comprising the amino acid sequence of SEQ ID NO: 1, a VH sequence of SEQ ID NO: 12 and a VL sequence of SEQ ID NO: 24; or (c) a HCDR1 comprising the amino acid sequence of SEQ ID NO: 2, a VH sequence of SEQ ID NO: 13 and a VL sequence of SEQ ID NO: 25.

5. The antibody or antigen-binding fragment thereof of any of the preceding claims, wherein said antibody or said antigen-binding fragment
(i) binds to human CD137 with a dissociation constant (KD) of less than 50nM, particularly less than 10 nM, particularly less than 5 nM, particularly less than 1nM, particularly less than 500 pM, more particularly less than 100 pM, more particularly less than 50 pM;
(ii) binds to human CD137 with a K_{off} rate of 10⁻³ s⁻¹ or less, or 10⁻⁴ s⁻¹ or less, or 10⁻⁵ s⁻¹ or less as measured by SPR;
(iii) binds to human CD137 with a Kₒₙ rate of at least 10⁴ M⁻¹s⁻¹ or greater, at least 10⁵ M⁻¹s⁻¹ or greater, at least 10⁶ M⁻¹s⁻¹ or greater, as measured by SPR;
(iv) does not cross-compete with urelumab and utolimumab; and/or
(v) is cross-reactive with Macaca fascicularis (Cynomolgus) CD137.

6. The isolated antibody or antigen-binding fragment thereof of any of the previous claims, wherein the isolated antibody or antigen-binding fragment is selected from the group consisting of: a monoclonal antibody, a chimeric antibody, a Fab, an Fv, a scFv, dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology), preferably Fv or scFv.

7. The antibody or antigen-binding fragment thereof of claim 6, wherein said scFv has the amino acid sequence selected from the group consisting of SEQ ID NO:27, SEQ ID NO: 28, and SEQ ID NO: 29.

8. An isolated antibody or antigen-binding fragment thereof binding to essentially the same epitope as the antibody or antigen-binding fragment of any one of claims 1 to 7.

9. A multispecific molecule comprising any of the isolated antibody or antigen-binding fragments of any one of claims 1 to 8.

10. A pharmaceutical composition comprising the isolated antibody or antigen-binding fragment thereof of any one of claims 1-8, or the multispecific molecule of claims 9, and a pharmaceutically acceptable carrier.

11. The antibody or antigen-binding fragment thereof of any one of claims 8, or the multispecific molecule of claims 9, or the composition of claim 10 for use as a medicament.

12. The antibody or antigen-binding fragment thereof of any one of claims 1-8, or the multispecific molecule of claims 9, or the composition of claim 10 for use in a manufacture of a medicament.

13. A method of treating a cancer in a subject in need thereof comprising administering to the subject a therapeutically effective amount of the antibody or antigen-binding fragment thereof of any one of claims 1-8, or the multispecific molecule of claims 9, or the composition of claim 10.

14. A nucleic acid encoding the antibody or antigen-binding fragment thereof of claims 1-8.

15. A method of producing the antibody or antigen-binding fragment thereof of claims 1-8, the method comprising the step of culturing a host cell expressing a nucleic acid encoding the antibody or antigen-binding fragment thereof of claims 1-8.
